# EUROPEAN PATENT SPECIFICATION

(11) **EP 2 321 356 B1**
(45) Date of publication and mention of the grant of the patent: **11.04.2018**
(21) Application number: 09805438.0
(22) Date of filing: 04.08.2009
(51) Int. Cl.: C07K 16/38, C12P 21/08, A61K 39/395

(54) **MONOCLONAL ANTIBODIES AGAINST TISSUE FACTOR PATHWAY INHIBITOR (TFPI)**
MONOKLONALE ANTIKÖRPER GEGEN DEN GEWEBEFAKTORINHIBITOR (TFPI)
ANTICORPS MONOCLONAUX CONTRE L' INHIBITEUR DE LA VOIE DU FACTEUR TISSULAIRE (TFPI)

(30) Priority: 04.08.2008 US 85980 P
(43) Date of publication of application: 18.05.2011
(73) Proprietor: Bayer HealthCare, LLC, 100 Bayer Boulevard Whippany, New Jersey 07981-0915 (US)
(72) Inventor: WANG, Zhuozhi, Millbrae, CA 94030 (US); MURPHY, John, E., Berkeley California 94707 (US); PAN, Junliang, Moraga Town California 94556 (US); JIANG, Haiyan, San Francisco California 94121 (US); LIU, Bing, Richmond California 94803 (US)
(74) Representative: BIP Patents
(86) International application number: PCT/US2009/052702
(87) International publication number: WO 2010/017196

(56) References cited:
- WO-A1-92/07584
- WO-A1-97/09063
- WO-A1-2010/072691
- US-A1- 2002 082 206
- US-A1- 2002 197 605
- US-A1- 2003 004 324
- US-A1- 2003 028 920
- US-A1- 2003 059 937
- US-A1- 2003 064 491
- US-A1- 2003 157 082
- US-A1- 2003 233 675
- US-A1- 2004 029 129
- US-A1- 2004 029 129
- US-A1- 2004 031 072
- US-A1- 2004 034 888
- US-A1- 2004 052 799
- US-A1- 2004 123 343
- US-A1- 2004 171 538
- US-A1- 2004 172 684
- US-A1- 2004 214 272
- US-A1- 2005 004 354
- US-A1- 2005 108 791
- US-A1- 2005 118 643
- US-A1- 2005 158 313
- US-A1- 2006 003 425
- US-A1- 2006 019 260
- US-A1- 2006 024 297
- US-A1- 2006 068 386
- US-A1- 2006 246 071
- US-A1- 2006 251 658
- US-A1- 2007 020 624
- US-A1- 2007 020 625
- US-A1- 2007 021 600
- US-A1- 2007 044 171
- US-A1- 2007 061 916
- US-A1- 2007 065 439
- US-A1- 2007 083 334
- US-A1- 2007 118 916
- US-A1- 2007 202 552
- US-A1- 2007 202 566
- US-A1- 2007 271 630
- US-A1- 2008 025 980
- US-A1- 2008 050 774
- US-A1- 2008 095 775
- US-A1- 2008 148 432
- WOLBERG A S ET AL: "Mechanisms of autoantibody-induced monocyte tissue factor expression", THROMBOSIS RESEARCH, TARRYTOWN, NY, US, vol. 114, no. 5-6, 1 January 2004 (2004-01-01), pages 391-396, XP004613179, ISSN: 0049-3848, DOI: 10.1016/J.THROMRES.2004.06.012
- WELSCH D J ET AL: "Effect of lipoprotein-associated coagulation inhibitor (LACI) on thromboplastin-induced coagulation of normal and hemophiliac plasmas", THROMBOSIS RESEARCH, TARRYTOWN, NY, US, vol. 64, no. 2, 15 October 1991 (1991-10-15), pages 213-222, XP022879099, ISSN: 0049-3848, DOI: 10.1016/0049-3848(91)90120-L [retrieved on 1991-10-15]
- YANG Y ET AL: "[Preparation and characterization of a McAb against tissue factor pathway inhibitor]", MEDLINE,, 1 March 1997 (1997-03-01), XP002574353,
- ABUMIYA T ET AL: "An anti-tissue factor pathway inhibitor (TFPI) monoclonal antibody recognized the third Kunitz domain (K3) of free-form TFPI but not lipoprotein-associated forms in plasma", MEDLINE,, 1 July 1995 (1995-07-01), XP002574354,

## Description

### Sequence listing submission

The Sequence Listing associated with this application is filed in electronic format *via* EFS-Web and hereby incorporated by reference into the specification in its entirety. The name of the text file containing the Sequence Listing is
MSB7329PCT_Sequence_Listing_ST25.

### Field of the embodiments

Provided are isolated monoclonal antibodies and fragments thereof that bind human tissue factor pathway inhibitor (TFPI) and related inventions.

### Background

Blood coagulation is a process by which blood forms stable clots to stop bleeding. The process involves a number of proenzymes and procofactors (or "coagulation factors") that are circulating in the blood. Those proenzymes and procofactors interact through several pathways through which they are converted, either sequentially or simultaneously, to the activated form. Ultimately, the process results in the activation of prothrombin to thrombin by activated Factor X (FXa) in the presence of Factor Va, ionic calcium, and platelets. The activated thrombin in turn induces platelet aggregation and converts fibrinogen into fibrin, which is then cross linked by activated Factor XIII (FXIIIa) to form a clot.

The process leading to the activation of Factor X can be carried out by two distinct pathways: the contact activation pathway (formerly known as the intrinsic pathway) and the tissue factor pathway (formerly known as the extrinsic pathway). It was previously thought that the coagulation cascade consisted of two pathways of equal importance joined to a common pathway. It is now known that the primary pathway for the initiation of blood coagulation is the tissue factor pathway.

Factor X can be activated by tissue factor (TF) in combination with activated Factor VII (FVIIa). The complex of Factor VIIa and its essential cofactor, TF, is a potent initiator of the clotting cascade.

The tissue factor pathway of coagulation is negatively controlled by tissue factor pathway inhibitor ("TFPI"). TFPI is a natural, FXa-dependent feedback inhibitor of the FVIIa/TF complex. It is a member of the multivalent Kunitz-type serine protease inhibitors. Physiologically, TFPI binds to activated Factor X (FXa) to form a heterodimeric complex, which subsequently interacts with the FVIIa/TF complex to inhibit its activity, thus shutting down the tissue factor pathway of coagulation. In principle, blocking TFPI activity can restore FXa and FVIIa/TF activity, thus prolonging the duration of action of the tissue factor pathway and amplifying the generation of FXa, which is the common defect in hemophilia A and B.

Indeed, some preliminary experimental evidence has indicated that blocking the TFPI activity by antibodies against TFPI normalizes the prolonged coagulation time or shortens the bleeding time. For instance, Nordfang et al. showed that the prolonged dilute prothrombin time of hemophilia plasma was normalized after treating the plasma with antibodies to TFPI (Thromb. Haemost., 1991, 66(4): 464-467). Similarly, Erhardtsen et al. showed that the bleeding time in hemophilia A rabbit model was significantly shortened by anti-TFPI antibodies (Blood Coagulation and Fibrinolysis, 1995, 6: 388-394). These studies suggest that inhibition of TFPI by anti-TFPI antibodies may be useful for the treatment of hemophilia A or B. Only polyclonal anti-TFPI antibody was used in these studies.

Using hybridoma techniques, monoclonal antibodies against recombinant human TFPI (rhTFPI) were prepared and identified. *See* Yang et al., Chin. Med. J., 1998, 111(8): 718-721. The effect of the monoclonal antibody on dilute prothrombin time (PT) and activated partial thromboplastin time (APTT) was tested. Experiments showed that anti-TFPI monoclonal antibody shortened dilute thromboplastin coagulation time of Factor IX deficient plasma. It is suggested that the tissue factor pathway plays an important role not only in physiological coagulation but also in hemorrhage of hemophilia (Yang et al., Hunan Yi Ke Da Xue Xue Bao, 1997, 22(4): 297-300).

U.S. Patent No. 7,015,194 to Kjalke et al. discloses compositions comprising FVIIa and a TFPI inhibitor, including polyclonal or monoclonal antibodies, or a fragment thereof, for treatment or prophylaxis of bleeding episodes or coagulative treatment. The use of such composition to reduce clotting time in normal mammalian plasma is also disclosed. It is further suggested that a Factor VIII or a variant thereof may be included in the disclosed composition of FVIIa and TFPI inhibitor. A combination of FVIII or Factor IX with TFPI monoclonal antibody is not suggested.

Welsch, D. J. et al., "Effect of lipoprotein-associated coagulation inhibitor (LACI) on thromboplastin-induced coagulation of normal and hemophiliac plasmas", Thrombosis Res., 64(2): 213-222 (1991), discloses the curative effect on clotting time of hemophilia patients after administration of polyclonal and monoclonal Ab binding to LACI (TFPI), but does not disclose a fully human anti-TFPI mAb or mAb characterized by (any) specific CDRs.

In addition to the treatment for hemophilia, it has also been suggested that TFPI inhibitors, including polyclonal or monoclonal antibodies, can be used for cancer treatment (see U.S. Pat. No. 5,902,582 to Hung).

Accordingly, antibodies specific for TFPI are needed for treating hematological diseases and cancer.

Generally, therapeutic antibodies for human diseases have been generated using genetic engineering to create murine, chimeric, humanized or fully human antibodies. Murine monoclonal antibodies were shown to have limited use as therapeutic agents because of a short serum half-life, an inability to trigger human effector functions, and the production of human antimouse-antibodies. Brekke and Sandlie, "Therapeutic Antibodies for Human Diseases at the Dawn of the Twenty-first Century," Nature 2, 53, 52-62 (Jan. 2003). Chimeric antibodies have been shown to give rise to human anti-chimeric antibody responses. Humanized antibodies further minimize the mouse component of antibodies. However, a fully human antibody avoids the immunogenicity associated with murine elements completely. Thus, there is a need to develop fully human antibodies to avoid the immunogenicity associated with other forms of genetically engineered monoclonal antibodies. In particular, chronic prophylactic treatment such as would be required for hemophilia treatment with an anti-TFPI monoclonal antibody has a high risk of development of an immune response to the therapy if an antibody with a murine component or murine origin is used due to the frequent dosing required and the long duration of therapy. For example, antibody therapy for hemophilia A may require weekly dosing for the lifetime of a patient. This would be a continual challenge to the immune system. Thus, the need exists for a fully human antibody for antibody therapy for hemophilia and related genetic and acquired deficiencies or defects in coagulation.

Therapeutic antibodies have been made through hybridoma technology described by Koehler and Milstein in "Continuous Cultures of Fused Cells Secreting Antibody of Predefined Specificity," Nature 256, 495-497 (1975). Fully human antibodies may also be made recombinantly in prokaryotes and eukaryotes. Recombinant production of an antibody in a host cell rather than hybridoma production is preferred for a therapeutic antibody. Recombinant production has the advantages of greater product consistency, likely higher production level, and a controlled manufacture that minimizes or eliminates the presence of animal-derived proteins. For these reasons, it is desirable to have a recombinantly produced monoclonal anti-TFPI antibody.

### Summary

Monoclonal antibodies to human tissue factor pathway inhibitor (TFPI) are provided. Further provided are the isolated nucleic acid molecules encoding the same. Pharmaceutical compositions comprising the anti-TFPI monoclonal antibodies and their use for the treatment of genetic and acquired deficiencies or defects in coagulation such as hemophilia A and B are also disclosed. Also disclosed are uses of these antibodies for shortening the bleeding time by administering said anti-TFPI monoclonal antibody to a patient in need thereof. Methods for producing a monoclonal antibody that binds human TFPI according to the present invention are also disclosed.

### Brief description of the drawings

Fig. 1: The binding activity of representative examples of Fabs, selected from the panning and screening, to human TFPI ("h-TFPI") and mouse TFPI ("m-TFPI"). A control Fab against Estradiol-BSA ("EsB") and 12 Fabs (1-4 and 6-13) selected from panning TFPI were tested. Y-axis denotes fluorescence units of ELISA results.
Fig. 2: The dose-dependent in vitro functional activity of four representative anti-TFPI antibodies (4B7: TP-4B7, 2A8: TP-2A8, 2G6: TP-2G6, 2G7: TP-2G7) obtained from the panning and screening of a human antibody library as shown by their shortening dPT. The experiment involved 0.5 ug/mL of mTFPI spiked into TFPI depleted plasma.
Fig. 3: The in vitro functional activity of anti-TFPI Fab, Fab-2A8 (from TP-2A8), as tested in ROTEM assay.
Fig. 4: The binding activity to human TFPI and mouse TFPI of clones TP-2G6 ("2G6") after the conversion to IgG. Δ: IgG-2G6 binding to mouse TFPI; □: IgG-2G6 binding to human TFPI; ▲: control IgG binding to mouse TFPI; ■: control IgG binding to human IgG.
Fig 5: The anti-TFPI antibodies TP-2A8 ("2A8"), TP-3G1 ("3G1"), and TP-3C2 ("3C2") shortened the whole blood clotting time in hemophilia A mice as tested in ROTEM assay. Each dot represents one individual hemophilia A mouse.

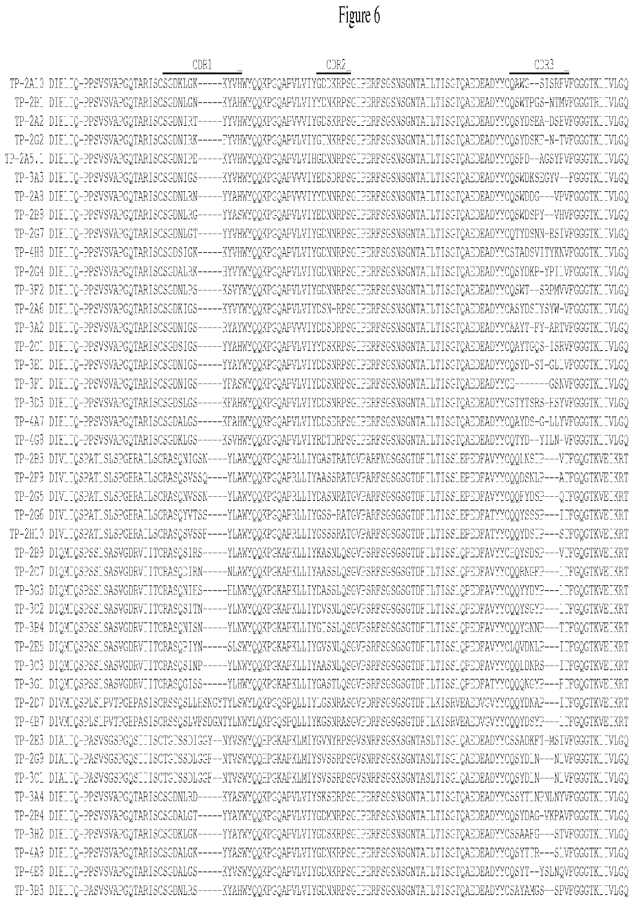

Fig. 6: The amino acid sequence alignment between the variable light chains of anti-TFPI monoclonal antibodies TP-2A10 (SEQ ID NO: 18), TP-2B1 (SEQ ID NO: 22), TP-2A2 (SEQ ID NO: 2), TP-2G2 (SEQ ID NO: 66), TP-2A5.1 (SEQ ID NO: 6), TP-3A3 (SEQ ID NO: 98), TP-2A8 (SEQ ID NO: 14), TP-2B8 (SEQ ID NO: 34), TP-2G7 (SEQ ID NO: 82), TP-4H8 (SEQ ID NO: 170), TP-2G4 (SEQ ID NO: 70), TP-3F2 (SEQ ID NO: 134), TP-2A6 (SEQ ID NO: 10), TP-3A2 (SEQ ID NO: 94), TP-2C1 (SEQ ID NO: 42), TP-3E1 (SEQ ID NO: 126), TP-3F1 (SEQ ID NO: 130), TP-3D3 (SEQ ID NO: 122), TP-4A7 (SEQ ID NO: 150), TP-4G8 (SEQ ID NO: 166), TP-2B3 (SEQ ID NO: 26), TP-2F9 (SEQ ID NO: 62), TP-2G5 (SEQ ID NO: 74), TP-2G6 (SEQ ID NO: 78), TP-2H10 (SEQ ID NO: 90), TP-2B9 (SEQ ID NO: 38), TP-2C7 (SEQ ID NO: 46), TP-3G3 (SEQ ID NO: 142), TP-3C2 (SEQ ID NO: 114), TP-3B4 (SEQ ID NO: 110), TP-2E5 (SEQ ID NO: 58), TP-3C3 (SEQ ID NO: 118), TP-3G1 (SEQ ID NO: 138), TP-2D7 (SEQ ID NO: 50), TP-4B7 (SEQ ID NO: 158), TP-2E3 (SEQ ID NO: 54), TP-2G9 (SEQ ID NO: 86), TP-3C1 (SEQ ID NO: 86), TP-3A4 (SEQ ID NO: 102), TP-2B4 (SEQ ID NO: 30), TP-3H2 (SEQ ID NO: 146), TP-4A9 (SEQ ID NO: 154), TP-4E8 (SEQ ID NO: 162), and TP-3B3 (SEQ ID NO: 106).

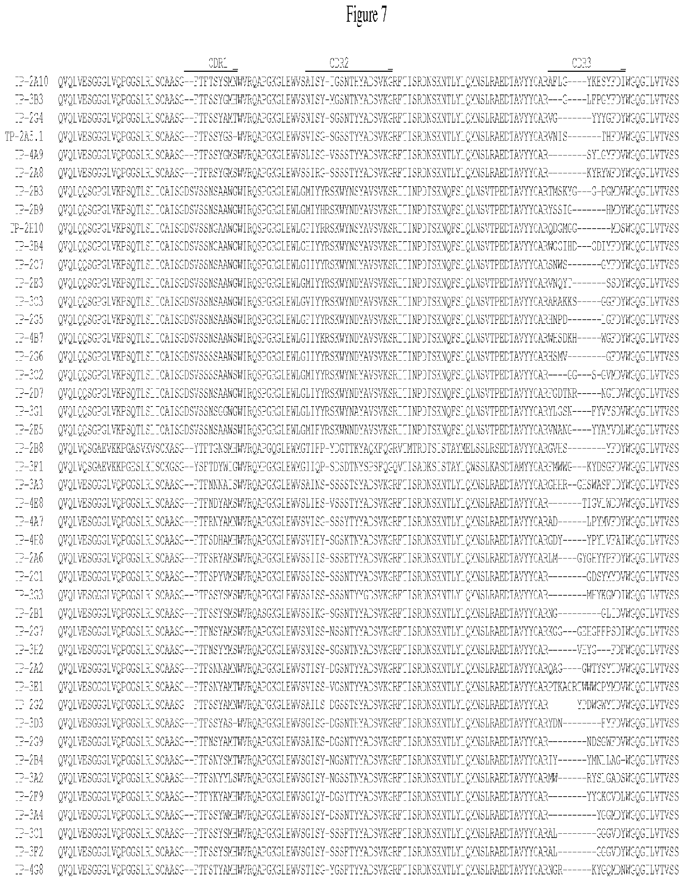

Fig. 7: The amino acid sequence alignment between the variable heavy chains of anti-TFPI monoclonal antibodies TP-2A10 (SEQ ID NO: 20), TP-3B3 (SEQ ID NO: 108), TP-2G4 (SEQ ID NO: 72), TP-2A5.1 (SEQ ID NO: 8), TP-4A9 (SEQ ID NO: 156), TP-2A8 (SEQ ID NO: 16), TP-2B3 (SEQ ID NO: 28), TP-2B9 (SEQ ID NO: 40), TP-2H10 (SEQ ID NO: 92), TP-3B4 (SEQ ID NO: 112), TP-2C7 (SEQ ID NO: 48), TP-2E3 (SEQ ID NO: 56), TP-3C3 (SEQ ID NO: 120), TP-2G5 (SEQ ID NO: 76), TP-4B7 (SEQ ID NO: 160), TP-2G6 (SEQ ID NO: 80), TP-3C2 (SEQ ID NO: 116), TP-2D7 (SEQ ID NO: 52), TP-3G1 (SEQ ID NO: 140), TP-2E5 (SEQ ID NO: 60), TP-2B8 (SEQ ID NO: 36), TP-3F1 (SEQ ID NO: 132), TP-3A3 (SEQ ID NO: 100), TP-4E8 (SEQ ID NO: 164), TP-4A7 (SEQ ID NO: 152), TP-4H8 (SEQ ID NO: 172), TP-2A6 (SEQ ID NO: 12), TP-2C1 (SEQ ID NO: 44), TP-3G3 (SEQ ID NO: 144), TP-2B1 (SEQ ID NO: 24), TP-2G7 (SEQ ID NO: 84), TP-3H2 (SEQ ID NO: 148), TP-2A2 (SEQ ID NO: 4), TP-3E1 (SEQ ID NO: 128), TP-2G2 (SEQ ID NO: 68), TP-3D3 (SEQ ID NO: 124), TP-2G9 (SEQ ID NO: 88), TP-2B4 (SEQ ID NO: 32), TP-3A2 (SEQ ID NO: 96), TP-2F9 (SEQ ID NO: 64), TP-3A4 (SEQ ID NO: 104), TP-3C1 (SEQ ID NO: 136), TP-3F2 (SEQ ID NO: 136), and TP-4G8 (SEQ ID NO: 168).
Fig. 8: Graph showing the survival rate over 24 hours post-tail vein transection for mice treated with (1) the anti-TFPI antibody TP-2A8 ("2A8"), (2) 2A8 and recombinant factor VIII, (3) mouse IgG, and (4) recombinant factor VIII.
Fig. 9: Graphs showing clotting time and clot formation time assays for mice treated with the anti-TFPI antibody TP-2A8 ("2A8"), factor VIIa, and the combination of 2A8 and factor VIIa.
Fig. 10: Graph showing clotting time for normal human blood treated with a FVIII inhibitor with the anti-TFPI antibody TP-2A8 ("2A8")and anti-TFPI antibody TP-4B7 ("4B7") as compared to FVIII inhibitor alone.

### Detailed description

### Definitions

The term "tissue factor pathway inhibitor" or "TFPI" as used herein refers to any variant, isoform and species homolog of human TFPI that is naturally expressed by cells. In a preferred embodiment of the invention, the binding of an antibody of the invention to TFPI reduces the blood clotting time.

As used herein, an "antibody" refers to a whole antibody and any antigen binding fragment (i.e., "antigen-binding portion") or single chain thereof. The term includes a full-length immunoglobulin molecule (e.g., an IgG antibody) that is naturally occurring or formed by normal immunoglobulin gene fragment recombinatorial processes, or an immunologically active portion of an immunoglobulin molecule, such as an antibody fragment, that retains the specific binding activity. Regardless of structure, an antibody fragment binds with the same antigen that is recognized by the full-length antibody. For example, an anti-TFPI monoclonal antibody fragment binds to an epitope of TFPI. The antigen-binding function of an antibody can be performed by fragments of a full-length antibody. Examples of binding fragments encompassed within the term "antigen-binding portion" of an antibody include (i) a Fab fragment, a monovalent fragment consisting of the V_{L}, V_{H}, C_{L} and C_{H1} domains; (ii) a F(ab')₂ fragment, a bivalent fragment comprising two Fab fragments linked by a disulfide bridge at the hinge region; (iii) a Fd fragment consisting of the V_{H} and C_{H1} domains; (iv) a Fv fragment consisting of the V_{L} and V_{H} domains of a single arm of an antibody, (v) a dAb fragment (Ward et al., (1989) Nature 341:544-546), which consists of a V_{H} domain; and (vi) an isolated complementarity determining region (CDR). Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see e.g., Bird et al. (1988) Science 242:423-426; and Huston et al (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies.

As used herein, the terms "inhibits binding" and "blocks binding" (e.g., referring to inhibition/blocking of binding of TFPI ligand to TFPI) are used interchangeably and encompass both partial and complete inhibition or blocking. Inhibition and blocking are also intended to include any measurable decrease in the binding affinity of TFPI to a physiological substrate when in contact with an anti-TFPI antibody as compared to TFPI not in contact with an anti-TFPI antibody, e.g., the blocking of the interaction of TFPI with factor Xa or blocking the interaction of a TFPI-factor Xa complex with tissue factor, factor VIIa or the complex of tissue factor/factor VIIa by at least about 10%, 20%, 30%, 40%, 50%, 60%, 70%, 80%, 90%, 95%, 96%, 97%, 98%, 99%, or 100%.

The terms "monoclonal antibody" or "monoclonal antibody composition" as used herein refer to a preparation of antibody molecules of single molecular composition. A monoclonal antibody composition displays a single binding specificity and affinity for a particular epitope. Accordingly, the term "human monoclonal antibody" refers to antibodies displaying a single binding specificity which have variable and constant regions derived from human germline immunoglobulin sequences. The human antibodies of the invention may include amino acid residues not encoded by human germline immunoglobulin sequences (e.g., mutations introduced by random or site-specific mutagenesis in vitro or by somatic mutation in vivo).

An "isolated antibody," as used herein, is intended to refer to an antibody which is substantially free of other antibodies having different antigenic specificities (e.g., an isolated antibody that binds to TFPI is substantially free of antibodies that bind antigens other than TFPI). An isolated antibody that binds to an epitope, isoform or variant of human TFPI may, however, have cross-reactivity to other related antigens, e.g., from other species (e.g., TFPI species homologs). Moreover, an isolated antibody may be substantially free of other cellular material and/or chemicals.

As used herein, "specific binding" refers to antibody binding to a predetermined antigen. Typically, the antibody binds with an affinity of at least about 10⁵ M⁻¹ and binds to the predetermined antigen with an affinity that is higher, for example at least two-fold greater, than its affinity for binding to an irrelevant antigen (e.g., BSA, casein) other than the predetermined antigen or a closely-related antigen. The phrases "an antibody recognizing an antigen" and "an antibody specific for an antigen" are used interchangeably herein with the term "an antibody which binds specifically to an antigen."

As used herein, the term "high affinity" for an IgG antibody refers to a binding affinity of at least about 10⁷M⁻¹, in some embodiments at least about 10⁸M⁻¹, in some embodiments at least about 10⁹M⁻¹, 10¹⁰M⁻¹, 10¹¹M⁻¹ or greater, e.g., up to 10¹³M⁻¹ or greater. However, "high affinity" binding can vary for other antibody isotypes. For example, "high affinity" binding for an IgM isotype refers to a binding affinity of at least about 1.0 x 10⁷M⁻¹. As used herein, "isotype" refers to the antibody class (e.g., IgM or IgG1) that is encoded by heavy chain constant region genes.

"Complementarity-determining region" or "CDR" refers to one of three hypervariable regions within the variable region of the heavy chain or the variable region of the light chain of an antibody molecule that form the N-terminal antigen-binding surface that is complementary to the three-dimensional structure of the bound antigen. Proceeding from the N-terminus of a heavy or light chain, these complementarity-determining regions are denoted as "CDR1," "CDR2," and "CDR3," respectively. CDRs are involved in antigen-antibody binding, and the CDR3 comprises a unique region specific for antigen-antibody binding. An antigen-binding site, therefore, may include six CDRs, comprising the CDR regions from each of a heavy and a light chain V region.

As used herein, "conservative substitutions" refers to modifications of a polypeptide that involve the substitution of one or more amino acids for amino acids having similar biochemical properties that do not result in loss of a biological or biochemical function of the polypeptide. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine), and aromatic side chains (e.g., tyrosine, phenylalanine, tryptophan, histidine). It is envisioned that the antibodies of the present invention may have conservative amino acid substitutions and still retain activity.

For nucleic acids and polypeptides, the term "substantial homology" indicates that two nucleic acids or two polypeptides, or designated sequences thereof, when optimally aligned and compared, are identical, with appropriate nucleotide or amino acid insertions or deletions, in at least about 80% of the nucleotides or amino acids, usually at least about 85%, preferably about 90%, 91%, 92%, 93%, 94%, or 95%, more preferably at least about 96%, 97%, 98%, 99%, 99.1%, 99.2%, 99.3%, 99.4%, or 99.5% of the nucleotides or amino acids. Alternatively, substantial homology for nucleic acids exists when the segments will hybridize under selective hybridization conditions to the complement of the strand. The invention includes nucleic acid sequences and polypeptide sequences having substantial homology to the specific nucleic acid sequences and amino acid sequences recited herein.

The percent identity between two sequences is a function of the number of identical positions shared by the sequences (i.e., % homology = # of identical positions / total # of positions x 100), taking into account the number of gaps, and the length of each gap, which need to be introduced for optimal alignment of the two sequences. The comparison of sequences and determination of percent identity between two sequences can be accomplished using a mathematical algorithm, such as without limitation the AlignX™ module of VectorNTI™ (Invitrogen Corp., Carlsbad, CA). For AlignX™, the default parameters of multiple alignment are: gap opening penalty: 10; gap extension penalty: 0.05; gap separation penalty range: 8; % identity for alignment delay: 40. (further details found at http://www.invitrogen.com/site/us/en/home/LINNEA-Online-Guides/LINNEA-Communities/Vector-NTI-Community/Sequence-analysis-and-data-management-software-for-PCs/AlignX-Module-for-Vector-NTI-Advance.reg.us.html).

Another method for determining the best overall match between a query sequence (a sequence of the present invention) and a subject sequence, also referred to as a global sequence alignment, can be determined using the CLUSTALW computer program (Thompson et al., Nucleic Acids Research, 1994, 2(22): 4673-4680), which is based on the algorithm of Higgins et al., (Computer Applications in the Biosciences (CABIOS), 1992, 8(2): 189-191). In a sequence alignment the query and subject sequences are both DNA sequences. The result of said global sequence alignment is in percent identity. Preferred parameters used in a CLUSTALW alignment of DNA sequences to calculate percent identity via pairwise alignments are: Matrix = IUB, k-tuple = 1, Number of Top Diagonals = 5, Gap Penalty = 3, Gap Open Penalty = 10, Gap Extension Penalty = 0.1. For multiple alignments, the following CLUSTALW parameters are preferred: Gap Opening Penalty = 10, Gap Extension Parameter = 0.05; Gap Separation Penalty Range = 8; % Identity for Alignment Delay = 40.

The nucleic acids may be present in whole cells, in a cell lysate, or in a partially purified or substantially pure form. A nucleic acid is "isolated" or "rendered substantially pure" when purified away from other cellular components with which it is normally associated in the natural environment. To isolate a nucleic acid, standard techniques such as the following may be used: alkaline/SDS treatment, CsCl banding, column chromatography, agarose gel electrophoresis and others well known in the art.

### Monoclonal Antibodies

Forty-four TFPI-binding antibodies were identified from panning and screening of human antibody libraries against human TFPI. The heavy chain variable region and light chain variable region of each monoclonal antibody were sequenced and their CDR regions were identified. The sequence identifier numbers ("SEQ ID NO") correspond to these regions of each monoclonal antibody are summarized in Table 1.

**Table 1. Summary of the sequence identifier numbers ("SEQ ID NO") of the heavy chain variable region ("VH") and light chain variable region ("VL") of each TFPI-binding monoclonal antibodies. The sequence identifier numbers for the CDR regions ("CDR1," "CDR2," and "CDR3") of each heavy and light chain are also provided. N.A.: nucleic acid sequence; A.A.: amino acid sequence.**

| Clone | VL | | VH | | VL | | | VH | | |
|---|---|---|---|---|---|---|---|---|---|---|
| | N.A. | A.A. | N.A. | A.A. | CDR1 | CDR2 | CDR3 | CDR1 | CDR2 | CDR3 |
| TP-2A2 | 1 | 2 | 3 | 4 | 173 | 216 | 259 | 302 | 345 | 388 |
| TP-2A5.1 | 5 | 6 | 7 | 8 | 174 | 217 | 260 | 303 | 346 | 389 |
| TP-2A6 | 9 | 10 | 11 | 12 | 175 | 218 | 261 | 304 | 347 | 390 |
| TP-2A8 | 13 | 14 | 15 | 16 | 176 | 219 | 262 | 305 | 348 | 391 |
| TP-2A10 | 17 | 18 | 19 | 20 | 177 | 220 | 263 | 306 | 349 | 392 |
| TP-2B1 | 21 | 22 | 23 | 24 | 178 | 221 | 264 | 307 | 350 | 393 |
| TP-2B3 | 25 | 26 | 27 | 28 | 179 | 222 | 265 | 308 | 351 | 394 |
| TP-2B4 | 29 | 30 | 31 | 32 | 180 | 223 | 266 | 309 | 352 | 395 |
| TP-2B8 | 33 | 34 | 35 | 36 | 181 | 224 | 267 | 310 | 353 | 396 |
| TP-2B9 | 37 | 38 | 39 | 40 | 182 | 225 | 268 | 311 | 354 | 397 |
| TP-2C1 | 41 | 42 | 43 | 44 | 183 | 226 | 269 | 312 | 355 | 398 |
| TP-2C7 | 45 | 46 | 47 | 48 | 184 | 227 | 270 | 313 | 356 | 399 |
| TP-2D7 | 49 | 50 | 51 | 52 | 185 | 228 | 271 | 314 | 357 | 400 |
| TP-2E3 | 53 | 54 | 55 | 56 | 186 | 229 | 272 | 315 | 358 | 401 |
| TP-2E5 | 57 | 58 | 59 | 60 | 187 | 230 | 273 | 316 | 359 | 402 |
| TP-2F9 | 61 | 62 | 63 | 64 | 188 | 231 | 274 | 317 | 360 | 403 |
| TP-2G2 | 65 | 66 | 67 | 68 | 189 | 232 | 275 | 318 | 361 | 404 |
| TP-2G4 | 69 | 70 | 71 | 72 | 190 | 233 | 276 | 319 | 362 | 405 |
| TP-2G5 | 73 | 74 | 75 | 76 | 191 | 234 | 277 | 320 | 363 | 406 |
| TP-2G6 | 77 | 78 | 79 | 80 | 192 | 235 | 278 | 321 | 364 | 407 |
| TP-2G7 | 81 | 82 | 83 | 84 | 193 | 236 | 279 | 322 | 365 | 408 |
| TP-2G9 | 85 | 86 | 87 | 88 | 194 | 237 | 280 | 323 | 366 | 409 |
| TP-2H10 | 89 | 90 | 91 | 92 | 195 | 238 | 281 | 324 | 367 | 410 |
| TP-3A2 | 93 | 94 | 95 | 96 | 196 | 239 | 282 | 325 | 368 | 411 |
| TP-3A3 | 97 | 98 | 99 | 100 | 197 | 240 | 283 | 326 | 369 | 412 |
| TP-3A4 | 101 | 102 | 103 | 104 | 198 | 241 | 284 | 327 | 370 | 413 |
| TP-3B3 | 105 | 106 | 107 | 108 | 199 | 242 | 285 | 328 | 371 | 414 |
| TP-3B4 | 109 | 110 | 111 | 112 | 200 | 243 | 286 | 329 | 372 | 415 |
| TP-3C2 | 113 | 114 | 115 | 116 | 201 | 244 | 287 | 330 | 373 | 416 |
| TP-3C3 | 117 | 118 | 119 | 120 | 202 | 245 | 288 | 331 | 374 | 417 |
| TP-3D3 | 121 | 122 | 123 | 124 | 203 | 246 | 289 | 332 | 375 | 418 |
| TP-3E1 | 125 | 126 | 127 | 128 | 204 | 247 | 290 | 333 | 376 | 419 |
| TP-3F1 | 129 | 130 | 131 | 132 | 205 | 248 | 291 | 334 | 377 | 420 |
| TP-3F2 | 133 | 134 | 135 | 136 | 206 | 249 | 292 | 335 | 378 | 421 |
| TP-3G1 | 137 | 138 | 139 | 140 | 207 | 250 | 293 | 336 | 379 | 422 |
| TP-3G3 | 141 | 142 | 143 | 144 | 208 | 251 | 294 | 337 | 380 | 423 |
| TP-3H2 | 145 | 146 | 147 | 148 | 209 | 252 | 295 | 338 | 381 | 424 |
| TP-4A7 | 149 | 150 | 151 | 152 | 210 | 253 | 296 | 339 | 382 | 425 |
| TP-4A9 | 153 | 154 | 155 | 156 | 211 | 254 | 297 | 340 | 383 | 426 |
| TP-4B7 | 157 | 158 | 159 | 160 | 212 | 255 | 298 | 341 | 384 | 427 |
| TP-4E8 | 161 | 162 | 163 | 164 | 213 | 256 | 299 | 342 | 385 | 428 |
| TP-4G8 | 165 | 166 | 167 | 168 | 214 | 257 | 300 | 343 | 386 | 429 |
| TP-4H8 | 169 | 170 | 171 | 172 | 215 | 258 | 301 | 344 | 387 | 430 |
| TP-3C1 | 85 | 86 | 135 | 136 | 194 | 237 | 280 | 335 | 378 | 421 |

Disclosed are isolated monoclonal antibodies that bind to human tissue factor pathway inhibitor, wherein the antibodies comprise a CDR3 comprising an amino acid sequence selected from the group consisting of SEQ ID NOs: 388-430. These CDR3s are identified from the heavy chains of the antibodies identified during panning and screening. Further disclosed are antibodies further comprising (a) a CDR1 comprising an amino acid sequence selected from the group consisting of SEQ ID NOs: 302-344, (b) a CDR2 comprising an amino acid sequence selected from the group consisting of SEQ ID NOs: 345-387, or (c) both a CDR1 comprising an amino acid sequence selected from the group consisting of SEQ ID NOs: 302-344 and a CDR2 comprising an amino acid sequence selected from the group consisting of SEQ ID NOs: 345-387.

Further disclosed are antibodies that share a CDR3 from one of the light chains of the antibodies identified during panning and screening. Thus, the present disclosure is directed to an isolated monoclonal antibody that binds to human tissue factor pathway inhibitor, wherein the antibody comprises a CDR3 comprising an amino acid sequence selected from the group consisting of SEQ ID NOs: 259-301. Further disclosed are antibodies further comprising (a) a CDR1 comprising an amino acid sequence selected from the group consisting of SEQ ID NOs: 173-215, (b) a CDR2 comprising an amino acid sequence selected from the group consisting of SEQ ID NOs: 216-258, or (c) both a CDR1 comprising an amino acid sequence selected from the group consisting of SEQ ID NOs: 173-215 and a CDR2 comprising an amino acid sequence selected from the group consisting of SEQ ID NOs: 216-258.

Further disclosed are antibodies comprising a CDR3 from a heavy chain and a CDR3 from a light chain of the antibodies identified from screening and panning. Thus, disclosed is an antibody that binds to human tissue factor pathway inhibitor, wherein the antibody comprises a CDR3 comprising an amino acid sequence selected from the group consisting of SEQ ID NOs: 388-430 and a CDR3 comprising an amino acid sequence selected from the group consisting of SEQ ID NOs: 259-301. The disclosed antibody further comprises (a) a CDR1 comprising an amino acid sequence selected from the group consisting of SEQ ID NOs: 302-344, (b) a CDR2 comprising an amino acid sequence selected from the group consisting of SEQ ID NOs: 345-387, (c) a CDR1 comprising an amino acid sequence selected from the group consisting of SEQ ID NOs: 173-215, and/or (d) a CDR2 comprising an amino acid sequence selected from the group consisting of SEQ ID NOs: 216-258.

The disclosed antibody comprises heavy and light chain variable regions comprising:
(a) a light chain variable region comprising an amino acid sequence comprising SEQ ID NOs: 173, 216 and 259 and a heavy chain variable region comprising an amino acid sequence comprising SEQ ID NOs: 302, 345 and 388;
(b) a light chain variable region comprising an amino acid sequence comprising SEQ ID NOs: 174, 217 and 260 and a heavy chain variable region comprising an amino acid sequence comprising SEQ ID NOs: 303, 346 and 389;
(c) a light chain variable region comprising an amino acid sequence comprising SEQ ID NOs: 175, 218 and 261 and a heavy chain variable region comprising an amino acid sequence comprising SEQ ID NOs: 304, 347 and 390;
(d) a light chain variable region comprising an amino acid sequence comprising SEQ ID NOs: 176, 219 and 262 and a heavy chain variable region comprising an amino acid sequence comprising SEQ ID NOs: 305, 348 and 391;
(e) a light chain variable region comprising an amino acid sequence comprising SEQ ID NOs: 177, 220 and 263 and a heavy chain variable region comprising an amino acid sequence comprising SEQ ID NOs: 306, 349 and 392;
(f) a light chain variable region comprising an amino acid sequence comprising SEQ ID NOs: 178, 221 and 264 and a heavy chain variable region comprising an amino acid sequence comprising SEQ ID NOs: 307, 350 and 393;
(g) a light chain variable region comprising an amino acid sequence comprising SEQ ID NOs: 179, 222 and 265 and a heavy chain variable region comprising an amino acid sequence comprising SEQ ID NOs: 308, 351 and 394;
(h) a light chain variable region comprising an amino acid sequence comprising SEQ ID NOs: 180, 223 and 266 and a heavy chain variable region comprising an amino acid sequence comprising SEQ ID NOs: 309, 352 and 395;
(i) a light chain variable region comprising an amino acid sequence comprising SEQ ID NOs: 181, 224 and 267 and a heavy chain variable region comprising an amino acid sequence comprising SEQ ID NOs: 310, 353 and 396;
(j) a light chain variable region comprising an amino acid sequence comprising SEQ ID NOs: 182, 225 and 268 and a heavy chain variable region comprising an amino acid sequence comprising SEQ ID NOs: 311, 354 and 397;
(k) a light chain variable region comprising an amino acid sequence comprising SEQ ID NOs: 183, 226 and 269 and a heavy chain variable region comprising an amino acid sequence comprising SEQ ID NOs: 312, 355 and 398;
(l) a light chain variable region comprising an amino acid sequence comprising SEQ ID NOs: 184, 227 and 270 and a heavy chain variable region comprising an amino acid sequence comprising SEQ ID NOs: 313, 356 and 399;
(m) a light chain variable region comprising an amino acid sequence comprising SEQ ID NOs: 185, 228 and 271 and a heavy chain variable region comprising an amino acid sequence comprising SEQ ID NOs: 314, 357 and 400;
(n) a light chain variable region comprising an amino acid sequence comprising SEQ ID NOs: 186, 229 and 272 and a heavy chain variable region comprising an amino acid sequence comprising SEQ ID NOs: 315, 358 and 401;
(o) a light chain variable region comprising an amino acid sequence comprising SEQ ID NOs: 187, 230 and 273 and a heavy chain variable region comprising an amino acid sequence comprising SEQ ID NOs: 316, 359 and 402;
(p) a light chain variable region comprising an amino acid sequence comprising SEQ ID NOs: 188, 231 and 274 and a heavy chain variable region comprising an amino acid sequence comprising SEQ ID NOs: 317, 360 and 403;
(q) a light chain variable region comprising an amino acid sequence comprising SEQ ID NOs: 189, 232 and 275 and a heavy chain variable region comprising an amino acid sequence comprising SEQ ID NOs: 318, 361 and 404;
(r) a light chain variable region comprising an amino acid sequence comprising SEQ ID NOs: 190, 233 and 276 and a heavy chain variable region comprising an amino acid sequence comprising SEQ ID NOs: 319, 362 and 405;
(s) a light chain variable region comprising an amino acid sequence comprising SEQ ID NOs: 191, 234 and 277 and a heavy chain variable region comprising an amino acid sequence comprising SEQ ID NOs: 320, 363 and 406;
(t) a light chain variable region comprising an amino acid sequence comprising SEQ ID NOs: 192, 235 and 278 and a heavy chain variable region comprising an amino acid sequence comprising SEQ ID NOs: 321, 364 and 407;
(u) a light chain variable region comprising an amino acid sequence comprising SEQ ID NOs: 193, 236 and 279 and a heavy chain variable region comprising an amino acid sequence comprising SEQ ID NOs: 322, 365 and 408;
(v) a light chain variable region comprising an amino acid sequence comprising SEQ ID NOs: 194, 237 and 280 and a heavy chain variable region comprising an amino acid sequence comprising SEQ ID NOs: 323, 366 and 409;
(w) a light chain variable region comprising an amino acid sequence comprising SEQ ID NOs: 195, 238 and 281 and a heavy chain variable region comprising an amino acid sequence comprising SEQ ID NOs: 324, 367 and 410;
(x) a light chain variable region comprising an amino acid sequence comprising SEQ ID NOs: 196, 239 and 282 and a heavy chain variable region comprising an amino acid sequence comprising SEQ ID NOs: 325, 368 and 411;
(y) a light chain variable region comprising an amino acid sequence comprising SEQ ID NOs: 197, 240 and 283 and a heavy chain variable region comprising an amino acid sequence comprising SEQ ID NOs: 326, 369 and 412;
(z) a light chain variable region comprising an amino acid sequence comprising SEQ ID NOs: 198, 241 and 284 and a heavy chain variable region comprising an amino acid sequence comprising SEQ ID NOs: 327, 370 and 413;
(aa) a light chain variable region comprising an amino acid sequence comprising SEQ ID NOs: 199, 242 and 285 and a heavy chain variable region comprising an amino acid sequence comprising SEQ ID NOs: 328, 371 and 414;
(bb) a light chain variable region comprising an amino acid sequence comprising SEQ ID NOs: 200, 243 and 286 and a heavy chain variable region comprising an amino acid sequence comprising SEQ ID NOs: 329, 372 and 415;
(cc) a light chain variable region comprising an amino acid sequence comprising SEQ ID NOs: 201, 244 and 287 and a heavy chain variable region comprising an amino acid sequence comprising SEQ ID NOs: 330, 373 and;
(dd) a light chain variable region comprising an amino acid sequence comprising SEQ ID NOs: 202, 245 and 288 and a heavy chain variable region comprising an amino acid sequence comprising SEQ ID NOs: 331, 374 and 417;
(ee) a light chain variable region comprising an amino acid sequence comprising SEQ ID NOs: 203, 246 and 289 and a heavy chain variable region comprising an amino acid sequence comprising SEQ ID NOs: 332, 375 and 418;
(ff) a light chain variable region comprising an amino acid sequence comprising SEQ ID NOs: 204, 247 and 290 and a heavy chain variable region comprising an amino acid sequence comprising SEQ ID NOs: 333, 376 and 419;
(gg) a light chain variable region comprising an amino acid sequence comprising SEQ ID NOs: 205, 248 and 291 and a heavy chain variable region comprising an amino acid sequence comprising SEQ ID NOs: 334, 377 and 420;
(hh) a light chain variable region comprising an amino acid sequence comprising SEQ ID NOs: 206, 249 and 292 and a heavy chain variable region comprising an amino acid sequence comprising SEQ ID NOs: 335, 378 and 421;
(ii) a light chain variable region comprising an amino acid sequence comprising SEQ ID NOs: 207, 250 and 293 and a heavy chain variable region comprising an amino acid sequence comprising SEQ ID NOs: 336, 379 and 422;
(jj) a light chain variable region comprising an amino acid sequence comprising SEQ ID NOs: 208, 251 and 294 and a heavy chain variable region comprising an amino acid sequence comprising SEQ ID NOs: 337, 380 and 423;
(kk) a light chain variable region comprising an amino acid sequence comprising SEQ ID NOs: 209, 252 and 295 and a heavy chain variable region comprising an amino acid sequence comprising SEQ ID NOs: 338, 381 and 424;
(11) a light chain variable region comprising an amino acid sequence comprising SEQ ID NOs: 210, 253 and 296 and a heavy chain variable region comprising an amino acid sequence comprising SEQ ID NOs: 339, 382 and 425;
(mm) a light chain variable region comprising an amino acid sequence comprising SEQ ID NOs: 211, 254 and 297 and a heavy chain variable region comprising an amino acid sequence comprising SEQ ID NOs: 340, 383 and 426;
(nn) a light chain variable region comprising an amino acid sequence comprising SEQ ID NOs: 212, 255 and 298 and a heavy chain variable region comprising an amino acid sequence comprising SEQ ID NOs: 341, 384 and 427;
(oo) a light chain variable region comprising an amino acid sequence comprising SEQ ID NOs: 213, 256 and 299 and a heavy chain variable region comprising an amino acid sequence comprising SEQ ID NOs: 342, 385 and 428;
(pp) a light chain variable region comprising an amino acid sequence comprising SEQ ID NOs: 214, 257 and 300 and a heavy chain variable region comprising an amino acid sequence comprising SEQ ID NOs: 343, 386 and 429;
(qq) a light chain variable region comprising an amino acid sequence comprising SEQ ID NOs: 215, 258 and 301 and a heavy chain variable region comprising an amino acid sequence comprising SEQ ID NOs: 344, 387 and 430; or
(rr) a light chain variable region comprising an amino acid sequence comprising SEQ ID NOs: 194, 237 and 280 and a heavy chain variable region comprising an amino acid sequence comprising SEQ ID NOs: 335, 378 and 421.

Further disclosed are antibodies comprising:
(a) a light chain variable region having the polypeptide sequence of SEQ ID NO: 2 and a heavy chain variable region having the polypeptide sequence of SEQ ID NO: 4;
(b) a light chain variable region having the polypeptide sequence of SEQ ID NO: 6 and a heavy chain variable region having the polypeptide sequence of SEQ ID NO: 8;
(c) a light chain variable region having the polypeptide sequence of SEQ ID NO: 10 and a heavy chain variable region having the polypeptide sequence of SEQ ID NO: 12;
(d) a light chain variable region having the polypeptide sequence of SEQ ID NO: 14 and a heavy chain variable region having the polypeptide sequence of SEQ ID NO: 16;
(e) a light chain variable region having the polypeptide sequence of SEQ ID NO: 18 and a heavy chain variable region having the polypeptide sequence of SEQ ID NO: 20;
(f) a light chain variable region having the polypeptide sequence of SEQ ID NO: 22 and a heavy chain variable region having the polypeptide sequence of SEQ ID NO: 24;
(g) a light chain variable region having the polypeptide sequence of SEQ ID NO: 26 and a heavy chain variable region having the polypeptide sequence of SEQ ID NO: 28;
(h) a light chain variable region having the polypeptide sequence of SEQ ID NO: 30 and a heavy chain variable region having the polypeptide sequence of SEQ ID NO: 32;
(i) a light chain variable region having the polypeptide sequence of SEQ ID NO: 34 and a heavy chain variable region having the polypeptide sequence of SEQ ID NO: 36;
(j) a light chain variable region having the polypeptide sequence of SEQ ID NO: 38 and a heavy chain variable region having the polypeptide sequence of SEQ ID NO: 40;
(k) a light chain variable region having the polypeptide sequence of SEQ ID NO: 42 and a heavy chain variable region having the polypeptide sequence of SEQ ID NO: 44;
(1) a light chain variable region having the polypeptide sequence of SEQ ID NO: 46 and a heavy chain variable region having the polypeptide sequence of SEQ ID NO: 48;
(m) a light chain variable region having the polypeptide sequence of SEQ ID NO: 50 and a heavy chain variable region having the polypeptide sequence of SEQ ID NO: 52;
(n) a light chain variable region having the polypeptide sequence of SEQ ID NO: 54 and a heavy chain variable region having the polypeptide sequence of SEQ ID NO: 56;
(o) a light chain variable region having the polypeptide sequence of SEQ ID NO: 58 and a heavy chain variable region having the polypeptide sequence of SEQ ID NO: 60;
(p) a light chain variable region having the polypeptide sequence of SEQ ID NO: 62 and a heavy chain variable region having the polypeptide sequence of SEQ ID NO: 64;
(q) a light chain variable region having the polypeptide sequence of SEQ ID NO: 66 and a heavy chain variable region having the polypeptide sequence of SEQ ID NO: 68;
(r) a light chain variable region having the polypeptide sequence of SEQ ID NO: 70 and a heavy chain variable region having the polypeptide sequence of SEQ ID NO: 72;
(s) a light chain variable region having the polypeptide sequence of SEQ ID NO: 74 and a heavy chain variable region having the polypeptide sequence of SEQ ID NO: 76;
(t) a light chain variable region having the polypeptide sequence of SEQ ID NO: 78 and a heavy chain variable region having the polypeptide sequence of SEQ ID NO: 80;
(u) a light chain variable region having the polypeptide sequence of SEQ ID NO: 82 and a heavy chain variable region having the polypeptide sequence of SEQ ID NO: 84;
(v) a light chain variable region having the polypeptide sequence of SEQ ID NO: 86 and a heavy chain variable region having the polypeptide sequence of SEQ ID NO: 88;
(w) a light chain variable region having the polypeptide sequence of SEQ ID NO: 90 and a heavy chain variable region having the polypeptide sequence of SEQ ID NO: 92;
(x) a light chain variable region having the polypeptide sequence of SEQ ID NO: 94 and a heavy chain variable region having the polypeptide sequence of SEQ ID NO: 96;
(y) a light chain variable region having the polypeptide sequence of SEQ ID NO: 98 and a heavy chain variable region having the polypeptide sequence of SEQ ID NO: 100;
(z) a light chain variable region having the polypeptide sequence of SEQ ID NO: 102 and a heavy chain variable region having the polypeptide sequence of SEQ ID NO: 104;
(aa) a light chain variable region having the polypeptide sequence of SEQ ID NO: 106 and a heavy chain variable region having the polypeptide sequence of SEQ ID NO: 108;
(bb) a light chain variable region having the polypeptide sequence of SEQ ID NO: 110 and a heavy chain variable region having the polypeptide sequence of SEQ ID NO: 112;
(cc) a light chain variable region having the polypeptide sequence of SEQ ID NO: 114 and a heavy chain variable region having the polypeptide sequence of SEQ ID NO: 116;
(dd) a light chain variable region having the polypeptide sequence of SEQ ID NO: 118 and a heavy chain variable region having the polypeptide sequence of SEQ ID NO: 120;
(ee) a light chain variable region having the polypeptide sequence of SEQ ID NO: 122 and a heavy chain variable region having the polypeptide sequence of SEQ ID NO: 124;
(ff) a light chain variable region having the polypeptide sequence of SEQ ID NO: 126 and a heavy chain variable region having the polypeptide sequence of SEQ ID NO: 128;
(gg) a light chain variable region having the polypeptide sequence of SEQ ID NO: 130 and a heavy chain variable region having the polypeptide sequence of SEQ ID NO: 132;
(hh) a light chain variable region having the polypeptide sequence of SEQ ID NO: 134 and a heavy chain variable region having the polypeptide sequence of SEQ ID NO: 136;
(ii) a light chain variable region having the polypeptide sequence of SEQ ID NO: 138 and a heavy chain variable region having the polypeptide sequence of SEQ ID NO: 140;
(jj) a light chain variable region having the polypeptide sequence of SEQ ID NO: 142 and a heavy chain variable region having the polypeptide sequence of SEQ ID NO: 144;
(kk) a light chain variable region having the polypeptide sequence of SEQ ID NO: 146 and a heavy chain variable region having the polypeptide sequence of SEQ ID NO: 148;
(11) a light chain variable region having the polypeptide sequence of SEQ ID NO: 150 and a heavy chain variable region having the polypeptide sequence of SEQ ID NO: 152;
(mm) a light chain variable region having the polypeptide sequence of SEQ ID NO: 154 and a heavy chain variable region having the polypeptide sequence of SEQ ID NO: 156;
(nn) a light chain variable region having the polypeptide sequence of SEQ ID NO: 158 and a heavy chain variable region having the polypeptide sequence of SEQ ID NO: 160;
(oo) a light chain variable region having the polypeptide sequence of SEQ ID NO: 162 and a heavy chain variable region having the polypeptide sequence of SEQ ID NO: 164;
(pp) a light chain variable region having the polypeptide sequence of SEQ ID NO: 166 and a heavy chain variable region having the polypeptide sequence of SEQ ID NO: 168;
(qq) a light chain variable region having the polypeptide sequence of SEQ ID NO: 170 and a heavy chain variable region having the polypeptide sequence of SEQ ID NO: 172; or
(rr) a light chain variable region having the polypeptide sequence of SEQ ID NO: 86 and a heavy chain variable region having the polypeptide sequence of SEQ ID NO: 136.

Also disclosed is an isolated monoclonal antibody that binds to human tissue factor pathway inhibitor, wherein the antibody comprises a human heavy chain variable region comprising an amino acid sequence having at least 89%, 90%, 91%, 92%, 93%, 94%, 95%, 96%, 97%, 98%, 99%, or 99.5% identity to an amino acid sequence selected from the group consisting of the amino acid sequences set forth in SEQ ID NO:4, SEQ ID NO:8, SEQ ID NO:12, SEQ ID NO:16, SEQ ID NO:20, SEQ ID NO:24, SEQ ID NO:28, SEQ ID NO:32, SEQ ID NO:36, SEQ ID NO:40, SEQ ID NO:44, SEQ ID NO:48, SEQ ID NO:52, SEQ ID NO:56, SEQ ID NO:60, SEQ ID NO:64, SEQ ID NO:68, SEQ ID NO:72, SEQ ID NO:76, SEQ ID NO:80, SEQ ID NO:84, SEQ ID NO:88, SEQ ID NO:92, SEQ ID NO:96, SEQ ID NO:100, SEQ ID NO:104 SEQ ID NO:108 SEQ ID NO:112, SEQ ID NO:116, SEQ ID NO: 120, SEQ ID NO: 124, SEQ ID NO: 128, SEQ ID NO: 132, SEQ ID NO: 136, SEQ ID NO:140, SEQ ID NO: 144, SEQ ID NO:148, SEQ ID NO: 152, SEQ ID NO:156, SEQ ID NO:160, SEQ ID NO:164, SEQ ID NO:168, and SEQ ID NO:172.

Also disclosed is an isolated monoclonal antibody that binds to human tissue factor pathway inhibitor, wherein the antibody comprises a human light chain variable region comprising an amino acid sequence having at least 93%, 94%, 95%, 96%, 97%, 98%, 99%, or 99.5 % identity to an amino acid sequence selected from the group consisting of the amino acid sequences set forth in SEQ ID NO:2, SEQ ID NO:6, SEQ ID NO:10, SEQ ID NO:14, SEQ ID NO:18, SEQ ID NO:22, SEQ ID NO:26, SEQ ID NO:30, SEQ ID NO:34, SEQ ID NO:38, SEQ ID NO:42, SEQ ID NO:46, SEQ ID NO:50, SEQ ID NO:54, SEQ ID NO:58, SEQ ID NO:62, SEQ ID NO:66, SEQ ID NO:70, SEQ ID NO:74, SEQ ID NO:78, SEQ ID NO:82, SEQ ID NO:86, SEQ ID NO:90, SEQ ID NO:94, SEQ ID NO:98, SEQ ID NO:102, SEQ ID NO:106 SEQ ID NO:110, SEQ ID NO:114, SEQ ID NO:118, SEQ ID NO:122, SEQ ID NO:126, SEQ ID NO:130, SEQ ID NO:134, SEQ ID NO:138, SEQ ID NO:142, SEQ ID NO:146, SEQ ID NO:150, SEQ ID NO:154, SEQ ID NO:158, SEQ ID NO:162, SEQ ID NO:166, and SEQ ID NO: 170.

In addition to relying on the antibody descriptions using the sequence identifiers discussed above, some may also be described by reference to the Fab clones isolated in the experiments described herein. In some cases, the recombinant antibodies comprise the heavy and/or light chain CDR3s of the following clones: TP-2A2, TP-2A5.1, TP-2A6, TP-2A8, TP-2A10, TP-2B1, TP-2B3, TP-2B4, TP-2B8, TP-2B9, TP-2C1, TP-2C7, TP-2D7, TP-2E3, TP-2E5, TP-2F9, TP-2G2, TP-2G4, TP-2G5, TP-2G6, TP-2G7, TP-2G9, TP-2H10, TP-3A2, TP-3A3, TP-3A4, TP-3B3, TP-3B4, TP-3C1, TP-3C2, TP-3C3, TP-3D3, TP-3E1, TP-3F1, TP-3F2, TP-3G1, TP-3G3, TP-3H2, TP-4A7, TP-4A9, TP-4B7, TP-4E8, TP-4G8, or TP-4H8. In some cases, the antibodies further can comprise the CDR2s of these antibodies and still further comprise the CDR1s of these antibodies. In other cases, the antibodies can further comprise any combinations of the CDRs.

Accordingly disclosed are anti-TFPI antibodies comprising: (1) human heavy chain framework regions, a human heavy chain CDR1 region, a human heavy chain CDR2 region, and a human heavy chain CDR3 region, wherein the human heavy chain CDR3 region is the heavy chain CDR3 of TP-2A2, TP-2A5.1, TP-2A6, TP-2A8, TP-2A10, TP-2B1, TP-2B3, TP-2B4, TP-2B8, TP-2B9, TP-2C1, TP-2C7, TP-2D7, TP-2E3, TP-2E5, TP-2F9, TP-2G2, TP-2G4, TP-2G5, TP-2G6, TP-2G7, TP-2G9, TP-2H10, TP-3A2, TP-3A3, TP-3A4, TP-3B3, TP-3B4, TP-3C1, TP-3C2, TP-3C3, TP-3D3, TP-3E1, TP-3F1, TP-3F2, TP-3G1, TP-3G3, TP-3H2, TP-4A7, TP-4A9, TP-4B7, TP-4E8, TP-4G8, or TP-4H8; and (2) human light chain framework regions, a human light chain CDR1 region, a human light chain CDR2 region, and a human light chain CDR3 region, wherein the human light chain CDR3 region is the light chain CDR3 of TP-2A2, TP-2A5.1, TP-2A6, TP-2A8, TP-2A10, TP-2B1, TP-2B3, TP-2B4, TP-2B8, TP-2B9, TP-2C1, TP-2C7, TP-2D7, TP-2E3, TP-2E5, TP-2F9, TP-2G2, TP-2G4, TP-2G5, TP-2G6, TP-2G7, TP-2G9, TP-2H10, TP-3A2, TP-3A3, TP-3A4, TP-3B3, TP-3B4, TP-3C1, TP-3C2, TP-3C3, TP-3D3, TP-3E1, TP-3F1, TP-3F2, TP-3G1, TP-3G3, TP-3H2, TP-4A7, TP-4A9, TP-4B7, TP-4E8, TP-4G8, or TP-4H8, wherein the antibody binds TFPI. The antibody may further comprise the heavy chain CDR2 and/or the light chain CDR2 of TP-2A2, TP-2A5.1, TP-2A6, TP-2A8, TP-2A10, TP-2B1, TP-2B3, TP-2B4, TP-2B8, TP-2B9, TP-2C1, TP-2C7, TP-2D7, TP-2E3, TP-2E5, TP-2F9, TP-2G2, TP-2G4, TP-2G5, TP-2G6, TP-2G7, TP-2G9, TP-2H10, TP-3A2, TP-3A3, TP-3A4, TP-3B3, TP-3B4, TP-3C1, TP-3C2, TP-3C3, TP-3D3, TP-3E1, TP-3F1, TP-3F2, TP-3G1, TP-3G3, TP-3H2, TP-4A7, TP-4A9, TP-4B7, TP-4E8, TP-4G8, or TP-4H8. The antibody may further comprise the heavy chain CDR1 and/or the light chain CDR1 of TP-2A2, TP-2A5.1, TP-2A6, TP-2A8, TP-2A10, TP-2B1, TP-2B3, TP-2B4, TP-2B8, TP-2B9, TP-2C1, TP-2C7, TP-2D7, TP-2E3, TP-2E5, TP-2F9, TP-2G2, TP-2G4, TP-2G5, TP-2G6, TP-2G7, TP-2G9, TP-2H10, TP-3A2, TP-3A3, TP-3A4, TP-3B3, TP-3B4, TP-3C1, TP-3C2, TP-3C3, TP-3D3, TP-3E1, TP-3F1, TP-3F2, TP-3G1, TP-3G3, TP-3H2, TP-4A7, TP-4A9, TP-4B7, TP-4E8, TP-4G8, or TP-4H8.

The CDR1, 2, and/or 3 regions of the engineered antibodies described above can comprise the exact amino acid sequence(s) as those of TP-2A2, TP-2A5.1, TP-2A6, TP-2A8, TP-2A10, TP-2B1, TP-2B3, TP-2B4, TP-2B8, TP-2B9, TP-2C1, TP-2C7, TP-2D7, TP-2E3, TP-2E5, TP-2F9, TP-2G2, TP-2G4, TP-2G5, TP-2G6, TP-2G7, TP-2G9, TP-2H10, TP-3A2, TP-3A3, TP-3A4, TP-3B3, TP-3B4, TP-3C1, TP-3C2, TP-3C3, TP-3D3, TP-3E1, TP-3F1, TP-3F2, TP-3G1, TP-3G3, TP-3H2, TP-4A7, TP-4A9, TP-4B7, TP-4E8, TP-4G8, or TP-4H8 disclosed herein.

However, the ordinarily skilled artisan will appreciate that some deviation from the exact CDR sequences of TP-2A2, TP-2A5.1, TP-2A6, TP-2A8, TP-2A10, TP-2B1, TP-2B3, TP-2B4, TP-2B8, TP-2B9, TP-2C1, TP-2C7, TP-2D7, TP-2E3, TP-2E5, TP-2F9, TP-2G2, TP-2G4, TP-2G5, TP-2G6, TP-2G7, TP-2G9, TP-2H10, TP-3A2, TP-3A3, TP-3A4, TP-3B3, TP-3B4, TP-3C1, TP-3C2, TP-3C3, TP-3D3, TP-3E1, TP-3F1, TP-3F2, TP-3G1, TP-3G3, TP-3H2, TP-4A7, TP-4A9, TP-4B7, TP-4E8, TP-4G8, or TP-4H8 may be possible while still retaining the ability of the antibody to bind TFPI effectively. Accordingly, the engineered antibody may be composed of one or more CDRs that are, for example, at least 90%, 91%, 92%, 93%, 94%, 95%, 96%, 97%, 98%, 99% or 99.5% identical to one or more CDRs of TP-2A2, TP-2A5.1, TP-2A6, TP-2A8, TP-2A10, TP-2B1, TP-2B3, TP-2B4, TP-2B8, TP-2B9, TP-2C1, TP-2C7, TP-2D7, TP-2E3, TP-2E5, TP-2F9, TP-2G2, TP-2G4, TP-2G5, TP-2G6, TP-2G7, TP-2G9, TP-2H10, TP-3A2, TP-3A3, TP-3A4, TP-3B3, TP-3B4, TP-3C1, TP-3C2, TP-3C3, TP-3D3, TP-3E1, TP-3F1, TP-3F2, TP-3G1, TP-3G3, TP-3H2, TP-4A7, TP-4A9, TP-4B7, TP-4E8, TP-4G8, or TP-4H8.

The antibody may be of any of the various classes of antibodies, such as without limitation an IgG1, an IgG2, an IgG3, an IgG4, an IgM, an IgA1, an IgA2, a secretory IgA, an IgD, and an IgE antibody.

In one embodiment, provided is an isolated fully human monoclonal antibody to human tissue factor pathway inhibitor.

Also disclosed is an isolated fully human monoclonal antibody to Kunitz domain 2 of human tissue factor pathway inhibitor.

### Nucleic Acids

Also provided are isolated nucleic acid molecules encoding any of the monoclonal antibodies described above.

### Methods of Prepaying Antibodies to TFPI

The monoclonal antibody may be produced recombinantly by expressing a nucleotide sequence encoding the variable regions of the monoclonal antibody according to the invention in a host cell. With the aid of an expression vector, a nucleic acid containing the nucleotide sequence may be transfected and expressed in a host cell suitable for the production. Accordingly, also disclosed is a method for producing a monoclonal antibody that binds with human TFPI comprising:
(a) transfecting a nucleic acid molecule encoding a monoclonal antibody of the invention into a host cell,
(b) culturing the host cell so to express the monoclonal antibody in the host cell, and optionally
(c) isolating and purifying the produced monoclonal antibody,
wherein the nucleic acid molecule comprises a nucleotide sequence encoding a monoclonal antibody of the present invention.

In one example, to express the antibodies, or antibody fragments thereof, DNAs encoding partial or full-length light and heavy chains obtained by standard molecular biology techniques are inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vectors or, more typically, both genes are inserted into the same expression vector. The antibody genes are inserted into the expression vector by standard methods (e.g., ligation of complementary restriction sites on the antibody gene fragment and vector, or blunt end ligation if no restriction sites are present). The light and heavy chain variable regions of the antibodies described herein can be used to create full-length antibody genes of any antibody isotype by inserting them into expression vectors already encoding heavy chain constant and light chain constant regions of the desired isotype such that the V_{H} segment is operatively linked to the C_{H} segment(s) within the vector and the V_{L} segment is operatively linked to the C_{L} segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (i.e., a signal peptide from a non-immunoglobulin protein).

In addition to the antibody chain encoding genes, the recombinant expression vectors of the invention carry regulatory sequences that control the expression of the antibody chain genes in a host cell. The term "regulatory sequence" is intended to include promoters, enhancers and other expression control elements (e.g., polyadenylation signals) that control the transcription or translation of the antibody chain genes. Such regulatory sequences are described, for example, in Goeddel; Gene Expression Technology. Methods in Enzymology 185, Academic Press, San Diego, Calif. (1990). It will be appreciated by those skilled in the art that the design of the expression vector, including the selection of regulatory sequences may depend on such factors as the choice of the host cell to be transformed, the level of expression of protein desired, etc. Examples of regulatory sequences for mammalian host cell expression include viral elements that direct high levels of protein expression in mammalian cells, such as promoters and/or enhancers derived from cytomegalovirus (CMV), Simian Virus 40 (SV40), adenovirus, (e.g., the adenovirus major late promoter (AdMLP)) and polyoma. Alternatively, nonviral regulatory sequences may be used, such as the ubiquitin promoter or β-globin promoter.

In addition to the antibody chain genes and regulatory sequences, the recombinant expression vectors may carry additional sequences, such as sequences that regulate replication of the vector in host cells (e.g., origins of replication) and selectable marker genes. The selectable marker gene facilitates selection of host cells into which the vector has been introduced (see, e.g., U.S. Pat. Nos. 4,399,216, 4,634,665 and 5,179,017, all by Axel et al.). For example, typically the selectable marker gene confers resistance to drugs, such as G418, hygromycin or methotrexate, on a host cell into which the vector has been introduced. Examples of selectable marker genes include the dihydrofolate reductase (DHFR) gene (for use in dhfr- host cells with methotrexate selection/amplification) and the neo gene (for G418 selection).

For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. Although it is theoretically possible to express the antibodies of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody.

Examples of mammalian host cells for expressing the recombinant antibodies include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, e.g., as described in R. J. Kaufman and P. A. Sharp (1982) Mol. Biol. 159:601-621), NSO myeloma cells, COS cells, HKB11 cells and SP2 cells. When recombinant expression vectors encoding antibody genes are introduced into mammalian host cells, the antibodies are produced by culturing the host cells for a period of time sufficient to allow for expression of the antibody in the host cells or secretion of the antibody into the culture medium in which the host cells are grown. Antibodies can be recovered from the culture medium using standard protein purification methods, such as ultrafiltration, size exclusion chromatography, ion exchange chromatography and centrifugation.

### Use of Partial Antibody Sequences to Express Intact Antibodies

Antibodies interact with target antigens predominantly through amino acid residues that are located in the six heavy and light chain CDRs. For this reason, the amino acid sequences within CDRs are more diverse between individual antibodies than sequences outside of CDRs. See, e.g., Figs. 6 and 7, in which the CDR regions in the light and heavy variable chains, respectively, of the monoclonal antibody according to the present invention are identified. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann, L. et al., 1998, Nature 332:323-327; Jones, P. et al., 1986, Nature 321:522-525; and Queen, C. et al., 1989, Proc. Natl. Acad. Sci. U.S.A. 86:10029-10033). Such framework sequences can be obtained from public DNA databases that include germline antibody gene sequences. These germline sequences will differ from mature antibody gene sequences because they will not include completely assembled variable genes, which are formed by V(D)J joining during B cell maturation. It is not necessary to obtain the entire DNA sequence of a particular antibody in order to recreate an intact recombinant antibody having binding properties similar to those of the original antibody (see WO 99/45962). Partial heavy and light chain sequence spanning the CDR regions is typically sufficient for this purpose. The partial sequence is used to determine which germline variable and joining gene segments contributed to the recombined antibody variable genes. The germline sequence is then used to fill in missing portions of the variable regions. Heavy and light chain leader sequences are cleaved during protein maturation and do not contribute to the properties of the final antibody. For this reason, it is necessary to use the corresponding germline leader sequence for expression constructs. To add missing sequences, cloned cDNA sequences can be combined with synthetic oligonucleotides by ligation or PCR amplification. Alternatively, the entire variable region can be synthesized as a set of short, overlapping, oligonucleotides and combined by PCR amplification to create an entirely synthetic variable region clone. This process has certain advantages such as elimination or inclusion or particular restriction sites, or optimization of particular codons.

The nucleotide sequences of heavy and light chain transcripts are used to design an overlapping set of synthetic oligonucleotides to create synthetic V sequences with identical amino acid coding capacities as the natural sequences. The synthetic heavy and kappa chain sequences can differ from the natural sequences in three ways: strings of repeated nucleotide bases are interrupted to facilitate oligonucleotide synthesis and PCR amplification; optimal translation initiation sites are incorporated according to Kozak's rules (Kozak, 1991, J. Biol. Chem. 266:19867-19870); and HindIII sites are engineered upstream of the translation initiation sites.

For both the heavy and light chain variable regions, the optimized coding, and corresponding non-coding, strand sequences are broken down into 30-50 nucleotide sections at approximately the midpoint of the corresponding non-coding oligonucleotide. Thus, for each chain, the oligonucleotides can be assembled into overlapping double stranded sets that span segments of 150-400 nucleotides. The pools are then used as templates to produce PCR amplification products of 150-400 nucleotides. Typically, a single variable region oligonucleotide set will be broken down into two pools which are separately amplified to generate two overlapping PCR products. These overlapping products are then combined by PCR amplification to form the complete variable region. It may also be desirable to include an overlapping fragment of the heavy or light chain constant region in the PCR amplification to generate fragments that can easily be cloned into the expression vector constructs.

The reconstructed heavy and light chain variable regions are then combined with cloned promoter, translation initiation, constant region, 3' untranslated, polyadenylation, and transcription termination sequences to form expression vector constructs. The heavy and light chain expression constructs can be combined into a single vector, co-transfected, serially transfected, or separately transfected into host cells which are then fused to form a host cell expressing both chains.

Thus, in another aspect, the structural features of a human anti-TFPI antibody, e.g., TP2A8, TP2G6, TP2G7, TP4B7, etc., are used to create structurally related human anti-TFPI antibodies that retain the function of binding to TFPI. More specifically, one or more CDRs of the specifically identified heavy and light chain regions of the monoclonal antibodies of the invention can be combined recombinantly with known human framework regions and CDRs to create additional, recombinantly-engineered, human anti-TFPI antibodies of the invention.

Accordingly, in another aspect, disclosed is a method for preparing an anti-TFPI antibody comprising: preparing an antibody comprising (1) human heavy chain framework regions and human heavy chain CDRs, wherein the human heavy chain CDR3 comprises an amino acid sequence selected from the amino acid sequences of SEQ ID NOs: 388-430 and/or (2) human light chain framework regions and human light chain CDRs, wherein the light chain CDR3 comprises an amino acid sequence selected from the amino acid sequences of SEQ ID NOs: 259-301; wherein the antibody retains the ability to bind to TFPI. In other embodiments, the method is practiced using other CDRs of the invention.

### Pharmaceutical Compositions

Also provided are pharmaceutical compositions comprising therapeutically effective amounts of anti-TFPI monoclonal antibody and a pharmaceutically acceptable carrier. "Pharmaceutically acceptable carrier" is a substance that may be added to the active ingredient to help formulate or stabilize the preparation and causes no significant adverse toxicological effects to the patient. Examples of such carriers are well known to those skilled in the art and include water, sugars such as maltose or sucrose, albumin, salts such as sodium chloride, etc. Other carriers are described for example in Remington's Pharmaceutical Sciences by E. W. Martin. Such compositions will contain a therapeutically effective amount of at least one anti-TFPI monoclonal antibody.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. The composition is preferably formulated for parenteral injection. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. In some cases, it will include isotonic agents, for example, sugars, polyalcohols such as mannitol, sorbitol, or sodium chloride in the composition.

Sterile injectable solutions can be prepared by incorporating the active compound in the required amount in an appropriate solvent with one or a combination of ingredients enumerated above, as required, followed by sterilization micro filtration. Generally, dispersions are prepared by incorporating the active compound into a sterile vehicle that contains a basic dispersion medium and the required other ingredients from those enumerated above. In the case of sterile powders for the preparation of sterile injectable solutions, some methods of preparation are vacuum drying and freeze-drying (lyophilization) that yield a powder of the active ingredient plus any additional desired ingredient from a previously sterile-filtered solution thereof.

### Pharmaceutical Uses

The monoclonal antibody can be used for therapeutic purposes for treating genetic and acquired deficiencies or defects in coagulation. For example, the monoclonal antibodies described above may be used to block the interaction of TFPI with FXa, or to prevent TFPI-dependent inhibition of the TF/FVIIa activity. Additionally, the monoclonal antibody may also be used to restore the TF/FVIIa-driven generation of FXa to bypass the insufficiency of FVIII- or FIX-dependent amplification of FXa.

The monoclonal antibodies have therapeutic use in the treatment of disorders of hemostasis such as thrombocytopenia, platelet disorders and bleeding disorders (e.g., hemophilia A and hemophilia B). Such disorders may be treated by administering a therapeutically effective amount of the anti-TFPI monoclonal antibody to a patient in need thereof. The monoclonal antibodies also have therapeutic use in the treatment of uncontrolled bleeds in indications such as trauma and hemorrhagic stroke. Thus, also provided is a method for shortening the bleeding time comprising administering a therapeutically effective amount of an anti-TFPI monoclonal antibody of the invention to a patient in need thereof.

The antibodies can be used as monotherapy or in combination with other therapies to address a hemostatic disorder. For example, co-administration of one or more antibodies of the invention with a clotting factor such as factor VIIa, factor VIII or factor IX is believed useful for treating hemophilia. The antibodies may be used in the treatment of genetic and acquired deficiencies or defects in coagulation via administering (a) a first amount of a monoclonal antibody that binds to human tissue factor pathway inhibitor and (b) a second amount of factor VIII or factor IX, wherein said first and second amounts together are effective for treating said deficiencies or defects. The antibodies may also be used in the treatment of genetic and acquired deficiencies or defects in coagulation via administering (a) a first amount of a monoclonal antibody that binds to human tissue factor pathway inhibitor and (b) a second amount of factor VIII or factor IX, wherein said first and second amounts together are effective for treating said deficiencies or defects, and further wherein factor VII is not coadministered. Also disclosed is a pharmaceutical composition comprising a therapeutically effective amount of the combination of a monoclonal antibody of the invention and factor VIII or factor IX, wherein the composition does not contain factor VII. "Factor VII" includes factor VII and factor VIIa. These combination therapies are likely to reduce the necessary infusion frequency of the clotting factor. By co-administration or combination therapy is meant administration of the two therapeutic drugs each formulated separately or formulated together in one composition, and, when formulated separately, administered either at approximately the same time or at different times, but over the same therapeutic period.

The pharmaceutical compositions may be parenterally administered to subjects suffering from hemophilia A or B at a dosage and frequency that may vary with the severity of the bleeding episode or, in the case of prophylactic therapy, may vary with the severity of the patient's clotting deficiency.

The compositions may be administered to patients in need as a bolus or by continuous infusion. For example, a bolus administration of an inventive antibody present as a Fab fragment may be in an amount of from 0.0025 to 100 mg/kg body weight, 0.025 to 0.25 mg/kg, 0.010 to 0.10 mg/kg or 0.10-0.50 mg/kg. For continuous infusion, an inventive antibody present as an Fab fragment may be administered at 0.001 to 100 mg/kg body weight/minute, 0.0125 to 1.25 mg/kg/min., 0.010 to 0.75 mg/kg/min., 0.010 to 1.0 mg/kg/min. or 0.10-0.50 mg/kg/min. for a period of 1-24 hours, 1-12 hours, 2-12 hours, 6-12 hours, 2-8 hours, or 1-2 hours. For administration of an inventive antibody present as a full-length antibody (with full constant regions), dosage amounts may be about 1-10 mg/kg body weight, 2-8 mg/kg, or 5-6 mg/kg. Such full-length antibodies would typically be administered by infusion extending for a period of thirty minutes to three hours. The frequency of the administration would depend upon the severity of the condition. Frequency could range from three times per week to once every two or three weeks.

Additionally, the compositions may be administered to patients via subcutaneous injection. For example, a dose of 10 to 100 mg anti-TFPI antibody can be administered to patients via subcutaneous injection weekly, biweekly or monthly.

As used herein, "therapeutically effective amount" means an amount of an anti-TFPI monoclonal antibody or of a combination of such antibody and factor VIII or factor IX that is needed to effectively increase the clotting time in vivo or otherwise cause a measurable benefit in vivo to a patient in need. The precise amount will depend upon numerous factors, including, but not limited to the components and physical characteristics of the therapeutic composition, intended patient population, individual patient considerations, and the like, and can readily be determined by one skilled in the art.

### Examples

### General Materials and Methods

### Example 1 Panning and screening of human antibody library against human TFPI Panning human antibody library against TFPI

Anti-TFPI antibodies were selected by panning phage displayed combinatorial human antibody library HuCal Gold (Rothe et al., J. Mol. Biol., 2008, 376: 1182-1200) against human TFPI (American Diagnostica). Briefly, 200 µl of TFPI (5 µg/ml) was coated on 96-well Maxisorp plates for overnight at 4°C and the plates were then blocked with a PBS buffer containing 5% milk. After the plates were washed with PBS containing 0.01% Tween-20 (PBST), an aliquot of combinatorial human antibody library was added to the TFPI-coated wells and incubated for 2 hours. Unbound phage was washed away with PBST, and the antigen-bound phage was eluted with dithiothreitol, infected and amplified in E. coli strain TG1. The phage was rescued by helper phage for next round of panning. A total of three rounds of panning were conducted and the clones from last two rounds were screened against human TFPI in an ELISA assay.

### Screening antibody clones by antigen-binding in an ELISA

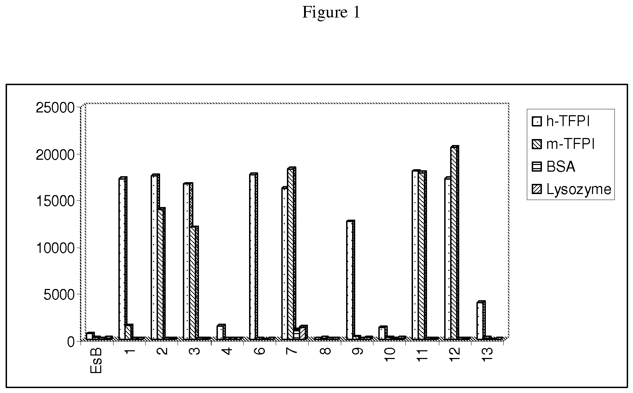

To select antibody clones that bind to human TFPI, Fab genes of the phage clones from the second and third round of panning were subcloned into a bacterial expression vector and expressed in E. coli strain TG1. The bacterial lysate was added to the wells of the human TFPI-coated Maxisorp plates. After washing, HRP-conjugated goat anti-human Fab was used as a detection antibody and the plates were developed by adding AmplexRed (Invitrogen) with hydrogen peroxide. A signal of at least five-fold higher than the background was considered as positive. The cross reactivity of the anti-human TFPI antibodies to mouse TFPI was determined by a similar mouse TFPI-binding ELISA. The plates were coated with mouse TFPI (R&D System), BSA and lysozyme. The later two antigens were used as negative controls. A representative set of data is shown in Fig 1.

### Sequences of anti-TFPI human antibodies

After the panning and screening of the HuCal Gold human antibody library against TFPI, DNA sequencing was performed on the positive antibody clones, resulting in 44 unique antibody sequences (Table 2). Among these antibody sequences, 29 were lambda light chains and 15 were kappa light chains. Our analysis of variable region of heavy chains reveals 28 of VH3, 14 of VH6, 1 of VH1 and 1 of VH5.

**Table 2. Peptide sequence of variable region of 44 anti-TFPI antibodies**

| Clone | VL | VH |
|---|---|---|
| TP-2A2 | | |
| TP-2A5.1 | | |
| TP-2A6 | | |
| TP-2A8 | | |
| TP-2A10 | | |
| TP-2B1 | | |
| TP-2B3 | | |
| TP-2B4 | | |
| TP-2B8 | | |
| TP-2B9 | | |
| TP-2C1 | | |
| TP-2C7 | | |
| TP-2D7 | | |
| TP-2E3 | | |
| TP-2E5 | | |
| TP-2F9 | | |
| TP-2G2 | | |
| TP-2G4 | | |
| TP-2G5 | | |
| TP-2G6 | | |
| TP-2G7 | | |
| TP-2G9 | | |
| TP-2H10 | | |
| TP-3A2 | | |
| TP-3A3 | | |
| TP-3A4 | | |
| TP-3B3 | | |
| TP-3B4 | | |
| TP-3C1 | | |
| TP-3C2 | | |
| TP-3C3 | | |
| TP-3D3 | | |
| TP-3E1 | | |
| TP-3F1 | | |
| TP-3F2 | | |
| TP-3G1 | | |
| TP-3G3 | | |
| TP-3H2 | | |
| TP-4A7 | | |
| TP-4A9 | | |
| TP-4B7 | | |
| TP-4E8 | | |
| TP-4G8 | | |
| TP-4H8 | | |

### Cross-reactivity to mouse TFPI

The above 44 human TFPI-binding clones were also tested for binding to mouse TFPI in ELISA. Nineteen antibodies were found cross-reactive to mouse TFPI. To facilitate the study using mouse hemophilia model, we further characterized these 19 antibodies as well as five antibodies that were specific to human TFPI. A representative set of data is shown in Fig. 1. None of these antibodies bound to BSA or lysozyme in ELISA.

### Example 2 Expression and purification of anti-TFPI antibodies

Anti-TFPI antibodies (as Fab fragments) were expressed and purified from the bacterial strain TG1. Briefly, a single colony of bacterial strain TG1 containing the antibody expression plasmid was picked and grown overnight in 8 ml of 2xYT medium in the presence of 34 µg/ml chloramphenicol and 1% glucose. A volume of 7 ml culture was transferred to 250 ml fresh 2xYT medium containing 34 µg/ml chloramphenicol and 0.1% glucose. After 3 hours of incubation, 0.5 mM IPTG was added to induce Fab expression. The culture was continued overnight at 25 °C. The culture was centrifuged to pellet the bacterial cells. The pellet was then resuspended in a Bug Buster lysis buffer (Novagen). After centrifugation, the supernatant of bacterial lysis was filtered. The Fab fragments were affinity-purified through a Ni-NTA column (Qiagen) according to the manufacturer's instruction.

### Example 3 Determination of EC₅₀ and binding affinity of Anti-TFPI antibodies

Purified Fab antibodies were used to determine EC₅₀ of anti-TFPI antibodies to human or mouse TFPI. EC₅₀ was assessed in an ELISA, similarly as described above. The results were analyzed using SoftMax. The binding affinity of anti-TFPI antibodies was determined in a Biacore assay. Briefly, the antigen, either human or mouse TFPI, was immobilized on the CM5-chips using the amine coupling kit (GE HealthCare) according to the instructions of the manufacturer. The amount of immobilized TFPI was adjusted to the mass of the antigen to give approximate 300 RU. The antibody Fabs were analyzed in mobile phase and at least five different concentrations (0.1, 0.4, 1.6, 6.4 and 25 nM) of the purified antibodies were used in the Biacore assay. The kinetics and binding affinity were calculated using Biacore T100 Evaluation software.

As shown in Table 3, the 24 anti-TFPI Fabs showed various EC₅₀ to human TFPI (0.09 to 792 nM) and mouse TFPI (0.06 to 1035 nM), and the affinity determined by Biacore was accordingly various to human TFPI (1.25 to 1140 nM). In the Biacore study of the Fabs to mouse TFPI, the variation of affinity was smaller (3.08 to 51.8 nM).

**Table 3. The binding activity of 24 antibodies against human or mouse TFPI as determined by ELISA and Biacore (hTFPI: human TFPI; mTFPI: mouse TFPI; Neg: signal was less than two fold of background; ND, not done).**

| | Binding EC₅₀ (nM) | | Affinity (nM) | |
|---|---|---|---|---|
| Antibody clones | hTFPI | mTFPI | hTFPI | mTFPI |
| TP-2A2 | 0.62 | 1035.88 | 6.57 | 29.8 |
| TP-2A5 | 28.64 | 14.54 | 35.4 | 19.6 |
| TP-2A8 | 0.09 | 0.06 | 1.25 | 3.08 |
| TP-2B11 | 11.52 | 0.52 | 21.5 | 16.3 |
| TP-2B3 | 0.84 | 20.18 | 7.40 | 27.0 |
| TP-2C1 | 0.40 | Neg | 2.64 | Neg |
| TP-2C7 | 0.60 | 0.60 | 2.01 | 9.33 |
| TP-2E5 | 791.60 | 202.28 | 115 | 25.2 |
| TP-2G5 | 342.52 | 871.34 | 42.1 | 16.1 |
| TP-2G6 | 0.48 | 5.18 | 5.06 | 46.1 |
| TP-2G7 | 23.48 | Neg | 26.9 | Neg |
| TP-2G9 | 10.80 | 194.42 | 48.5 | 35.7 |
| TP-2H10 | 2.18 | 32.40 | 10.2 | 11.5 |
| TP-3A4 | 42.84 | 326.58 | 21.6 | 23.7 |
| TP-3B4 | 35.76 | 34.62 | 14.1 | 20.4 |
| TP-3C1 | 32.80 | 108.40 | 21.6 | 33.6 |
| TP-3C2 | 59.00 | 956.68 | 17.1 | 28.5 |
| TP-3G1 | 74.40 | 8.68 | 1140 | 49.1 |
| TP-3G3 | 33.60 | 47.06 | 16.0 | 25.7 |
| TP-4A9 | 0.17 | 117.68 | 7.60 | Neg |
| TP-4B7 | 0.74 | 2.64 | 15.8 | 51.8 |
| TP-4E8 | 36.94 | Neg | 35.9 | ND |
| TP-4G8 | 846.92 | Neg | 25.2 | ND |
| TP-4H8 | 72.50 | Neg | 32.2 | ND |

### Example 4 Conversion of anti-TFPI Fab to IgG

All of the identified anti-TFPI antibodies are fully human Fabs that can be feasibly converted to human IgG as therapeutic agent. In this example, however, the selected Fabs were converted to a chimeric antibody containing a mouse IgG constant region, so they are more suitable for testing in mouse model. The variable region of the selected antibodies was grafted into a mammalian expression vector containing mouse constant regions. The fully assembled IgG molecule was then transfected and expressed in HKB11 cells (Mei et al., Mol. Biotechnol., 2006, 34: 165-178). The culture supernatant was collected and concentrated. The anti-TFPI IgG molecules were affinity purified through a Hitrap Protein G column (GE Healthcare) following the manufacturer's instruction.

### Example 5 Selection of anti-TFPI neutralizing antibodies

Anti-TFPI neutralizing antibodies were selected based on their inhibition of the TFPI activity under three experimental conditions. The activity of TFPI was measured using ACTICHROME® TFPI activity assay (American Diagnostica Inc., Stamford, CT), a three stage chromogenic assay to measures the ability of TFPI to inhibit the catalytic activity of the TF/FVIIa complex to activate factor X to factor Xa. The neutralizing activity of the anti-TFPI antibody is proportional to the amount of the restored FXa generation. In the first setting, purified anti-TFPI antibodies were incubated with human or mouse recombinant TFPI (R&D System) at the indicated concentrations. After incubation, the samples were mixed with TF/FVIIa and FX, and the residual activity of the TF/FVIIa complex was then measured using SPECTROZYME® FXa, a highly specific fXa chromogenic substrate. This substrate was cleaved only by FXa generated in the assay, releasing a p-nitroaniline (pNA) chromophore, which was measured at 405 nm. The TFPI activity present in the sample was interpolated from a standard curve constructed using known TFPI activity levels. The assay was performed in an end-point mode. In two other settings, anti-TFPI antibodies were spiked into normal human plasma or hemophilic A plasma, and the restored FXa generation was measured.

### Example 6 Anti-TFPI antibodies shorten clotting time in a diluted prothrombin time (dPT) assay

The dPT assay was carried out essentially as described in Welsch et al., Thrombosis Res., 1991, 64(2): 213-222. Briefly, human normal plasma (FACT, George King Biomedical), human TFPI depleted plasma (American Diagnostica) or hemophilic A plasma (George King Biomedical) were prepared by mixing plasma with 0.1 volumes of control buffer or anti-TFPI antibodies. After incubation for 30 min at 25 °C, plasma samples (100 µl) were combined with 200 µl of appropriately diluted (1:500 dilution) Simplastin (Biometieux) as a source of thromboplastin and the clotting time was determined using a fibrometer STA4 (Stago). Thromboplastin was diluted with PBS or 0.05 M Tris based buffer (pH 7.5) containing 0.1 M sodium chloride, 0.1 % bovine serum albumin and 20 µM calcium chloride.

### Example 7 Anti-TFPI antibodies, alone or in combination with recombinant factor VIII or factor IX, shorten blood clotting time in a ROTEM assay

The ROTEM system (Pentapharm GmbH) included a four-channel instrument, a computer, plasma standards, activators and disposable cups and pins. Thrombelastographic parameters of ROTEM hemostasis systems included: Clotting Time (CT), which reflects the reaction time (the time required to obtain 2 mm amplitude following the initiation of data collection) to initiate blood clotting; Clot Formation Time (CFT) and the alpha angle to reflect clotting propagation, and the maximum amplitude and the maximum elastic modulus to reflect clot firmness. In the ROTEM assay, 300 µl of fresh citrated whole blood or plasma was assessed. All constituents were reconstituted and mixed according to the manufacturer's instructions, with data collection for the time period required for each system. Briefly, samples were mixed by withdrawing/dispensing 300 µl of blood or plasma with an automated pipette into ROTEM cups with 20 µl of CaCl₂ (200 mmol) added, followed immediately by mixing of the sample and initiation of data collection. Data were collected for 2 hr using a computer-controlled (software version 2.96) ROTEM system.

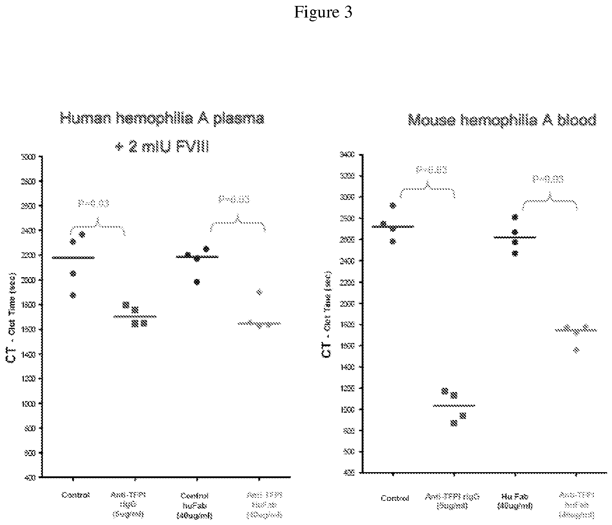

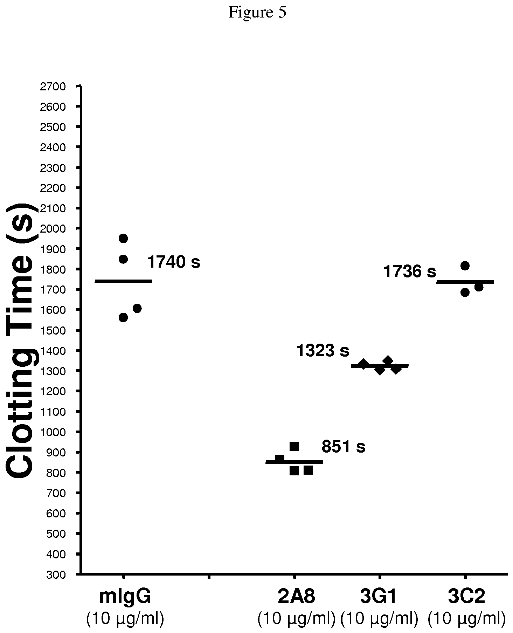

An exemplary result of ROTEM assay in detecting the effect of anti-TFPI antibodies in shortening blood clotting time is shown in Fig. 3 and 5. Fig. 3 shows that TP-2A8-Fab shortened clotting time in human hemophiliac A plasma or mouse hemophiliac A whole blood, alone or in combination with recombinant FVIII, when ROTEM system was initiated with NATEM. Fig. 5 shows that anti-TFPI antibodies in IgG format (TP-2A8, TP-3G1, and TP-3C2) shortened clotting times as compared to a negative control mouse IgG antibody. Based on these results and the understanding in the field, the skilled person would expect that these anti-TFPI antibodies also shorten clotting time in combination with recombinant FIX as compared to these antibodies alone.

### Example 8 In vitro functional activity of anti-TFPI antibodies

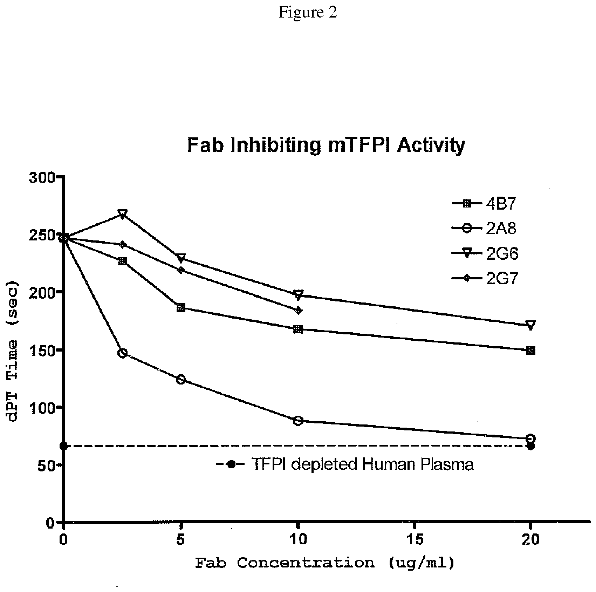

To investigate the TFPI antibodies in blocking the function of TFPI, both chromogenic assay ACTICHROME and diluted prothrombin time (dPT) were used to test the functional activity of the antibodies obtained from the panning and screening. In both assays, a monoclonal rat anti-TFPI antibody (R&D System) was used as positive control and human polyclonal Fab was used as negative control. In the chromogenic assay, eight of the antibodies inhibited more than 50% of TFPI activity compared with the rat monoclonal antibody (Table 4). In dPT assay, all of these eight anti-TFPI Fabs showed a highly inhibitory effect, shortening the clotting time below 80 seconds, and four of the eight Fabs shortened dPT below 70 seconds. Dose-dependence of four of representative clones in shortening the dPT is shown in Fig. 2. However, other human anti-TFPI Fabs with low or no TFPI inhibitory activity also shortened clotting time in dPT. For example, TP-3B4 and TP-2C7, although showing less than 25% inhibitory activity, could shorten the dPT to less than 70 seconds. A simple linear regression analysis of inhibitory activity and dPT suggests significant correlation (p=0.0095) but large variance (R square = 0.258).

**Table 4. The in vitro functional activity of the anti-TFPI antibodies as determined by their inhibition activity in human TFPI assay and dPT assay.**

| clone | % inhibition of hTFPI activity | dPT in hemoA plasma (sec) |
|---|---|---|
| anti-TFPI | 100% | 63.5 |
| TP-2B3 | 100% | 74.0 |
| TP-4B7 | 100% | 53.9 |
| TP-3G1 | 93% | 75.1 |
| TP-3C2 | 92% | 68.9 |
| TP-2G6 | 86% | 62.8 |
| TP-2A8 | 100% | 57.9 |
| TP-2H10 | 63% | 79.5 |
| TP-2G7 | 55% | 72.2 |
| TP-4G8 | 39% | 73.9 |
| TP-2G5 | 36% | 73.2 |
| TP-2A5 | 30% | 70.8 |
| TP-4E8 | 29% | 71.9 |
| TP-4H8 | 28% | 76.5 |
| TP-3B4 | 25% | 69.1 |
| TP-2A2 | 23% | 70.9 |
| TP-2C1 | 21% | 70.9 |
| TP-3G3 | 15% | 70.7 |
| TP-2E5 | 0% | 79.0 |
| TP-3A4 | 0% | 72.3 |
| TP-3C1 | 0% | 72.3 |
| TP-2B11 | 0% | 82.6 |
| TP-2C7 | 0% | 62.5 |
| TP-2G9 | 0% | 82.7 |
| Untreated | 0% | 92.9 |

One of the anti-TFPI Fab, Fab-2A8, was also tested in ROTEM assay in which either human hemophilia A plasma with a low level of factor VIII or mouse hemophilia A whole blood was used. As shown in Fig. 3, comparing a polyclonal rabbit anti-TFPI antibody, Fab-2A8 showed similar activity in human hemophilia A plasma, decreasing clotting time (CT) from 2200 seconds to approximate 1700 seconds. When mouse hemophilia A whole blood was used, the control antibody, rabbit anti-TFPI shortened CT from 2700 seconds to 1000 seconds, whereas Fab-2A8 shorten CT from 2650 seconds to 1700 seconds. These results indicate that Fab-2A8 can significantly shorten clotting time in both human plasma and mouse blood (p = 0.03).

### Example 9 Function of anti-TFPI antibodies following conversion to chimeric IgG

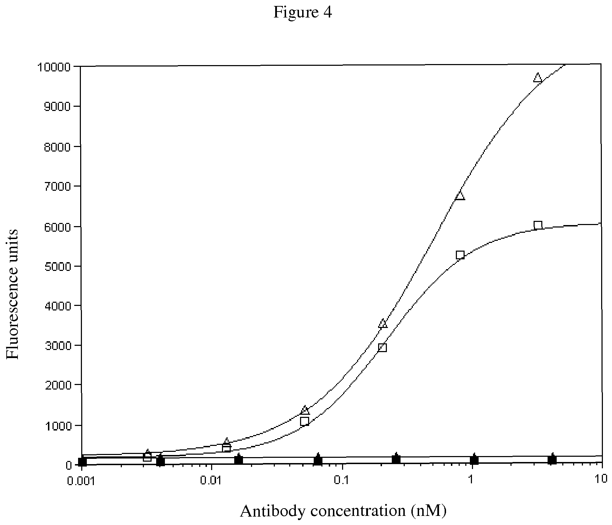

In-vitro assays of factor Xa generation and diluted prothrombin time indicate that at least six of the 24 anti-TFPI Fabs, TP-2A8, TP-2B3, TP-2G6, TP-3C2, TP-3G1 and TP-4B7, could block TFPI function. To facilitate in vivo study using hemophilia A mice, we converted these six anti-TFPI human Fabs into chimeric IgG, using the murine IgG1 isotype. The IgG expression vector was transfected into HKB 11 cells, and the expressed antibody was collected in the culture supernatant and purified on Protein G column. When a representative clone 2G6-Fab was converted to IgG, the 2G6-IgG showed two fold increase of EC₅₀ binding to human TFPI (from 0.48 nM to 0.22 nM) and 10-fold increase to mouse TFPI (from 5.18 nM to 0.51 nM). IgG-2G6 binding to human and mouse TFPI is shown in Fig. 4.

### Example 10 Effect on survival rate in hemophilia A (HemA) mouse tail vein transection model

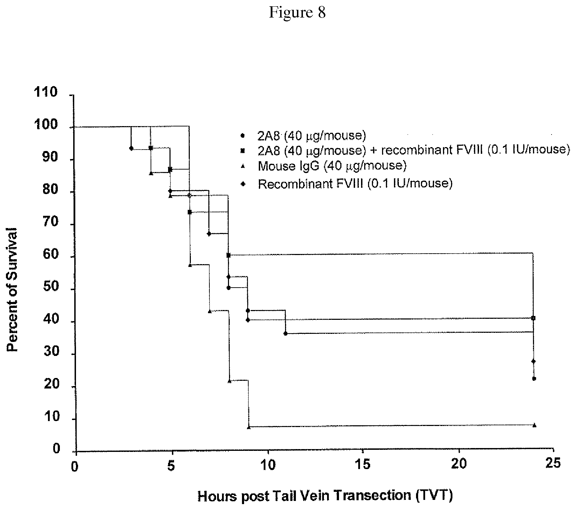

A mouse tail vein transection model has been established for pharmacologic evaluation. This model simulates the wide range of bleeding phenotypes observed between normal individuals and severe hemophiliacs. For these studies, male hemophilia A mice (8 weeks old and 20 to 26 grams) were used. Mice were dosed via tail vein infusion with anti-TFPI monoclonal antibody (40 µg/mouse), alone or together with a clotting factor such as FVIII (0.1 IU/mouse) prior to the injury. At 24 hours post-dosing, the left vein of the tail at 2.7 mm from the tip (in diameter) was transected. Survival was observed over 24 hours post transection. Survival rate was demonstrated to be dose-dependent when given with recombinant FVIII (data not shown). Data shown in Fig. 8 were from two separate studies (n = 15 and n = 10, respectively). The results showed that TP-2A8-IgG significantly prolonged the survival of hemophilia A mice as compared to control mouse IgG; and, in combination with recombinant FVIII, displayed a better survival rate than either agent alone.

### Example 11 Combination of anti-TFPI antibody with recombinant factor VIIa further shortened clotting time and clot formation time

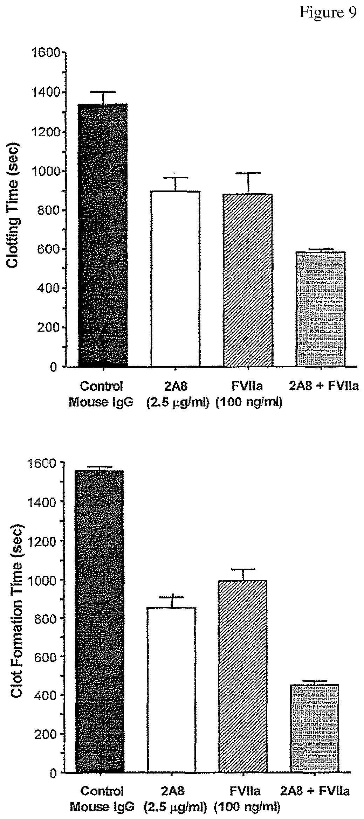

The combined effect of anti-TFPI antibody and recombinant FVIIa (Novo Nordisk) was assessed in a ROTEM system using EXTEM (1:1000 dilution) and mouse hemophilia A whole blood. The indicated amounts of anti-TFPI antibody, TP-2A8-IgG ("2A8"), and recombinant FVIIa ("FVIIa"), were added into 300 µl of citrated mouse hemophilia A whole blood, and blood clotting was initiated using EXTEM system. Fig. 9 shows that addition of TP-2A8-IgG or recombinant FVIIa into mouse hemophilia A whole blood shortened clotting time and clot formation time, respectively. Combination of TP-2A8-IgG and recombinant FVIIa ("2A8 + FVIIa") further shortened clotting time and clot formation time, indicating that combination of anti-TFPI antibody with recombinant FVIIa is useful in the treatment of hemophilia patients with or without inhibitors.

### Example 12 Anti-TFPI antibodies shortened clotting time in FVIII inhibitor-induced human hemophiliac blood

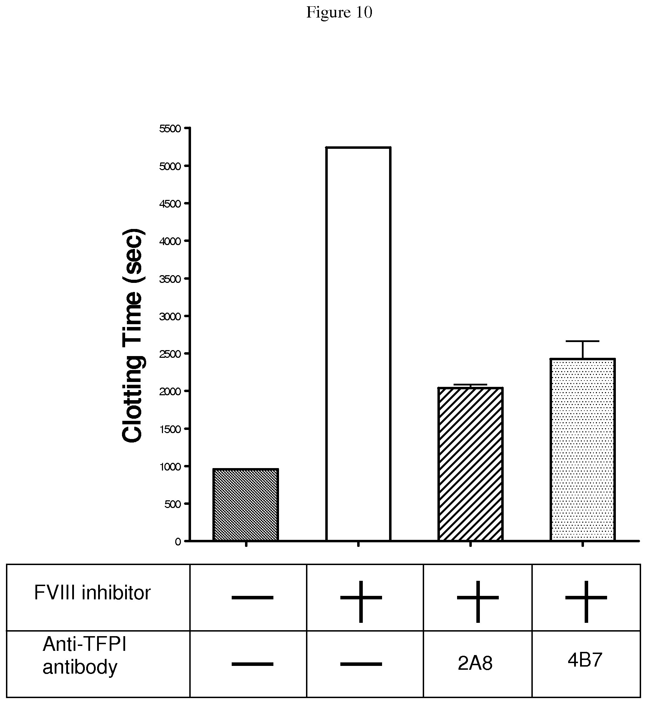

Selected anti-TFPI antibodies, 2A8 and 4B7 were also tested in a ROTEM assay using neutralizing FVIII antibodies to induce hemophilia in whole blood drawn from non-hemophilic patienst. Figure 10 shows that normal human blood has a clotting time of approximately 1000 seconds. In the presence of FVIII neutralizing antibodies (PAH, 100 microgram/mL), the clotting time was prolonged to approximately 5200 seconds. The prolonged clotting time was significantly shortened by addition of an anti-TFPI antibody, 2A8 or 4B7, indicating that anti-TFPI antibody is useful in the treatment of hemophilia patients with inhibitors.

### Example 13 Inhibitory anti-TFPI antibodies bind to Kunitz domain 2 of human TFPI

Western blots and ELISA were used to determine which domain(s) of TFPI of the inhibitory antibodies bind. Recombinant full length human TFPI or TFPI domains were used for these studies. ELISA was similar to Example 3. In the Western Blot, human TFPI or domains were run on 4-12% Bis-Tris SDS PAGE running buffer MES (Invitrogen, Carlsbad, CA) and then transferred to cellulose membrane. After incubation with inhibitory antibodies for 10 min, the membrane was washed three times using SNAPid system (Millipore, Billerica, MA). A HRP conjugated donkey anti-mouse antibody (Pierce, Rockford, IL) at 1 to 10,000 dilution was incubated with the membrane for 10 min. After a similar wash step, the membrane was developed using SuperSignal substrate (Pierce, Rockford, IL). Whereas the control anti-Kunitz domain 1 antibody binds to full length TFPI, truncated TFPI and domains, inhibitory anti-TFPI antibodies only bind to TFPI containing Kunitz domain 2. This indicates that binding to Kunitz domain 2 is necessary for antibody's inhibitory function.

**Table 5. The domains bound by antibodies as determined by Western blots and ELISA**

| | Anti-K1 | mIgG | TP-2A8 | TP-2B3 | TP-2G6 | TP-3C2 | TP-3G1 | TP-4B7 |
|---|---|---|---|---|---|---|---|---|
| Full length | + | - | + | + | + | + | + | + |
| K1+K2+K3 | + | - | + | + | + | + | + | + |
| K1+K2 | + | - | + | + | + | + | + | + |
| K1 | + | | | | - - | - - | - - | - |

## Claims

1. An isolated human monoclonal antibody that binds to human tissue factor pathway inhibitor, wherein the antibody comprises heavy and light chain variable regions comprising:
(a) a light chain variable region comprising an amino acid sequence comprising SEQ ID NOs: 173, 216 and 259 and a heavy chain variable region comprising an amino acid sequence comprising SEQ ID NOs: 302, 345 and 388; or
(b) a light chain variable region comprising an amino acid sequence comprising SEQ ID NOs: 174, 217 and 260 and a heavy chain variable region comprising an amino acid sequence comprising SEQ ID NOs: 303, 346 and 389; or
(c) a light chain variable region comprising an amino acid sequence comprising SEQ ID NOs: 176, 219 and 262 and a heavy chain variable region comprising an amino acid sequence comprising SEQ ID NOs: 305, 348 and 391; or
(d) a light chain variable region comprising an amino acid sequence comprising SEQ ID NOs: 178, 221 and 264 and a heavy chain variable region comprising an amino acid sequence comprising SEQ ID NOs: 307, 350 and 393; or
(e) a light chain variable region comprising an amino acid sequence comprising SEQ ID NOs: 179, 222 and 265 and a heavy chain variable region comprising an amino acid sequence comprising SEQ ID NOs: 308, 351 and 394; or
(f) a light chain variable region comprising an amino acid sequence comprising SEQ ID NOs: 184, 227 and 270 and a heavy chain variable region comprising an amino acid sequence comprising SEQ ID NOs: 313, 356 and 399; or
(g) a light chain variable region comprising an amino acid sequence comprising SEQ ID NOs: 187, 230 and 273 and a heavy chain variable region comprising an amino acid sequence comprising SEQ ID NOs: 316, 359 and 402; or
(h) a light chain variable region comprising an amino acid sequence comprising SEQ ID NOs: 191, 234 and 277 and a heavy chain variable region comprising an amino acid sequence comprising SEQ ID NOs: 320, 363 and 406; or
(i) a light chain variable region comprising an amino acid sequence comprising SEQ ID NOs: 192, 235 and 278 and a heavy chain variable region comprising an amino acid sequence comprising SEQ ID NOs: 321, 364 and 407; or
(j) a light chain variable region comprising an amino acid sequence comprising SEQ ID NOs: 194, 237 and 280 and a heavy chain variable region comprising an amino acid sequence comprising SEQ ID NOs: 323, 366 and 409; or
(k) a light chain variable region comprising an amino acid sequence comprising SEQ ID NOs: 195, 238 and 281 and a heavy chain variable region comprising an amino acid sequence comprising SEQ ID NOs: 324, 367 and 410; or
(I) a light chain variable region comprising an amino acid sequence comprising SEQ ID NOs: 198, 241 and 284 and a heavy chain variable region comprising an amino acid sequence comprising SEQ ID NOs: 327, 370 and 413; or
(m) a light chain variable region comprising an amino acid sequence comprising SEQ ID NOs: 200, 243 and 286 and a heavy chain variable region comprising an amino acid sequence comprising SEQ ID NOs: 329, 372 and 415; or
(n) a light chain variable region comprising an amino acid sequence comprising SEQ ID NOs: 201, 244 and 287 and a heavy chain variable region comprising an amino acid sequence comprising SEQ ID NOs: 330, 373 and 416; or
(o) a light chain variable region comprising an amino acid sequence comprising SEQ ID NOs: 207, 250 and 293 and a heavy chain variable region comprising an amino acid sequence comprising SEQ ID NOs: 336, 379 and 422; or
(p) a light chain variable region comprising an amino acid sequence comprising SEQ ID NOs: 208, 251 and 294 and a heavy chain variable region comprising an amino acid sequence comprising SEQ ID NOs: 337, 380 and 423; or
(q) a light chain variable region comprising an amino acid sequence comprising SEQ ID NOs: 211, 254 and 297 and a heavy chain variable region comprising an amino acid sequence comprising SEQ ID NOs: 340, 383 and 426; or
(r) a light chain variable region comprising an amino acid sequence comprising SEQ ID NOs: 212, 255 and 298 and a heavy chain variable region comprising an amino acid sequence comprising SEQ ID NOs: 341, 384 and 427; or
(s) a light chain variable region comprising an amino acid sequence comprising SEQ ID NOs: 194, 237 and 280 and a heavy chain variable region comprising an amino acid sequence comprising SEQ ID NOs: 335, 378 and 421.

2. The human monoclonal antibody of claim 1, comprising:
(a) a light chain variable region having the polypeptide sequence of SEQ ID NO: 2 and a heavy chain variable region having the polypeptide sequence of SEQ ID NO: 4; or
(b) a light chain variable region having the polypeptide sequence of SEQ ID NO: 6 and a heavy chain variable region having the polypeptide sequence of SEQ ID NO: 8; or
(c) a light chain variable region having the polypeptide sequence of SEQ ID NO: 14 and a heavy chain variable region having the polypeptide sequence of SEQ ID NO: 16; or
(d) a light chain variable region having the polypeptide sequence of SEQ ID NO: 22 and a heavy chain variable region having the polypeptide sequence of SEQ ID NO: 24; or
(e) a light chain variable region having the polypeptide sequence of SEQ ID NO: 26 and a heavy chain variable region having the polypeptide sequence of SEQ ID NO: 28; or
(f) a light chain variable region having the polypeptide sequence of SEQ ID NO: 46 and a heavy chain variable region having the polypeptide sequence of SEQ ID NO: 48; or
(g) a light chain variable region having the polypeptide sequence of SEQ ID NO: 58 and a heavy chain variable region having the polypeptide sequence of SEQ ID NO: 60; or
(h) a light chain variable region having the polypeptide sequence of SEQ ID NO: 74 and a heavy chain variable region having the polypeptide sequence of SEQ ID NO: 76; or
(i) a light chain variable region having the polypeptide sequence of SEQ ID NO: 78 and a heavy chain variable region having the polypeptide sequence of SEQ ID NO: 80; or
(j) a light chain variable region having the polypeptide sequence of SEQ ID NO: 86 and a heavy chain variable region having the polypeptide sequence of SEQ ID NO: 88; or
(k) a light chain variable region having the polypeptide sequence of SEQ ID NO: 90 and a heavy chain variable region having the polypeptide sequence of SEQ ID NO: 92; or
(l) a light chain variable region having the polypeptide sequence of SEQ ID NO: 102 and a heavy chain variable region having the polypeptide sequence of SEQ ID NO: 104; or
(m) a light chain variable region having the polypeptide sequence of SEQ ID NO: 110 and a heavy chain variable region having the polypeptide sequence of SEQ ID NO: 112; or
(n) a light chain variable region having the polypeptide sequence of SEQ ID NO: 114 and a heavy chain variable region having the polypeptide sequence of SEQ ID NO: 116; or
(o) a light chain variable region having the polypeptide sequence of SEQ ID NO: 138 and a heavy chain variable region having the polypeptide sequence of SEQ ID NO: 140; or
(p) a light chain variable region having the polypeptide sequence of SEQ ID NO: 142 and a heavy chain variable region having the polypeptide sequence of SEQ ID NO: 144; or
(q) a light chain variable region having the polypeptide sequence of SEQ ID NO: 154 and a heavy chain variable region having the polypeptide sequence of SEQ ID NO: 156; or
(r) a light chain variable region having the polypeptide sequence of SEQ ID NO: 158 and a heavy chain variable region having the polypeptide sequence of SEQ ID NO: 160; or
(s) a light chain variable region having the polypeptide sequence of SEQ ID NO: 86 and a heavy chain variable region having the polypeptide sequence of SEQ ID NO: 136.

3. A pharmaceutical composition comprising a therapeutically effective amount of the monoclonal antibody of claim 1 or 2 and a pharmaceutically acceptable carrier.

4. A pharmaceutical composition comprising a therapeutically effective amount of the combination of (a) a monoclonal antibody of claim 1 or 2 and (b) factor VIII or factor IX, wherein the composition does not contain factor VII.

5. An isolated nucleic acid molecule encoding a monoclonal antibody of claim 1.

## Patentansprüche

1. Isolierter humaner monoklonaler Antikörper, der an den Inhibitor des menschlichen Gewebefaktorweges bindet, wobei der Antikörper variable Schwere- und Leichtekettenregionen umfasst, welche umfassen:
(a) eine variable Leichtekettenregion, umfassend eine Aminosäuresequenz, die die SEQ ID NO: 173, 216 und 259 umfasst, und eine variable Schwerekettenregion, umfassend eine Aminosäuresequenz, die die SEQ ID NO: 302, 345 und 388 umfasst; oder
(b) eine variable Leichtekettenregion, umfassend eine Aminosäuresequenz, die die SEQ ID NO: 174, 217 und 260 umfasst, und eine variable Schwerekettenregion, umfassend eine Aminosäuresequenz, die die SEQ ID NO: 303, 346 und 389 umfasst; oder
(c) eine variable Leichtekettenregion, umfassend eine Aminosäuresequenz, die die SEQ ID NO: 176, 219 und 262 umfasst, und eine variable Schwerekettenregion, umfassend eine Aminosäuresequenz, die die SEQ ID NO: 305, 348 und 391 umfasst; oder
(d) eine variable Leichtekettenregion, umfassend eine Aminosäuresequenz, die die SEQ ID NO: 178, 221 und 264 umfasst, und eine variable Schwerekettenregion, umfassend eine Aminosäuresequenz, die die SEQ ID NO: 307, 350 und 393 umfasst; oder
(e) eine variable Leichtekettenregion, umfassend eine Aminosäuresequenz, die die SEQ ID NO: 179, 222 und 265 umfasst, und eine variable Schwerekettenregion, umfassend eine Aminosäuresequenz, die die SEQ ID NO: 308, 351 und 394 umfasst; oder
(f) eine variable Leichtekettenregion, umfassend eine Aminosäuresequenz, die die SEQ ID NO: 184, 227 und 270 umfasst, und eine variable Schwerekettenregion, umfassend eine Aminosäuresequenz, die die SEQ ID NO: 313, 356 und 399 umfasst; oder
(g) eine variable Leichtekettenregion, umfassend eine Aminosäuresequenz, die die SEQ ID NO: 187, 230 und 273 umfasst, und eine variable Schwerekettenregion, umfassend eine Aminosäuresequenz, die die SEQ ID NO: 316, 359 und 402 umfasst; oder
(h) eine variable Leichtekettenregion, umfassend eine Aminosäuresequenz, die die SEQ ID NO: 191, 234 und 277 umfasst, und eine variable Schwerekettenregion, umfassend eine Aminosäuresequenz, die die SEQ ID NO: 320, 363 und 406 umfasst; oder
(i) eine variable Leichtekettenregion, umfassend eine Aminosäuresequenz, die die SEQ ID NO: 192, 235 und 278 umfasst, und eine variable Schwerekettenregion, umfassend eine Aminosäuresequenz, die die SEQ ID NO: 321, 364 und 407 umfasst; oder
(j) eine variable Leichtekettenregion, umfassend eine Aminosäuresequenz, die die SEQ ID NO: 194, 237 und 280 umfasst, und eine variable Schwerekettenregion, umfassend eine Aminosäuresequenz, die die SEQ ID NO: 323, 366 und 409 umfasst; oder
(k) eine variable Leichtekettenregion, umfassend eine Aminosäuresequenz, die die SEQ ID NO: 195, 238 und 281 umfasst, und eine variable Schwerekettenregion, umfassend eine Aminosäuresequenz, die die SEQ ID NO: 324, 367 und 410 umfasst; oder
(l) eine variable Leichtekettenregion, umfassend eine Aminosäuresequenz, die die SEQ ID NO: 198, 241 und 284 umfasst, und eine variable Schwerekettenregion, umfassend eine Aminosäuresequenz, die die SEQ ID NO: 327, 370 und 413 umfasst; oder
(m) eine variable Leichtekettenregion, umfassend eine Aminosäuresequenz, die die SEQ ID NO: 200, 243 und 286 umfasst, und eine variable Schwerekettenregion, umfassend eine Aminosäuresequenz, die die SEQ ID NO: 329, 372 und 415 umfasst; oder
(n) eine variable Leichtekettenregion, umfassend eine Aminosäuresequenz, die die SEQ ID NO: 201, 244 und 287 umfasst, und eine variable Schwerekettenregion, umfassend eine Aminosäuresequenz, die die SEQ ID NO: 330, 373 und 416 umfasst; oder
(o) eine variable Leichtekettenregion, umfassend eine Aminosäuresequenz, die die SEQ ID NO: 207, 250 und 293 umfasst, und eine variable Schwerekettenregion, umfassend eine Aminosäuresequenz, die die SEQ ID NO: 336, 379 und 422 umfasst; oder
(p) eine variable Leichtekettenregion, umfassend eine Aminosäuresequenz, die die SEQ ID NO: 208, 251 und 294 umfasst, und eine variable Schwerekettenregion, umfassend eine Aminosäuresequenz, die die SEQ ID NO: 337, 380 und 423 umfasst; oder
(q) eine variable Leichtekettenregion, umfassend eine Aminosäuresequenz, die die SEQ ID NO: 211, 254 und 297 umfasst, und eine variable Schwerekettenregion, umfassend eine Aminosäuresequenz, die die SEQ ID NO: 340, 383 und 426 umfasst; oder
(r) eine variable Leichtekettenregion, umfassend eine Aminosäuresequenz, die die SEQ ID NO: 212, 255 und 298 umfasst, und eine variable Schwerekettenregion, umfassend eine Aminosäuresequenz, die die SEQ ID NO: 341, 384 und 427 umfasst; oder
(s) eine variable Leichtekettenregion, umfassend eine Aminosäuresequenz, die die SEQ ID NO: 194, 237 und 280 umfasst, und eine variable Schwerekettenregion, umfassend eine Aminosäuresequenz, die die SEQ ID NO: 335, 378 und 421 umfasst.

2. Humaner monoklonaler Antikörper nach Anspruch 1, umfassend:
(a) eine variable Leichtekettenregion mit der Polypeptidsequenz von SEQ ID NO: 2 und eine variable Schwerekettenregion mit der Polypeptidsequenz von SEQ ID NO: 4; oder
(b) eine variable Leichtekettenregion mit der Polypeptidsequenz von SEQ ID NO: 6 und eine variable Schwerekettenregion mit der Polypeptidsequenz von SEQ ID NO: 8; oder
(c) eine variable Leichtekettenregion mit der Polypeptidsequenz von SEQ ID NO: 14 und eine variable Schwerekettenregion mit der Polypeptidsequenz von SEQ ID NO: 16; oder
(d) eine variable Leichtekettenregion mit der Polypeptidsequenz von SEQ ID NO: 22 und eine variable Schwerekettenregion mit der Polypeptidsequenz von SEQ ID NO: 24; oder
(e) eine variable Leichtekettenregion mit der Polypeptidsequenz von SEQ ID NO: 26 und eine variable Schwerekettenregion mit der Polypeptidsequenz von SEQ ID NO: 28; oder
(f) eine variable Leichtekettenregion mit der Polypeptidsequenz von SEQ ID NO: 46 und eine variable Schwerekettenregion mit der Polypeptidsequenz von SEQ ID NO: 48; oder
(g) eine variable Leichtekettenregion mit der Polypeptidsequenz von SEQ ID NO: 58 und eine variable Schwerekettenregion mit der Polypeptidsequenz von SEQ ID NO: 60; oder
(h) eine variable Leichtekettenregion mit der Polypeptidsequenz von SEQ ID NO: 74 und eine variable Schwerekettenregion mit der Polypeptidsequenz von SEQ ID NO: 76; oder
(i) eine variable Leichtekettenregion mit der Polypeptidsequenz von SEQ ID NO: 78 und eine variable Schwerekettenregion mit der Polypeptidsequenz von SEQ ID NO: 80; oder
(j) eine variable Leichtekettenregion mit der Polypeptidsequenz von SEQ ID NO: 86 und eine variable Schwerekettenregion mit der Polypeptidsequenz von SEQ ID NO: 88; oder
(k) eine variable Leichtekettenregion mit der Polypeptidsequenz von SEQ ID NO: 90 und eine variable Schwerekettenregion mit der Polypeptidsequenz von SEQ ID NO: 92; oder
(l) eine variable Leichtekettenregion mit der Polypeptidsequenz von SEQ ID NO: 102 und eine variable Schwerekettenregion mit der Polypeptidsequenz von SEQ ID NO: 104; oder
(m) eine variable Leichtekettenregion mit der Polypeptidsequenz von SEQ ID NO: 110 und eine variable Schwerekettenregion mit der Polypeptidsequenz von SEQ ID NO: 112; oder
(n) eine variable Leichtekettenregion mit der Polypeptidsequenz von SEQ ID NO: 114 und eine variable Schwerekettenregion mit der Polypeptidsequenz von SEQ ID NO: 116; oder
(o) eine variable Leichtekettenregion mit der Polypeptidsequenz von SEQ ID NO: 138 und eine variable Schwerekettenregion mit der Polypeptidsequenz von SEQ ID NO: 140; oder
(p) eine variable Leichtekettenregion mit der Polypeptidsequenz von SEQ ID NO: 142 und eine variable Schwerekettenregion mit der Polypeptidsequenz von SEQ ID NO: 144; oder
(q) eine variable Leichtekettenregion mit der Polypeptidsequenz von SEQ ID NO: 154 und eine variable Schwerekettenregion mit der Polypeptidsequenz von SEQ ID NO: 156; oder
(r) eine variable Leichtekettenregion mit der Polypeptidsequenz von SEQ ID NO: 158 und eine variable Schwerekettenregion mit der Polypeptidsequenz von SEQ ID NO: 160; oder
(s) eine variable Leichtekettenregion mit der Polypeptidsequenz von SEQ ID NO: 86 und eine variable Schwerekettenregion mit der Polypeptidsequenz von SEQ ID NO: 136.

3. Pharmazeutische Zusammensetzung, umfassend eine therapeutisch wirksame Menge des monoklonalen Antikörpers nach Anspruch 1 oder 2 und einen pharmazeutisch akzeptablen Träger.

4. Pharmazeutische Zusammensetzung, umfassend eine therapeutisch wirksame Menge der Kombination von (a) einem monoklonalen Antikörper nach Anspruch 1 oder 2 und (b) Faktor VIII oder Faktor IX, wobei die Zusammensetzung keinen Faktor VII enthält.

5. Isoliertes Nukleinsäuremolekül, das einen monoklonalen Antikörper nach Anspruch 1 codiert.

## Revendications

1. Anticorps monoclonal humain isolé qui se lie à un inhibiteur de voie de facteur tissulaire humain, l'anticorps comprenant des régions variables de chaîne lourde et légère comprenant :
(a) une région variable de chaîne légère comprenant une séquence d'acides aminés comprenant SEQ ID NO: 173, 216 et 259 et une région variable de chaîne lourde comprenant une séquence d'acides aminés comprenant SEQ ID NO: 302, 345 et 388 ; ou
(b) une région variable de chaîne légère comprenant une séquence d'acides aminés comprenant SEQ ID NO: 174, 217 et 260 et une région variable de chaîne lourde comprenant une séquence d'acides aminés comprenant SEQ ID NO: 303, 346 et 389 ; ou
(c) une région variable de chaîne légère comprenant une séquence d'acides aminés comprenant SEQ ID NO: 176, 219 et 262 et une région variable de chaîne lourde comprenant une séquence d'acides aminés comprenant SEQ ID NO: 305, 348 et 391 ; ou
(d) une région variable de chaîne légère comprenant une séquence d'acides aminés comprenant SEQ ID NO: 178, 221 et 264 et une région variable de chaîne lourde comprenant une séquence d'acides aminés comprenant SEQ ID NO: 307, 350 et 393 ; ou
(e) une région variable de chaîne légère comprenant une séquence d'acides aminés comprenant SEQ ID NO: 179, 222 et 265 et une région variable de chaîne lourde comprenant une séquence d'acides aminés comprenant SEQ ID NO: 308, 351 et 394 ; ou
(f) une région variable de chaîne légère comprenant une séquence d'acides aminés comprenant SEQ ID NO: 184, 227 et 270 et une région variable de chaîne lourde comprenant une séquence d'acides aminés comprenant SEQ ID NO: 313, 356 et 399 ; ou
(g) une région variable de chaîne légère comprenant une séquence d'acides aminés comprenant SEQ ID NO: 187, 230 et 273 et une région variable de chaîne lourde comprenant une séquence d'acides aminés comprenant SEQ ID NO: 316, 359 et 402 ; ou
(h) une région variable de chaîne légère comprenant une séquence d'acides aminés comprenant SEQ ID NO: 191, 234 et 277 et une région variable de chaîne lourde comprenant une séquence d'acides aminés comprenant SEQ ID NO: 320, 363 et 406 ; ou
(i) une région variable de chaîne légère comprenant une séquence d'acides aminés comprenant SEQ ID NO: 192, 235 et 278 et une région variable de chaîne lourde comprenant une séquence d'acides aminés comprenant SEQ ID NO: 321, 364 et 407 ; ou
(j) une région variable de chaîne légère comprenant une séquence d'acides aminés comprenant SEQ ID NO: 194, 237 et 280 et une région variable de chaîne lourde comprenant une séquence d'acides aminés comprenant SEQ ID NO: 323, 366 et 409 ; ou
(k) une région variable de chaîne légère comprenant une séquence d'acides aminés comprenant SEQ ID NO: 195, 238 et 281 et une région variable de chaîne lourde comprenant une séquence d'acides aminés comprenant SEQ ID NO: 324, 367 et 410 ; ou
(l) une région variable de chaîne légère comprenant une séquence d'acides aminés comprenant SEQ ID NO: 198, 241 et 284 et une région variable de chaîne lourde comprenant une séquence d'acides aminés comprenant SEQ ID NO: 327, 370 et 413 ; ou
(m) une région variable de chaîne légère comprenant une séquence d'acides aminés comprenant SEQ ID NO: 200, 243 et 286 et une région variable de chaîne lourde comprenant une séquence d'acides aminés comprenant SEQ ID NO: 329, 372 et 415 ; ou
(n) une région variable de chaîne légère comprenant une séquence d'acides aminés comprenant SEQ ID NO: 201, 244 et 287 et une région variable de chaîne lourde comprenant une séquence d'acides aminés comprenant SEQ ID NO: 330, 373 et 416 ; ou
(o) une région variable de chaîne légère comprenant une séquence d'acides aminés comprenant SEQ ID NO: 207, 250 et 293 et une région variable de chaîne lourde comprenant une séquence d'acides aminés comprenant SEQ ID NO: 336, 379 et 422 ; ou
(p) une région variable de chaîne légère comprenant une séquence d'acides aminés comprenant SEQ ID NO: 208, 251 et 294 et une région variable de chaîne lourde comprenant une séquence d'acides aminés comprenant SEQ ID NO: 337, 380 et 423 ; ou
(q) une région variable de chaîne légère comprenant une séquence d'acides aminés comprenant SEQ ID NO: 211, 254 et 297 et une région variable de chaîne lourde comprenant une séquence d'acides aminés comprenant SEQ ID NO: 340, 383 et 426 ; ou
(r) une région variable de chaîne légère comprenant une séquence d'acides aminés comprenant SEQ ID NO: 212, 255 et 298 et une région variable de chaîne lourde comprenant une séquence d'acides aminés comprenant SEQ ID NO: 341, 384 et 427 ; ou
(s) une région variable de chaîne légère comprenant une séquence d'acides aminés comprenant SEQ ID NO: 194, 237 et 280 et une région variable de chaîne lourde comprenant une séquence d'acides aminés comprenant SEQ ID NO: 335, 378 et 421.

2. Anticorps monoclonal humain de la revendication 1, comprenant :
(a) une région variable de chaîne légère ayant la séquence polypeptidique de SEQ ID NO: 2 et une région variable de chaîne lourde ayant la séquence polypeptidique de SEQ ID NO: 4 ; ou
(b) une région variable de chaîne légère ayant la séquence polypeptidique de SEQ ID NO: 6 et une région variable de chaîne lourde ayant la séquence polypeptidique de SEQ ID NO: 8 ; ou
(c) une région variable de chaîne légère ayant la séquence polypeptidique de SEQ ID NO: 14 et une région variable de chaîne lourde ayant la séquence polypeptidique de SEQ ID NO: 16 ; ou
(d) une région variable de chaîne légère ayant la séquence polypeptidique de SEQ ID NO: 22 et une région variable de chaîne lourde ayant la séquence polypeptidique de SEQ ID NO: 24 ; ou
(e) une région variable de chaîne légère ayant la séquence polypeptidique de SEQ ID NO: 26 et une région variable de chaîne lourde ayant la séquence polypeptidique de SEQ ID NO: 28 ; ou
(f) une région variable de chaîne légère ayant la séquence polypeptidique de SEQ ID NO: 46 et une région variable de chaîne lourde ayant la séquence polypeptidique de SEQ ID NO: 48 ; ou
(g) une région variable de chaîne légère ayant la séquence polypeptidique de SEQ ID NO: 58 et une région variable de chaîne lourde ayant la séquence polypeptidique de SEQ ID NO: 60 ; ou
(h) une région variable de chaîne légère ayant la séquence polypeptidique de SEQ ID NO: 74 et une région variable de chaîne lourde ayant la séquence polypeptidique de SEQ ID NO: 76 ; ou
(i) une région variable de chaîne légère ayant la séquence polypeptidique de SEQ ID NO: 78 et une région variable de chaîne lourde ayant la séquence polypeptidique de SEQ ID NO: 80 ; ou
(j) une région variable de chaîne légère ayant la séquence polypeptidique de SEQ ID NO: 86 et une région variable de chaîne lourde ayant la séquence polypeptidique de SEQ ID NO: 88 ; ou
(k) une région variable de chaîne légère ayant la séquence polypeptidique de SEQ ID NO: 90 et une région variable de chaîne lourde ayant la séquence polypeptidique de SEQ ID NO: 92 ; ou
(l) une région variable de chaîne légère ayant la séquence polypeptidique de SEQ ID NO: 102 et une région variable de chaîne lourde ayant la séquence polypeptidique de SEQ ID NO: 104 ; ou
(m) une région variable de chaîne légère ayant la séquence polypeptidique de SEQ ID NO: 110 et une région variable de chaîne lourde ayant la séquence polypeptidique de SEQ ID NO: 112 ; ou
(n) une région variable de chaîne légère ayant la séquence polypeptidique de SEQ ID NO: 114 et une région variable de chaîne lourde ayant la séquence polypeptidique de SEQ ID NO: 116 ; ou
(o) une région variable de chaîne légère ayant la séquence polypeptidique de SEQ ID NO: 138 et une région variable de chaîne lourde ayant la séquence polypeptidique de SEQ ID NO: 140 ; ou
(p) une région variable de chaîne légère ayant la séquence polypeptidique de SEQ ID NO: 142 et une région variable de chaîne lourde ayant la séquence polypeptidique de SEQ ID NO: 144 ; ou
(q) une région variable de chaîne légère ayant la séquence polypeptidique de SEQ ID NO: 154 et une région variable de chaîne lourde ayant la séquence polypeptidique de SEQ ID NO: 156 ; ou
(r) une région variable de chaîne légère ayant la séquence polypeptidique de SEQ ID NO: 158 et une région variable de chaîne lourde ayant la séquence polypeptidique de SEQ ID NO: 160 ; ou
(s) une région variable de chaîne légère ayant la séquence polypeptidique de SEQ ID NO: 86 et une région variable de chaîne lourde ayant la séquence polypeptidique de SEQ ID NO: 136.

3. Composition pharmaceutique comprenant une quantité thérapeutiquement efficace de l'anticorps monoclonal de la revendication 1 ou 2 et un véhicule pharmaceutiquement acceptable.

4. Composition pharmaceutique comprenant une quantité thérapeutiquement efficace de la combinaison de (a) un anticorps monoclonal de la revendication 1 ou 2 et (b) le facteur VIII ou le facteur IX, la composition ne contenant pas de facteur VII.

5. Molécule d'acide nucléique isolée codant pour un anticorps monoclonal de la revendication 1.


### SEQUENCE LISTING

<110> Bayer HealthCare LLC
   Wang, zhuozhi
   Murphy, John
   Pan, Junliang
   Jiang, Haiyan
   Liu, Bing
<120> Monoclonal antibodies against tissue factor pathway inhibitor (TFPI)
<130> MSB-7329 PCT
<150> US 61/085,980
   <151> 2008-08-04
<160> 430
<170> Patent In version 3.5
<210> 1
   <211> 330
   <212> DNA
   <213> Homo sapiens
<400> 1
   gatatcgaac tgacccagcc gccttcagtg agcgttgcac caggtcagac cgcgcgtatc      60
   tcgtgtagcg gcgataatat tcgtacttat tatgttcatt ggtaccagca gaaacccggg     120
   caggcgccag ttgttgtgat ttatggtgat tctaagcgtc cctcaggcat cccggaacgc     180
   tttagcggat ccaacagcgg caacaccgcg accctgacca ttagcggcac tcaggcggaa     240
   gacgaagcgg attattattg ccagtcttat gattctgagg ctgattctga ggtgtttggc     300
   ggcggcacga agttaaccgt tcttggccag                                      330
<210> 2
   <211> 110
   <212> PRT
   <213> Homo sapiens
<400> 2
   Asp Ile Glu Leu Thr Gln Pro Pro Ser Val Ser Val Ala Pro Gly Gln
   1               5                   10                  15
   Thr Ala Arg Ile Ser Cys Ser Gly Asp Asn Ile Arg Thr Tyr Tyr Val
               20                  25                  30
   His Trp Tyr Gln Gln Lys Pro Gly Gln Ala Pro Val Val Val Ile Tyr
           35                  40                  45
   Gly Asp Ser Lys Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ser Tyr Asp Ser Glu Ala Asp Ser
                   85                  90                  95
   Glu Val Phe Gly Gly Gly Thr Lys Leu Thr Val Leu Gly Gln
               100                 105                 110
<210> 3
   <211> 365
   <212> DNA
   <213> Homo sapiens
<400> 3
   caggtgcaat tggtggaaag cggcggcggc ctggtgcaac cgggcggcag cctgcgtctg      60
   agctgcgcgg cctccggatt taccttttct aataatgcta tgaattgggt gcgccaagcc     120
   cctgggaagg gtctcgagtg ggtgagcact atctcttatg atggtagcaa tacctattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgtcaggct     300
   ggtggttgga cttattctta tactgatgtt tggggccaag gcaccctggt gacggttagc     360
   tcagc                                                                 365
<210> 4
   <211> 121
   <212> PRT
   <213> Homo sapiens
<400> 4
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Asn
               20                  25                  30
   Ala Met Asn Trp Val Arg Gln Ala Pro Gly Lys Gly Leu Glu Trp Val
           35                  40                  45
   Ser Thr Ile Ser Tyr Asp Gly Ser Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gln Ala Gly Gly Trp Thr Tyr Ser Tyr Thr Asp Val Trp Gly
               100                 105                 110
   Gln Gly Thr Leu Val Thr Val Ser Ser
           115                 120
<210> 5
   <211> 327
   <212> DNA
   <213> Homo sapiens
<400> 5
   gatatcgaac tgacccagcc gccttcagtg agcgttgcac caggtcagac cgcgcgtatc 60
   tcgtgtagcg gcgataatat tcctgagaag tatgttcatt ggtaccagca gaaacccggg 120
   caggcgccag ttcttgtgat tcatggtgat aataatcgtc cctcaggcat cccggaacgc 180
   tttagcggat ccaacagcgg caacaccgcg accctgacca ttagcggcac tcaggcggaa 240
   gacgaagcgg attattattg ccagtctttt gatgctggtt cttattttgt gtttggcggc 300
   ggcacgaagt taaccgttct tggccag 327
<210> 6
   <211> 109
   <212> PRT
   <213> Homo sapiens
<400> 6
   Asp Ile Glu Leu Thr Gln Pro Pro Ser Val Ser Val Ala Pro Gly Gln
   1               5                   10                  15
   Thr Ala Arg Ile Ser Cys Ser Gly Asp Asn Ile Pro Glu Lys Tyr Val
               20                  25                  30
   His Trp Tyr Gln Gln Lys Pro Gly Gln Ala Pro Val Leu Val Ile His
           35                  40                  45
   Gly Asp Asn Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ser Phe Asp Ala Gly Ser Tyr Phe
                   85                  90                  95
   Val Phe Gly Gly Gly Thr Lys Leu Thr Val Leu Gly Gln
               100                 105
<210> 7
   <211> 353
   <212> DNA
   <213> Homo sapiens
<400> 7
   caggtgcaat tggtggaaag cggcggcggc ctggtgcaac cgggcggcag cctgcgtctg 60
   agctgcgcgg cctccggatt taccttttct tcttatggtt cttgggtgcg ccaagcccct 120
   gggaagggtc tcgagtgggt gagcgttatc tctggttctg gtagctctac ctattatgcg 180
   gatagcgtga aaggccgttt taccatttca cgtgataatt cgaaaaacac cctgtatctg 240
   caaatgaaca gcctgcgtgc ggaagatacg gccgtgtatt attgcgcgcg tgttaatatt 300
   tctactcatt ttgatgtttg gggccaaggc accctggtga cggttagctc agc 353
<210> 8
   <211> 117
   <212> PRT
   <213> Homo sapiens
<400> 8
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Gly Ser Trp Val Arg Gln Ala Pro Gly Lys Gly Leu Glu Trp Val Ser
           35                  40                  45
   Val Ile Ser Gly Ser Gly Ser Ser Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Val Asn Ile Ser Thr His Phe Asp Val Trp Gly Gln Gly Thr Leu
               100                 105                 110
   Val Thr Val Ser Ser
           115
<210> 9
   <211> 327
   <212> DNA
   <213> Homo sapiens
<400> 9
   gatatcgaac tgacccagcc gccttcagtg agcgttgcac caggtcagac cgcgcgtatc      60
   tcgtgtagcg gcgataagat tggttctaag tatgtttatt ggtaccagca gaaacccggg     120
   caggcgccag ttcttgtgat ttatgattct aatcgtccct caggcatccc ggaacgcttt     180
   agcggatcca acagcggcaa caccgcgacc ctgaccatta gcggcactca ggcggaagac     240
   gaagcggatt attattgcgc ttcttatgat tctatttatt cttattgggt gtttggcggc     300
   ggcacgaagt taaccgttct tggccag                                         327
<210> 10
   <211> 109
   <212> PRT
   <213> Homo sapiens
<400> 10
   Asp Ile Glu Leu Thr Gln Pro Pro Ser Val Ser Val Ala Pro Gly Gln
   1               5                   10                  15
   Thr Ala Arg Ile Ser Cys Ser Gly Asp Lys Ile Gly Ser Lys Tyr Val
               20                  25                  30
   Tyr Trp Tyr Gln Gln Lys Pro Gly Gln Ala Pro Val Leu Val Ile Tyr
           35                  40                  45
   Asp Ser Asn Arg Pro Ser Gly Ile Pro Glu Arg Phe Ser Gly Ser Asn
       50                  55                  60
   Ser Gly Asn Thr Ala Thr Leu Thr Ile Ser Gly Thr Gln Ala Glu Asp
   65                  70                  75                  80
   Glu Ala Asp Tyr Tyr Cys Ala Ser Tyr Asp Ser Ile Tyr Ser Tyr Trp
                   85                  90                  95
   Val Phe Gly Gly Gly Thr Lys Leu Thr Val Leu Gly Gln
               100                 105
<210> 11
   <211> 365
   <212> DNA
   <213> Homo sapiens
<400> 11
   caggtgcaat tggtggaaag cggcggcggc ctggtgcaac cgggcggcag cctgcgtctg      60
   agctgcgcgg cctccggatt taccttttct cgttatgcta tgtcttgggt gcgccaagcc     120
   cctgggaagg gtctcgagtg ggtgagctct atcatttctt cttctagcga gacctattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgtcttatg     300
   ggttatggtc attattatcc ttttgattat tggggccaag gcaccctggt gacggttagc     360
   tcagc                                                                 365
<210> 12
   <211> 121
   <212> PRT
   <213> Homo sapiens
<400> 12
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ala Met Ser Trp Val Arg Gln Ala Pro Gly Lys Gly Leu Glu Trp Val
           35                  40                  45
   Ser Ser Ile Ile Ser Ser Ser Ser Glu Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Leu Met Gly Tyr Gly His Tyr Tyr Pro Phe Asp Tyr Trp Gly
               100                 105                 110
   Gln Gly Thr Leu Val Thr Val Ser Ser
           115                 120
<210> 13
   <211> 324
   <212> DNA
   <213> Homo sapiens
<400> 13
   gatatcgaac tgacccagcc gccttcagtg agcgttgcac caggtcagac cgcgcgtatc 60
   tcgtgtagcg gcgataatct tcgtaattat tatgctcatt ggtaccagca gaaacccggg 120
   caggcgccag ttgttgtgat ttattatgat aataatcgtc cctcaggcat cccggaacgc 180
   tttagcggat ccaacagcgg caacaccgcg accctgacca ttagcggcac tcaggcggaa 240
   gacgaagcgg attattattg ccagtcttgg gatgatggtg ttcctgtgtt tggcggcggc 300
   acgaagttaa ccgttcttgg ccag 324
<210> 14
   <211> 108
   <212> PRT
   <213> Homo sapiens
<400> 14
   Asp Ile Glu Leu Thr Gln Pro Pro Ser Val Ser Val Ala Pro Gly Gln
   1               5                   10                  15
   Thr Ala Arg Ile Ser Cys Ser Gly Asp Asn Leu Arg Asn Tyr Tyr Ala
               20                  25                  30
   His Trp Tyr Gln Gln Lys Pro Gly Gln Ala Pro Val Val Val Ile Tyr
           35                  40                  45
   Tyr Asp Asn Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ser Trp Asp Asp Gly Val Pro Val
                   85                  90                  95
   Phe Gly Gly Gly Thr Lys Leu Thr Val Leu Gly Gln
               100                 105
<210> 15
   <211> 353
   <212> DNA
   <213> Homo sapiens
<400> 15
   caggtgcaat tggtggaaag cggcggcggc ctggtgcaac cgggcggcag cctgcgtctg      60
   agctgcgcgg cctccggatt tacctttcgt tcttatggta tgtcttgggt gcgccaagcc     120
   cctgggaagg gtctcgagtg ggtgagctct atccgtggtt cttctagctc tacctattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgtaagtat     300
   cgttattggt ttgattattg gggccaaggc accctggtga cggttagctc agc            353
<210> 16
   <211> 117
   <212> PRT
   <213> Homo sapiens
<400> 16
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Arg Ser Tyr
               20                  25                  30
   Gly Met Ser Trp Val Arg Gln Ala Pro Gly Lys Gly Leu Glu Trp Val
           35                  40                  45
   Ser Ser Ile Arg Gly Ser Ser Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Lys Tyr Arg Tyr Trp Phe Asp Tyr Trp Gly Gln Gly Thr Leu
               100                 105                 110
   Val Thr Val Ser Ser
           115
<210> 17
   <211> 327
   <212> DNA
   <213> Homo sapiens
<400> 17
   gatatcgaac tgacccagcc gccttcagtg agcgttgcac caggtcagac cgcgcgtatc       60
   tcgtgtagcg gcgataagct tggtaagaag tatgttcatt ggtaccagca gaaacccggg      120
   caggcgccag ttcttgtgat ttatggtgat gataagcgtc cctcaggcat cccggaacgc      180
   tttagcggat ccaacagcgg caacaccgcg accctgacca ttagcggcac tcaggcggaa      240
   gacgaagcgg attattattg ccaggcttgg ggttctattt ctcgttttgt gtttggcggc      300
   ggcacgaagt taaccgttct tggccag                                          327
<210> 18
   <211> 109
   <212> PRT
   <213> Homo sapiens
<400> 18
   Asp Ile Glu Leu Thr Gln Pro Pro Ser Val Ser Val Ala Pro Gly Gln
   1               5                   10                  15
   Thr Ala Arg Ile Ser Cys Ser Gly Asp Lys Leu Gly Lys Lys Tyr Val
               20                  25                  30
   His Trp Tyr Gln Gln Lys Pro Gly Gln Ala Pro Val Leu Val Ile Tyr
           35                  40                  45
   Gly Asp Asp Lys Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ala Trp Gly Ser Ile Ser Arg Phe
                   85                  90                  95
   Val Phe Gly Gly Gly Thr Lys Leu Thr Val Leu Gly Gln
               100                 105
<210> 19
   <211> 365
   <212> DNA
   <213> Homo sapiens
<400> 19
   caggtgcaat tggtggaaag cggcggcggc ctggtgcaac cgggcggcag cctgcgtctg      60
   agctgcgcgg cctccggatt tacctttact tcttattcta tgaattgggt gcgccaagcc     120
   cctgggaagg gtctcgagtg ggtgagcgct atctcttata ctggtagcaa tacccattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgtgctttt     300
   cttggttata aggagtctta ttttgatatt tggggccaag gcaccctggt gacggttagc     360
   tcagc                                                                 365
<210> 20
   <211> 121
   <212> PRT
   <213> Homo sapiens
<400> 20
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Thr Ser Tyr
   20 25 30
   Ser Met Asn Trp Val Arg Gln Ala Pro Gly Lys Gly Leu Glu Trp Val
   35 40 45
   Ser Ala Ile Ser Tyr Thr Gly Ser Asn Thr His Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Ala Phe Leu Gly Tyr Lys Glu Ser Tyr Phe Asp Ile Trp Gly
   100 105 110
   Gln Gly Thr Leu Val Thr Val Ser Ser
   115 120
<210> 21
   <211> 330
   <212> DNA
   <213> Homo sapiens
<400> 21
   gatatcgaac tgacccagcc gccttcagtg agcgttgcac caggtcagac cgcgcgtatc 60
   tcgtgtagcg gcgataatct tggtaataag tatgctcatt ggtaccagca gaaacccggg 120
   caggcgccag ttcttgtgat ttattatgat aataagcgtc cctcaggcat cccggaacgc 180
   tttagcggat ccaacagcgg caacaccgcg accctgacca ttagcggcac tcaggcggaa 240
   gacgaagcgg attattattg ccagtcttgg actcctggtt ctaatactat ggtgtttggc 300
   ggcggcacga ggttaaccgt tcttggccag 330
<210> 22
   <211> 110
   <212> PRT
   <213> Homo sapiens
<400> 22
   Asp Ile Glu Leu Thr Gln Pro Pro Ser Val Ser Val Ala Pro Gly Gln
   1 5 10 15
   Thr Ala Arg Ile Ser Cys Ser Gly Asp Asn Leu Gly Asn Lys Tyr Ala
   20 25 30
   His Trp Tyr Gln Gln Lys Pro Gly Gln Ala Pro Val Leu Val Ile Tyr
   35 40 45
   Tyr Asp Asn Lys Arg Pro Ser Gly Ile Pro Glu Arg Phe Ser Gly Ser
   50 55 60
   Asn Ser Gly Asn Thr Ala Thr Leu Thr Ile Ser Gly Thr Gln Ala Glu
   65 70 75 80
   Asp Glu Ala Asp Tyr Tyr Cys Gln Ser Trp Thr Pro Gly Ser Asn Thr
   85 90 95
   Met Val Phe Gly Gly Gly Thr Arg Leu Thr Val Leu Gly Gln
   100 105 110
<210> 23
   <211> 350
   <212> DNA
   <213> Homo sapiens
<400> 23
   caggtgcaat tggtggaaag cggcggcggc ctggtgcaac cgggcggcag cctgcgtctg 60
   agctgcgcgg cctccggatt taccttttct tcttattcta tgtcttgggt gcgccaagcc 120
   tctgggaagg gtctcgagtg ggtgagctct atcaagggtt ctggtagcaa tacctattat 180
   gcggatagcg tgaaaggccg ttttaccatt tcacgtgata attcgaaaaa caccctgtat 240
   ctgcaaatga acagcctgcg tgcggaagat acggccgtgt attattgcgc gcgtaatggt 300
   ggtcttattg atgtttgggg ccaaggcacc ctggtgacgg ttagctcagc 350
<210> 24
   <211> 116
   <212> PRT
   <213> Homo sapiens
<400> 24
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ser Met Ser Trp Val Arg Gln Ala Ser Gly Lys Gly Leu Glu Trp Val
   35 40 45
   Ser Ser Ile Lys Gly Ser Gly Ser Asn Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asn Gly Gly Leu Ile Asp Val Trp Gly Gln Gly Thr Leu Val
   100 105 110
   Thr Val Ser Ser
   115
<210> 25
   <211> 330
   <212> DNA
   <213> Homo sapiens
<400> 25
   gatatcgtgc tgacccagag cccggcgacc ctgagcctgt ctccgggcga acgtgcgacc 60
   ctgagctgca gagcgagcca gaatattggt tctaattatc tggcttggta ccagcagaaa 120
   ccaggtcaag caccgcgtct attaatttat ggtgcttcta ctcgtgcaac tggggtcccg 180
   gcgcgtttta acggctctgg atccggcacg gattttaccc tgaccattag cagcctggaa 240
   cctgaagact ttgcggttta ttattgccag cagcttaatt ctattcctgt tacctttggc 300
   cagggtacga aagttgaaat taaacgtacg 330
<210> 26
   <211> 110
   <212> PRT
   <213> Homo sapiens
<400> 26
   Asp Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Asn Ile Gly Ser Asn
               20                  25                  30
   Tyr Leu Ala Trp Tyr Gln Gln Lys Pro Gly Gln Ala Pro Arg Leu Leu
           35                  40                  45
   Ile Tyr Gly Ala Ser Thr Arg Ala Thr Gly Val Pro Ala Arg Phe Asn
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln Leu Asn Ser Ile Pro
                   85                  90                  95
   Val Thr Phe Gly Gln Gly Thr Lys Val Glu Ile Lys Arg Thr
               100                 105                 110
<210> 27
   <211> 374
   <212> DNA
   <213> Homo sapiens
<400> 27
   caggtgcaat tgcaacagtc tggtccgggc ctggtgaaac cgagccaaac cctgagcctg      60
   acctgtgcga tttccggaga tagcgtgagc tctaattctg ctgcttgggg ttggattcgc     120
   cagtctcctg ggcgtggcct cgagtggctg ggcatgatct attatcgtag caagtggtat     180
   aactcttatg cggtgagcgt gaaaagccgg attaccatca acccggatac ttcgaaaaac     240
   cagtttagcc tgcaactgaa cagcgtgacc ccggaagata cggccgtgta ttattgcgcg     300
   cgtactatgt ctaagtatgg tggtcctggt atggatgttt ggggccaagg caccctggtg     360
   acggttagct cagc                                                       374
<210> 28
   <211> 124
   <212> PRT
   <213> Homo sapiens
<400> 28
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Gly Trp Ile Arg Gln Ser Pro Gly Arg Gly Leu Glu
           35                  40                  45
   Trp Leu Gly Met Ile Tyr Tyr Arg Ser Lys Trp Tyr Asn Ser Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Thr Met Ser Lys Tyr Gly Gly Pro Gly Met Asp
               100                 105                 110
   Val Trp Gly Gln Gly Thr Leu Val Thr Val Ser Ser
           115                 120
<210> 29
   <211> 330
   <212> DNA
   <213> Homo sapiens
<400> 29
   gatatcgaac tgacccagcc gccttcagtg agcgttgcac caggtcagac cgcgcgtatc      60
   tcgtgtagcg gcgatgctct tggtacttat tatgcttatt ggtaccagca gaaacccggg     120
   caggcgccag ttcttgtgat ttatggtgat atgaatcgtc cctcaggcat cccggaacgc     180
   tttagcggat ccaacagcgg caacaccgcg accctgacca ttagcggcac tcaggcggaa     240
   gacgaagcgg attattattg ccagtcttat gatgctggtg ttaagcctgc tgtgtttggc     300
   ggcggcacga agttaaccgt tcttggccag                                      330
<210> 30
   <211> 110
   <212> PRT
   <213> Homo sapiens
<400> 30
   Asp Ile Glu Leu Thr Gln Pro Pro Ser Val Ser Val Ala Pro Gly Gln
   1               5                   10                  15
   Thr Ala Arg Ile Ser Cys Ser Gly Asp Ala Leu Gly Thr Tyr Tyr Ala
               20                  25                  30
   Tyr Trp Tyr Gln Gln Lys Pro Gly Gln Ala Pro Val Leu Val Ile Tyr
           35                  40                  45
   Gly Asp Met Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ser Tyr Asp Ala Gly Val Lys Pro
                   85                  90                  95
   Ala Val Phe Gly Gly Gly Thr Lys Leu Thr Val Leu Gly Gln
               100                 105                 110
<210> 31
   <211> 355
   <212> DNA
   <213> Homo sapiens
<400> 31
   caggtgcaat tggtggaaag cggcggcggc ctggtgcacc gggcggcagc ctgcgtctga      60
   gctgcgcggc ctccggattt accttttcta attattctat gacttgggtg cgccaagccc     120
   ctgggaaggg tctcgagtgg gtgagcggta tctcttataa tggtagcaat acctattatg     180
   cggatagcgt gaaaggccgt tttaccattt cacgtgataa ttcgaaaaac accctgtatc     240
   tgcaaatgaa cagcctgcgt gcggaagata cggccgtgta ttattgcgcg cgtatttatt     300
   atatgaatct tcttgctggt tggggccaag gcaccctggt gacggttagc tcagc          355
<210> 32
   <211> 118
   <212> PRT
   <213> Homo sapiens
<400> 32
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Asn Tyr
   20 25 30
   Ser Met Thr Trp Val Arg Gln Ala Pro Gly Lys Gly Leu Glu Trp Val
   35 40 45
   Ser Gly Ile Ser Tyr Asn Gly Ser Asn Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Ile Tyr Tyr Met Asn Leu Leu Ala Gly Trp Gly Gln Gly Thr
   100 105 110
   Leu Val Thr Val Ser Ser
   115
<210> 33
   <211> 327
   <212> DNA
   <213> Homo sapiens
<400> 33
   gatatcgaac tgacccagcc gccttcagtg agcgttgcac caggtcagac cgcgcgtatc 60
   tcgtgtagcg gcgataatct tcgtggttat tatgcttctt ggtaccagca gaaacccggg 120
   caggcgccag ttcttgtgat ttatgaggat aataatcgtc cctcaggcat cccggaacgc 180
   tttagcggat ccaacagcgg caacaccgcg accctgacca ttagcggcac tcaggcggaa 240
   gacgaagcgg attattattg ccagtcttgg gattctcctt atgttcatgt gtttggcggc 300
   ggcacgaagt taaccgttct tggccag 327
<210> 34
   <211> 109
   <212> PRT
   <213> Homo sapiens
<400> 34
   Asp Ile Glu Leu Thr Gln Pro Pro Ser Val Ser Val Ala Pro Gly Gln
   1 5 10 15
   Thr Ala Arg Ile Ser Cys Ser Gly Asp Asn Leu Arg Gly Tyr Tyr Ala
   20 25 30
   Ser Trp Tyr Gln Gln Lys Pro Gly Gln Ala Pro Val Leu Val Ile Tyr
   35 40 45
   Glu Asp Asn Asn Arg Pro Ser Gly Ile Pro Glu Arg Phe Ser Gly Ser
   50 55 60
   Asn Ser Gly Asn Thr Ala Thr Leu Thr Ile Ser Gly Thr Gln Ala Glu
   65 70 75 80
   Asp Glu Ala Asp Tyr Tyr Cys Gln Ser Trp Asp Ser Pro Tyr Val His
   85 90 95
   Val Phe Gly Gly Gly Thr Lys Leu Thr Val Leu Gly Gln
   100 105
<210> 35
   <211> 353
   <212> DNA
   <213> Homo sapiens
<400> 35
   caggtgcaat tggttcagag cggcgcggaa gtgaaaaaac cgggcgcgag cgtgaaagtg 60
   agctgcaaag cctccggata tacctttact ggtaattcta tgcattgggt ccgccaagcc 120
   cctgggcagg gtctcgagtg gatgggcact atctttccgt atgatggcac tacgaagtac 180
   gcgcagaagt ttcagggccg ggtgaccatg acccgtgata ccagcattag caccgcgtat 240
   atggaactga gcagcctgcg tagcgaagat acggccgtgt attattgcgc gcgtggtgtt 300
   cattcttatt ttgattattg gggccaaggc accctggtga cggttagctc agc 353
<210> 36
   <211> 117
   <212> PRT
   <213> Homo sapiens
<400> 36
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Gly Asn
   20 25 30
   Ser Met His Trp Val Arg Gln Ala Pro Gly Gln Gly Leu Glu Trp Met
   35 40 45
   Gly Thr Ile Phe Pro Tyr Asp Gly Thr Thr Lys Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Ser Leu Arg Ser Glu Asp Thr Ala Val Tyr Tyr Cys
   85 90 95
   Ala Arg Gly Val His Ser Tyr Phe Asp Tyr Trp Gly Gln Gly Thr Leu
   100 105 110
   Val Thr Val Ser Ser
   115
<210> 37
   <211> 327
   <212> DNA
   <213> Homo sapiens
<400> 37
   gatatccaga tgacccagag cccgtctagc ctgagcgcga gcgtgggtga tcgtgtgacc 60
   attacctgca gagcgagcca gtctattcgt tcttatctgg cttggtacca gcagaaacca 120
   ggtaaagcac cgaaactatt aatttataag gcttctaatt tgcaaagcgg ggtcccgtcc 180
   cgttttagcg gctctggatc cggcactgat tttaccctga ccattagcag cctgcaacct 240
   gaagactttg cggtttatta ttgccatcag tattctgatt ctcctgttac ctttggccag 300
   ggtacgaaag ttgaaattaa acgtacg 327
<210> 38
   <211> 109
   <212> PRT
   <213> Homo sapiens
<400> 38
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Arg Ser Tyr
               20                  25                  30
   Leu Ala Trp Tyr Gln Gln Lys Pro Gly Lys Ala Pro Lys Leu Leu Ile
           35                  40                  45
   Tyr Lys Ala Ser Asn Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys His Gln Tyr Ser Asp Ser Pro Val
                   85                  90                  95
   Thr Phe Gly Gln Gly Thr Lys Val Glu Ile Lys Arg Thr
                       100                 105
<210> 39
   <211> 365
   <212> DNA
   <213> Homo sapiens
<400> 39
   caggtgcaat tgcaacagtc tggtccgggc ctggtgaaac cgagccaaac cctgagcctg      60
   acctgtgcga tttccggaga tagcgtgagc tctaattctg ctgcttgggg ttggattcgc     120
   cagtctcctg ggcgtggcct cgagtggctg ggcatgatct atcatcgtag caagtggtat     180
   aacgattatg cggtgagcgt gaaaagccgg attaccatca acccggatac ttcgaaaaac     240
   cagtttagcc tgcaactgaa cagcgtgacc ccggaagata cggccgtgta ttattgcgcg     300
   cgttattctt ctattggtca tatggattat tggggccaag gcaccctggt gacggttagc     360
   tcagc                                                                 365
<210> 40
   <211> 121
   <212> PRT
   <213> Homo sapiens
<400> 40
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Gly Trp Ile Arg Gln Ser Pro Gly Arg Gly Leu Glu
           35                  40                  45
   Trp Leu Gly Met Ile Tyr His Arg Ser Lys Trp Tyr Asn Asp Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Tyr Ser Ser Ile Gly His Met Asp Tyr Trp Gly
               100                 105                 110
   Gln Gly Thr Leu Val Thr Val Ser Ser
           115                 120
<210> 41
   <211> 330
   <212> DNA
   <213> Homo sapiens
<400> 41
   gatatcgaac tgacccagcc gccttcagtg agcgttgcac caggtcagac cgcgcgtatc        60
   tcgtgtagcg gcgattctat tggttcttat tatgctcatt ggtaccagca gaaacccggg       120
   caggcgccag ttcttgtgat ttattatgat tctaagcgtc cctcaggcat cccggaacgc       180
   tttagcggat ccaacagcgg caacaccgcg accctgacca ttagcggcac tcaggcggaa       240
   gacgaagcgg attattattg ccaggcttat actggtcagt ctatttctcg tgtgtttggc       300
   ggcggcacga agttaaccgt tcttggccag                                        330
<210> 42
   <211> 110
   <212> PRT
   <213> Homo sapiens
<400> 42
   Asp Ile Glu Leu Thr Gln Pro Pro Ser Val Ser Val Ala Pro Gly Gln
   1               5                   10                  15
   Thr Ala Arg Ile Ser Cys Ser Gly Asp Ser Ile Gly Ser Tyr Tyr Ala
               20                  25                  30
   His Trp Tyr Gln Gln Lys Pro Gly Gln Ala Pro Val Leu Val Ile Tyr
           35                  40                  45
   Tyr Asp Ser Lys Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ala Tyr Thr Gly Gln Ser Ile Ser
                   85                  90                  95
   Arg Val Phe Gly Gly Gly Thr Lys Leu Thr Val Leu Gly Gln
               100                 105                 110
<210> 43
   <211> 353
   <212> DNA
   <213> Homo sapiens
<400> 43
   caggtgcaat tggtggaaag cggcggcggc ctggtgcaac cgggcggcag cctgcgtctg     60
   agctgcgcgg cctccggatt taccttttct ccttatgtta tgtcttgggt gcgccaagcc    120
   cctgggaagg gtctcgagtg ggtgagctct atctcttctt cttctagcaa tacctattat    180
   gcggatagcg tgaaaggccg ttttaccatt tcacgtgata attcgaaaaa caccctgtat    240
   ctgcaaatga acagcctgcg tgcggaagat acggccgtgt attattgcgc gcgtggtgat    300
   tcttatatgt atgatgtttg gggccaaggc accctggtga cggttagctc agc          353
<210> 44
   <211> 117
   <212> PRT
   <213> Homo sapiens
<400> 44
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Val Met Ser Trp Val Arg Gln Ala Pro Gly Lys Gly Leu Glu Trp Val
           35                  40                  45
   Ser Ser Ile Ser Ser Ser Ser Ser Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Asp Ser Tyr Met Tyr Asp Val Trp Gly Gln Gly Thr Leu
               100                 105                 110
   Val Thr Val Ser Ser
           115
<210> 45
   <211> 327
   <212> DNA
   <213> Homo sapiens
<400> 45
   gatatccaga tgacccagag cccgtctagc ctgagcgcga gcgtgggtga tcgtgtgacc 60
   attacctgca gagcgagcca ggatattcgt aataatctgg cttggtacca gcagaaacca 120
   ggtaaagcac cgaaactatt aatttatgct gcttcttctt tgcaaagcgg ggtcccgtcc 180
   cgttttagcg gctctggatc cggcactgat tttaccctga ccattagcag cctgcaacct 240
   gaagactttg cggtttatta ttgccagcag cgtaatggtt ttcctcttac ctttggccag 300
   ggtacgaaag ttgaaattaa acgtacg 327
<210> 46
   <211> 109
   <212> PRT
   <213> Homo sapiens
<400> 46
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Asn
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Arg Asn Gly Phe Pro Leu
                   85                  90                  95
   Thr Phe Gly Gln Gly Thr Lys Val Glu Ile Lys Arg Thr
               100                 105
<210> 47
   <211> 365
   <212> DNA
   <213> Homo sapiens
<400> 47
   caggtgcaat tgcaacagtc tggtccgggc ctggtgaaac cgagccaaac cctgagcctg      60
   acctgtgcga tttccggaga tagcgtgagc tctaattctg ctgcttgggg ttggattcgc     120
   cagtctcctg ggcgtggcct cgagtggctg ggcattatct attatcgtag caagtggtat     180
   aaccattatg cggtgagcgt gaaaagccgg attaccatca acccggatac ttcgaaaaac     240
   cagtttagcc tgcaactgaa cagcgtgacc ccggaagata cggccgtgta ttattgcgcg     300
   cgttctaatt ggtctggtta ttttgattat tggggccaag gcaccctggt gacggttagc     360
   tcagc                                                                 365
<210> 48
   <211> 121
   <212> PRT
   <213> Homo sapiens
<400> 48
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Gly Trp Ile Arg Gln Ser Pro Gly Arg Gly Leu Glu
           35                  40                  45
   Trp Leu Gly Ile Ile Tyr Tyr Arg Ser Lys Trp Tyr Asn His Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Ser Asn Trp Ser Gly Tyr Phe Asp Tyr Trp Gly
               100                 105                 110
   Gln Gly Thr Leu Val Thr Val Ser Ser
           115                 120
<210> 49
   <211> 342
   <212> DNA
   <213> Homo sapiens
<400> 49
   gatatcgtga tgacccagag cccactgagc ctgccagtga ctccgggcga gcctgcgagc      60
   attagctgca gaagcagcca aagcctgctt cattctaatg gctatactta tctgtcttgg     120
   taccttcaaa aaccaggtca aagcccgcag ctattaattt atcttggttc taatcgtgcc     180
   agtggggtcc cggatcgttt tagcggctct ggatccggca ccgattttac cctgaaaatt     240
   agccgtgtgg aagctgaaga cgtgggcgtg tattattgcc agcagtatga taatgctcct     300
   attacctttg gccagggtac gaaagttgaa attaaacgta cg                        342
<210> 50
   <211> 114
   <212> PRT
   <213> Homo sapiens
<400> 50
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu His Ser
   20 25 30
   Asn Gly Tyr Thr Tyr Leu Ser Trp Tyr Leu Gln Lys Pro Gly Gln Ser
   35 40 45
   Pro Gln Leu Leu Ile Tyr Leu Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Gln Gln Tyr
   85 90 95
   Asp Asn Ala Pro Ile Thr Phe Gly Gln Gly Thr Lys Val Glu Ile Lys
   100 105 110
   Arg Thr
<210> 51
   <211> 371
   <212> DNA
   <213> Homo sapiens
<400> 51
   caggtgcaat tgcaacagtc tggtccgggc ctggtgaaac cgagccaaac cctgagcctg 60
   acctgtgcga tttccggaga tagcgtgagc tctaattctg ctgcttgggg ttggattcgc 120
   cagtctcctg ggcgtggcct cgagtggctg ggccttatct attatcgtag caagtggtat 180
   aacgattatg cggtgagcgt gaaaagccgg attaccatca acccggatac ttcgaaaaac 240
   cagtttagcc tgcaactgaa cagcgtgacc ccggaagata cggccgtgta ttattgcgcg 300
   cgttttggtg atactaatcg taatggtact gatgtttggg gccaaggcac cctggtgacg 360
   gttagctcag c 371
<210> 52
   <211> 123
   <212> PRT
   <213> Homo sapiens
<400> 52
   Gln Val Gln Leu Gln Gln Ser Gly Pro Gly Leu Val Lys Pro Ser Gln
   1 5 10 15
   Thr Leu Ser Leu Thr Cys Ala Ile Ser Gly Asp Ser Val Ser Ser Asn
   20 25 30
   Ser Ala Ala Trp Gly Trp Ile Arg Gln Ser Pro Gly Arg Gly Leu Glu
   35 40 45
   Trp Leu Gly Leu Ile Tyr Tyr Arg Ser Lys Trp Tyr Asn Asp Tyr Ala
   50 55 60
   Val Ser Val Lys Ser Arg Ile Thr Ile Asn Pro Asp Thr Ser Lys Asn
   65 70 75 80
   Gln Phe Ser Leu Gln Leu Asn Ser Val Thr Pro Glu Asp Thr Ala Val
   85 90 95
   Tyr Tyr Cys Ala Arg Phe Gly Asp Thr Asn Arg Asn Gly Thr Asp Val
   100 105 110
   Trp Gly Gln Gly Thr Leu Val Thr Val Ser Ser
   115 120
<210> 53
   <211> 339
   <212> DNA
   <213> Homo sapiens
<400> 53
   gatatcgcac tgacccagcc agcttcagtg agcggctcac caggtcagag cattaccatc 60
   tcgtgtacgg gtactagcag cgatattggt ggttataatt atgtgtcttg gtaccagcag 120
   catcccggga aggcgccgaa acttatgatt tatggtgtta attatcgtcc ctcaggcgtg 180
   agcaaccgtt ttagcggatc caaaagcggc aacaccgcga gcctgaccat tagcggcctg 240
   caagcggaag acgaagcgga ttattattgc tcttctgctg ataagtttac tatgtctatt 300
   gtgtttggcg gcggcacgaa gttaaccgtt cttggccag 339
<210> 54
   <211> 113
   <212> PRT
   <213> Homo sapiens
<400> 54
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Ile Gly Gly Tyr
               20                  25                  30
   Asn Tyr Val Ser Trp Tyr Gln Gln His Pro Gly Lys Ala Pro Lys Leu
           35                  40                  45
   Met Ile Tyr Gly Val Asn Tyr Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Ala Asp Lys Phe
                   85                  90                  95
   Thr Met Ser Ile Val Phe Gly Gly Gly Thr Lys Leu Thr Val Leu Gly
               100                 105                 110
   Gln
<210> 55
   <211> 306
   <212> DNA
   <213> Homo sapiens
<400> 55
   gacctgtgcg atttccggag atagcgtgag ctctaattct gctgcttggg gttggattcg      60
   ccagtctcct gggcgtggcc tcgagtggct gggcatgatc tattatcgta gcaagtggta     120
   taacgattat gcggtgagcg tgaaaagccg gattaccatc aacccggata cttcgaaaaa     180
   ccagtttagc ctgcaactga acagcgtgac cccggaagat acggccgtgt attattgcgc     240
   gcgtgttaat cagtatactt cttctgatta ttggggccaa ggcaccctgg tgacggttag     300
   ctcagc                                                                306
<210> 56
   <211> 121
   <212> PRT
   <213> Homo sapiens
<400> 56
   Gln Val Gln Leu Gln Gln Ser Gly Pro Gly Leu Val Lys Pro Ser Gln
   1 5 10 15
   Thr Leu Ser Leu Thr Cys Ala Ile Ser Gly Asp Ser Val Ser Ser Asn
   20 25 30
   Ser Ala Ala Trp Gly Trp Ile Arg Gln Ser Pro Gly Arg Gly Leu Glu
   35 40 45
   Trp Leu Gly Met Ile Tyr Tyr Arg Ser Lys Trp Tyr Asn Asp Tyr Ala
   50 55 60
   Val Ser Val Lys Ser Arg Ile Thr Ile Asn Pro Asp Thr Ser Lys Asn
   65 70 75 80
   Gln Phe Ser Leu Gln Leu Asn Ser Val Thr Pro Glu Asp Thr Ala Val
   85 90 95
   Tyr Tyr Cys Ala Arg Val Asn Gln Tyr Thr Ser Ser Asp Tyr Trp Gly
   100 105 110
   Gln Gly Thr Leu Val Thr Val Ser Ser
   115 120
<210> 57
   <211> 327
   <212> DNA
   <213> Homo sapiens
<400> 57
   gatatccaga tgacccagag cccgtctagc ctgagcgcga gcgtgggtga tcgtgtgacc 60
   attacctgca gagcgagcca gcctatttat aattctctgt cttggtacca gcagaaacca 120
   ggtaaagcac cgaaactatt aatttatggt gtttctaatt tgcaaagcgg ggtcccgtcc 180
   cgttttagcg gctctggatc cggcactgat tttaccctga ccattagcag cctgcaacct 240
   gaagactttg cggtttatta ttgccttcag gttgataatc ttcctattac ctttggccag 300
   ggtacgaaag ttgaaattaa acgtacg 327
<210> 58
   <211> 109
   <212> PRT
   <213> Homo sapiens
<400> 58
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Pro Ile Tyr Asn Ser
               20                  25                  30
   Leu Ser Trp Tyr Gln Gln Lys Pro Gly Lys Ala Pro Lys Leu Leu Ile
           35                  40                  45
   Tyr Gly Val Ser Asn Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Leu Gln Val Asp Asn Leu Pro Ile
                   85                  90                  95
   Thr Phe Gly Gln Gly Thr Lys Val Glu Ile Lys Arg Thr
               100                 105
<210> 59
   <211> 374
   <212> DNA
   <213> Homo sapiens
<400> 59
   caggtgcaat tgcaacagtc tggtccgggc ctggtgaaac cgagccaaac cctgagcctg       60
   acctgtgcga tttccggaga tagcgtgagc tctaattctg ctgcttggtc ttggattcgc      120
   cagtctcctg ggcgtggcct cgagtggctg ggcatgatct tttatcgtag caagtggaat      180
   aacgattatg cggtgagcgt gaaaagccgg attaccatca acccggatac ttcgaaaaac      240
   cagtttagcc tgcaactgaa cagcgtgacc ccggaagata cggccgtgta ttattgcgcg      300
   cgtgttaatg ctaatggtta ttatgcttat gttgatcttt ggggccaagg caccctggtg      360
   acggttagct cagc                                                        374
<210> 60
   <211> 124
   <212> PRT
   <213> Homo sapiens
<400> 60
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Ser Trp Ile Arg Gln Ser Pro Gly Arg Gly Leu Glu
   35 40 45
   Trp Leu Gly Met Ile Phe Tyr Arg Ser Lys Trp Asn Asn Asp Tyr Ala
   50 55 60
   Val Ser Val Lys Ser Arg Ile Thr Ile Asn Pro Asp Thr Ser Lys Asn
   65 70 75 80
   Gln Phe Ser Leu Gln Leu Asn Ser Val Thr Pro Glu Asp Thr Ala Val
   85 90 95
   Tyr Tyr Cys Ala Arg Val Asn Ala Asn Gly Tyr Tyr Ala Tyr Val Asp
   100 105 110
   Leu Trp Gly Gln Gly Thr Leu Val Thr Val Ser Ser
   115 120
<210> 61
   <211> 330
   <212> DNA
   <213> Homo sapiens
<400> 61
   gatatcgtgc tgacccagag cccggcgacc ctgagcctgt ctccgggcga acgtgcgacc 60
   ctgagctgca gagcgagcca gtctgtttct tctcagtatc tggcttggta ccagcagaaa 120
   ccaggtcaag caccgcgtct attaatttat gctgcttctt ctcgtgcaac tggggtcccg 180
   gcgcgtttta gcggctctgg atccggcacg gattttaccc tgaccattag cagcctggaa 240
   cctgaagact ttgcggttta ttattgccag caggattcta atcttcctgc tacctttggc 300
   cagggtacga aagttgaaat taaacgtacg 330
<210> 62
   <211> 110
   <212> PRT
   <213> Homo sapiens
<400> 62
   Asp Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Gln
               20                  25                  30
   Tyr Leu Ala Trp Tyr Gln Gln Lys Pro Gly Gln Ala Pro Arg Leu Leu
           35                  40                  45
   Ile Tyr Ala Ala Ser Ser Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln Asp Ser Asn Leu Pro
                   85                  90                  95
   Ala Thr Phe Gly Gln Gly Thr Lys Val Glu Ile Lys Arg Thr
               100                 105                 110
<210> 63
   <211> 351
   <212> DNA
   <213> Homo sapiens
<400> 63
   caggtgcaat tggtggaaag cggcggcggc ctggtgcaac cgggcggcag cctgcgtctg     60
   agctgcgcgg cctccggatt taccttttat aagtatgcta tgcattgggt gcgccaagcc    120
   cctgggaagg gtctcgagtg ggtgagcggt atccagtatg atggtagcta tacctattat    180
   gcggatagcg tgaaaggccg ttttaccatt tcacgtgata attcgaaaaa caccctgtat    240
   ctgcaaatga acagcctgcg tgcggaagat acggccgtgt attattgcgc gcgttattat    300
   tgtaagtgtg ttgatctttg gggccaaggc accctggtga cggttagctc a             351
<210> 64
   <211> 117
   <212> PRT
   <213> Homo sapiens
<400> 64
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Tyr Lys Tyr
               20                  25                  30
   Ala Met His Trp Val Arg Gln Ala Pro Gly Lys Gly Leu Glu Trp Val
           35                  40                  45
   Ser Gly Ile Gln Tyr Asp Gly Ser Tyr Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Tyr Tyr Cys Lys Cys Val Asp Leu Trp Gly Gln Gly Thr Leu
               100                 105                 110
   Val Thr Val Ser Ser
           115
<210> 65
   <211> 327
   <212> DNA
   <213> Homo sapiens
<400> 65
   gatatcgaac tgacccagcc gccttcagtg agcgttgcac caggtcagac cgcgcgtatc      60
   tcgtgtagcg gcgataatat tcgtaagttt tatgttcatt ggtaccagca gaaacccggg     120
   caggcgccag ttcttgtgat ttatggtact aataagcgtc cctcaggcat cccggaacgc     180
   tttagcggat ccaacagcgg caacaccgcg accctgacca ttagcggcac tcaggcggaa     240
   gacgaagcgg attattattg ccagtcttat gattctaagt ttaatactgt gtttggcggc     300
   ggcacgaagt taaccgttct tggccag                                         327
<210> 66
   <211> 109
   <212> PRT
   <213> Homo sapiens
<400> 66
   Asp Ile Glu Leu Thr Gln Pro Pro Ser Val Ser Val Ala Pro Gly Gln
   1               5                   10                  15
   Thr Ala Arg Ile Ser Cys Ser Gly Asp Asn Ile Arg Lys Phe Tyr Val
               20                  25                  30
   His Trp Tyr Gln Gln Lys Pro Gly Gln Ala Pro Val Leu Val Ile Tyr
           35                  40                  45
   Gly Thr Asn Lys Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ser Tyr Asp Ser Lys Phe Asn Thr
                   85                  90                  95
   Val Phe Gly Gly Gly Thr Lys Leu Thr Val Leu Gly Gln
               100                 105
<210> 67
   <211> 359
   <212> DNA
   <213> Homo sapiens
<400> 67
   caggtgcaat tggtggaaag cggcggcggc ctggtgcaac cgggcggcag cctgcgtctg      60
   agctgcgcgg cctccggatt tacctttcct tcttatgcta tgaattgggt gcgccaagcc     120
   cctgggaagg gtctcgagtg ggtgagcgct atcctttctg atggtagctc tacctcttat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgttatcct     300
   gattggggtt ggtatactga tgtttggggc caaggcaccc tggtgacggt tagctcagc      359
<210> 68
   <211> 119
   <212> PRT
   <213> Homo sapiens
<400> 68
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Asn Trp Val Arg Gln Ala Pro Gly Lys Gly Leu Glu Trp Val
           35                  40                  45
   Ser Ala Ile Leu Ser Asp Gly Ser Ser Thr Ser Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Tyr Pro Asp Trp Gly Trp Tyr Thr Asp Val Trp Gly Gln Gly
               100                 105                 110
   Thr Leu Val Thr Val Ser Ser
           115
<210> 69
   <211> 330
   <212> DNA
   <213> Homo sapiens
<400> 69
   gatatcgaac tgacccagcc gccttcagtg agcgttgcac caggtcagac cgcgcgtatc     60
   tcgtgtagcg gcgatgctct tcgtaagcat tatgtttatt ggtaccagca gaaacccggg    120
   caggcgccag ttcttgtgat ttatggtgat aataatcgtc cctcaggcat cccggaacgc    180
   tttagcggat ccaacagcgg caacaccgcg accctgacca ttagcggcac tcaggcggaa    240
   gacgaagcgg attattattg ccagtcttat gataagcctt atcctattct tgtgtttggc    300
   ggcggcacga agttaaccgt tcttggccag                                     330
<210> 70
   <211> 110
   <212> PRT
   <213> Homo sapiens
<400> 70
   Asp Ile Glu Leu Thr Gln Pro Pro Ser Val Ser Val Ala Pro Gly Gln
   1               5                   10                  15
   Thr Ala Arg Ile Ser Cys Ser Gly Asp Ala Leu Arg Lys His Tyr Val
               20                  25                  30
   Tyr Trp Tyr Gln Gln Lys Pro Gly Gln Ala Pro Val Leu Val Ile Tyr
           35                  40                  45
   Gly Asp Asn Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ser Tyr Asp Lys Pro Tyr Pro Ile
                   85                  90                  95
   Leu Val Phe Gly Gly Gly Thr Lys Leu Thr Val Leu Gly Gln
               100                 105                 110
<210> 71
   <211> 356
   <212> DNA
   <213> Homo sapiens
<400> 71
   caggtgcaat tggtggaaag cggcggcggc ctggtgcaac cgggcggcag cctgcgtctg      60
   agctgcgcgg cctccggatt taccttttct tcttatgcta tgacttgggt gcgccaagcc     120
   cctgggaagg gtctcgagtg ggtgagcaat atctcttatt ctggtagcaa tacctattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgtgttggt     300
   tattattatg gttttgatta ttggggccaa ggcaccctgg tgacggttag ctcagc         356
<210> 72
   <211> 118
   <212> PRT
   <213> Homo sapiens
<400> 72
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Thr Trp Val Arg Gln Ala Pro Gly Lys Gly Leu Glu Trp Val
           35                  40                  45
   Ser Asn Ile Ser Tyr Ser Gly Ser Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Val Gly Tyr Tyr Tyr Gly Phe Asp Tyr Trp Gly Gln Gly Thr
               100                 105                 110
   Leu Val Thr Val Ser Ser
           115
<210> 73
   <211> 330
   <212> DNA
   <213> Homo sapiens
<400> 73
   gatatcgtgc tgacccagag cccggcgacc ctgagcctgt ctccgggcga acgtgcgacc       60
   ctgagctgca gagcgagcca gaatgtttct tctaattatc tggcttggta ccagcagaaa      120
   ccaggtcaag caccgcgtct attaatttat gatgcttcta atcgtgcaac tggggtcccg      180
   gcgcgtttta gcggctctgg atccggcacg gattttaccc tgaccattag cagcctggaa      240
   cctgaagact ttgcggttta ttattgccag cagttttatg attctcctca gacctttggc      300
   cagggtacga aagttgaaat taaacgtacg                                       330
<210> 74
   <211> 110
   <212> PRT
   <213> Homo sapiens
<400> 74
   Asp Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Asn Val Ser Ser Asn
               20                  25                  30
   Tyr Leu Ala Trp Tyr Gln Gln Lys Pro Gly Gln Ala Pro Arg Leu Leu
           35                  40                  45
   Ile Tyr Asp Ala Ser Asn Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln Phe Tyr Asp Ser Pro
                   85                  90                  95
   Gln Thr Phe Gly Gln Gly Thr Lys Val Glu Ile Lys Arg Thr
               100                 105                 110
<210> 75
   <211> 365
   <212> DNA
   <213> Homo sapiens
<400> 75
   caggtgcaat tgcaacagtc tggtccgggc ctggtgaaac cgagccaaac cctgagcctg     60
   acctgtgcga tttccggaga tagcgtgagc tctaattctg ctgcttggtc ttggattcgc    120
   cagtctcctg ggcgtggcct cgagtggctg ggctttatct attatcgtag caagtggtat    180
   aacgattatg cggtgagcgt gaaaagccgg attaccatca acccggatac ttcgaaaaac    240
   cagtttagcc tgcaactgaa cagcgtgacc ccggaagata cggccgtgta ttattgcgcg    300
   cgtcataatc ctgatcttgg ttttgattat tggggccaag gcaccctggt gacggttagc    360
   tcagc                                                                365
<210> 76
   <211> 121
   <212> PRT
   <213> Homo sapiens
<400> 76
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Ser Trp Ile Arg Gln Ser Pro Gly Arg Gly Leu Glu
           35                  40                  45
   Trp Leu Gly Phe Ile Tyr Tyr Arg Ser Lys Trp Tyr Asn Asp Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg His Asn Pro Asp Leu Gly Phe Asp Tyr Trp Gly
               100                 105                 110
   Gln Gly Thr Leu Val Thr Val Ser Ser
           115                 120
<210> 77
   <211> 327
   <212> DNA
   <213> Homo sapiens
<400> 77
   gatatcgtgc tgacccagag cccggcgacc ctgagcctgt ctccgggcga acgtgcgacc      60
   ctgagctgca gagcgagcca gtatgttact tcttcttatc tggcttggta ccagcagaaa     120
   ccaggtcaag caccgcgtct attaatttat ggttcttctc gtgcaactgg ggtcccggcg     180
   cgttttagcg gctctggatc cggcacggat tttaccctga ccattagcag cctggaacct     240
   gaagactttg cgacttatta ttgccagcag tattcttctt ctcctattac ctttggccag     300
   ggtacgaaag ttgaaattaa acgtacg                                         327
<210> 78
   <211> 109
   <212> PRT
   <213> Homo sapiens
<400> 78
   Asp Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Tyr Val Thr Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ser Ser Arg Ala Thr Gly Val Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Thr Tyr Tyr Cys Gln Gln Tyr Ser Ser Ser Pro Ile
                   85                  90                  95
   Thr Phe Gly Gln Gly Thr Lys Val Glu Ile Lys Arg Thr
               100                 105
<210> 79
   <211> 362
   <212> DNA
   <213> Homo sapiens
<400> 79
   caggtgcaat tgcaacagtc tggtccgggc ctggtgaaac cgagccaaac cctgagcctg      60
   acctgtgcga tttccggaga tagcgtgagc tcttcttctg ctgcttggtc ttggattcgc     120
   cagtctcctg ggcgtggcct cgagtggctg ggcattatct attatcgtag caagtggtat     180
   aacgattatg cggtgagcgt gaaaagccgg attaccatca acccggatac ttcgaaaaac     240
   cagtttagcc tgcaactgaa cagcgtgacc ccggaagata cggccgtgta ttattgcgcg     300
   cgtcattcta tggttggttt tgatgtttgg ggccaaggca ccctggtgac ggttagctca     360
   gc                                                                    362
<210> 80
   <211> 120
   <212> PRT
   <213> Homo sapiens
<400> 80
   Gln Val Gln Leu Gln Gln Ser Gly Pro Gly Leu Val Lys Pro Ser Gln
   1 5 10 15
   Thr Leu Ser Leu Thr Cys Ala Ile Ser Gly Asp Ser Val Ser Ser Ser
   20 25 30
   Ser Ala Ala Trp Ser Trp Ile Arg Gln Ser Pro Gly Arg Gly Leu Glu
   35 40 45
   Trp Leu Gly Ile Ile Tyr Tyr Arg Ser Lys Trp Tyr Asn Asp Tyr Ala
   50 55 60
   Val Ser Val Lys Ser Arg Ile Thr Ile Asn Pro Asp Thr Ser Lys Asn
   65 70 75 80
   Gln Phe Ser Leu Gln Leu Asn Ser Val Thr Pro Glu Asp Thr Ala Val
   85 90 95
   Tyr Tyr Cys Ala Arg His Ser Met Val Gly Phe Asp Val Trp Gly Gln
   100 105 110
   Gly Thr Leu Val Thr Val Ser Ser
   115 120
<210> 81
   <211> 330
   <212> DNA
   <213> Homo sapiens
<400> 81
   gatatcgaac tgacccagcc gccttcagtg agcgttgcac caggtcagac cgcgcgtatc 60
   tcgtgtagcg gcgataatct tggtacttat tatgttcatt ggtaccagca gaaacccggg 120
   caggcgccag ttcttgtgat ttatggtgat aataatcgtc cctcaggcat cccggaacgc 180
   tttagcggat ccaacagcgg caacaccgcg accctgacca ttagcggcac tcaggcggaa 240
   gacgaagcgg attattattg ccagacttat gattctaata atgagtctat tgtgtttggc 300
   ggcggcacga agttaaccgt tcttggccag 330
<210> 82
   <211> 110
   <212> PRT
   <213> Homo sapiens
<400> 82
   Asp Ile Glu Leu Thr Gln Pro Pro Ser Val Ser Val Ala Pro Gly Gln
   1               5                   10                  15
   Thr Ala Arg Ile Ser Cys Ser Gly Asp Asn Leu Gly Thr Tyr Tyr Val
               20                  25                  30
   His Trp Tyr Gln Gln Lys Pro Gly Gln Ala Pro Val Leu Val Ile Tyr
           35                  40                  45
   Gly Asp Asn Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Thr Tyr Asp Ser Asn Asn Glu Ser
                   85                  90                  95
   Ile Val Phe Gly Gly Gly Thr Lys Leu Thr Val Leu Gly Gln
               100                 105                 110
<210> 83
   <211> 368
   <212> DNA
   <213> Homo sapiens
<400> 83
   caggtgcaat tggtggaaag cggcggcggc ctggtgcaac cgggcggcag cctgcgtctg      60
   agctgcgcgg cctccggatt tacctttaat tcttatgcta tgtcttgggt gcgccaagcc     120
   cctgggaagg gtctcgagtg ggtgagcaat atctcttcta attctagcaa tacctattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgtaagggt     300
   ggtggtgagc atggtttttt tccttctgat atttggggcc aaggcaccct ggtgacggtt     360
   agctcagc                                                              368
<210> 84
   <211> 122
   <212> PRT
   <213> Homo sapiens
<400> 84
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asn Ser Tyr
               20                  25                  30
   Ala Met Ser Trp Val Arg Gln Ala Pro Gly Lys Gly Leu Glu Trp Val
           35                  40                  45
   Ser Asn Ile Ser Ser Asn Ser Ser Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Lys Gly Gly Gly Glu His Gly Phe Phe Pro Ser Asp Ile Trp
               100                 105                 110
   Gly Gln Gly Thr Leu Val Thr Val Ser Ser
           115                 120
<210> 85
   <211> 333
   <212> DNA
   <213> Homo sapiens
<400> 85
   gatatcgcac tgacccagcc agcttcagtg agcggctcac caggtcagag cattaccatc        60
   tcgtgtacgg gtactagcag cgatcttggt ggttttaata ctgtgtcttg gtaccagcag       120
   catcccggga aggcgccgaa acttatgatt tattctgttt cttctcgtcc ctcaggcgtg       180
   agcaaccgtt ttagcggatc caaaagcggc aacaccgcga gcctgaccat tagcggcctg       240
   caagcggaag acgaagcgga ttattattgc cagtcttatg atcttaataa tcttgtgttt       300
   ggcggcggca cgaagttaac cgttcttggc cag                                    333
<210> 86
   <211> 111
   <212> PRT
   <213> Homo sapiens
<400> 86
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Leu Gly Gly Phe
               20                  25                  30
   Asn Thr Val Ser Trp Tyr Gln Gln His Pro Gly Lys Ala Pro Lys Leu
           35                  40                  45
   Met Ile Tyr Ser Val Ser Ser Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Ser Tyr Asp Leu Asn
                   85                  90                  95
   Asn Leu Val Phe Gly Gly Gly Thr Lys Leu Thr Val Leu Gly Gln
               100                 105                 110
<210> 87
   <211> 353
   <212> DNA
   <213> Homo sapiens
<400> 87
   caggtgcaat tggtggaaag cggcggcggc ctggtgcaac cgggcggcag cctgcgtctg      60
   agctgcgcgg cctccggatt tacctttaat tcttatgcta tgacttgggt gcgccaagcc     120
   cctgggaagg gtctcgagtg ggtgagcgct atcaagtctg atggtagcaa tacctattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgtaatgat     300
   tctggttggt ttgatgtttg gggccaaggc accctggtga cggttagctc agc            353
<210> 88
   <211> 117
   <212> PRT
   <213> Homo sapiens
<400> 88
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asn Ser Tyr
               20                  25                  30
   Ala Met Thr Trp Val Arg Gln Ala Pro Gly Lys Gly Leu Glu Trp Val
           35                  40                  45
   Ser Ala Ile Lys Ser Asp Gly Ser Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asn Asp Ser Gly Trp Phe Asp Val Trp Gly Gln Gly Thr Leu
               100                 105                 110
   Val Thr Val Ser Ser
           115
<210> 89
   <211> 330
   <212> DNA
   <213> Homo sapiens
<400> 89
   gatatcgtgc tgacccagag cccggcgacc ctgagcctgt ctccgggcga acgtgcgacc 60
   ctgagctgca gagcgagcca gtctgtttct tctttttatc tggcttggta ccagcagaaa 120
   ccaggtcaag caccgcgtct attaatttat ggttcttctt ctcgtgcaac tggggtcccg 180
   gcgcgtttta gcggctctgg atccggcacg gattttaccc tgaccattag cagcctggaa 240
   cctgaagact ttgcgactta ttattgccag cagtatgatt ctactccttc tacctttggc 300
   cagggtacga aagttgaaat taaacgtacg 330
<210> 90
   <211> 110
   <212> PRT
   <213> Homo sapiens
<400> 90
   Asp Ile Val Leu Thr Gln Ser Pro Ala Thr Leu Ser Leu Ser Pro Gly
   1 5 10 15
   Glu Arg Ala Thr Leu Ser Cys Arg Ala Ser Gln Ser Val Ser Ser Phe
   20 25 30
   Tyr Leu Ala Trp Tyr Gln Gln Lys Pro Gly Gln Ala Pro Arg Leu Leu
   35 40 45
   Ile Tyr Gly Ser Ser Ser Arg Ala Thr Gly Val Pro Ala Arg Phe Ser
   50 55 60
   Gly Ser Gly Ser Gly Thr Asp Phe Thr Leu Thr Ile Ser Ser Leu Glu
   65 70 75 80
   Pro Glu Asp Phe Ala Thr Tyr Tyr Cys Gln Gln Tyr Asp Ser Thr Pro
   85 90 95
   Ser Thr Phe Gly Gln Gly Thr Lys Val Glu Ile Lys Arg Thr
   100 105 110
<210> 91
   <211> 365
   <212> DNA
   <213> Homo sapiens
<400> 91
   caggtgcaat tgcaacagtc tggtccgggc ctggtgaaac cgagccaaac cctgagcctg 60
   acctgtgcga tttccggaga tagcgtgagc tctaatggtg ctgcttgggg ttggattcgc 120
   cagtctcctg ggcgtggcct cgagtggctg ggctttatct atcgtcgtag caagtggtat 180
   aactcttatg cggtgagcgt gaaaagccgg attaccatca acccggatac ttcgaaaaac 240
   cagtttagcc tgcaactgaa cagcgtgacc ccggaagata cggccgtgta ttattgcgcg 300
   cgtcaggatg gtatgggtgg tatggattct tggggccaag gcaccctggt gacggttagc 360
   tcagc 365
<210> 92
   <211> 121
   <212> PRT
   <213> Homo sapiens
<400> 92
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Gly Ala Ala Trp Gly Trp Ile Arg Gln Ser Pro Gly Arg Gly Leu Glu
           35                  40                  45
   Trp Leu Gly Phe Ile Tyr Arg Arg Ser Lys Trp Tyr Asn Ser Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Gln Asp Gly Met Gly Gly Met Asp Ser Trp Gly
               100                 105                 110
   Gln Gly Thr Leu Val Thr Val Ser Ser
           115                 120
<210> 93
   <211> 327
   <212> DNA
   <213> Homo sapiens
<400> 93
   gatatcgaac tgacccagcc gccttcagtg agcgttgcac caggtcagac cgcgcgtatc      60
   tcgtgtagcg gcgataatat tggttctcgt tatgcttatt ggtaccagca gaaacccggg     120
   caggcgccag ttgttgtgat ttatgatgat tctgatcgtc cctcaggcat cccggaacgc     180
   tttagcggat ccaacagcgg caacaccgcg accctgacca ttagcggcac tcaggcggaa     240
   gacgaagcgg attattattg cgctgcttat actttttatg ctcgtactgt gtttggcggc     300
   ggcacgaagt taaccgttct tggccag                                         327
<210> 94
   <211> 109
   <212> PRT
   <213> Homo sapiens
<400> 94
   Asp Ile Glu Leu Thr Gln Pro Pro Ser Val Ser Val Ala Pro Gly Gln
   1               5                   10                  15
   Thr Ala Arg Ile Ser Cys Ser Gly Asp Asn Ile Gly Ser Arg Tyr Ala
               20                  25                  30
   Tyr Trp Tyr Gln Gln Lys Pro Gly Gln Ala Pro Val Val Val Ile Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Ala Ala Tyr Thr Phe Tyr Ala Arg Thr
                   85                  90                  95
   Val Phe Gly Gly Gly Thr Lys Leu Thr Val Leu Gly Gln
               100                 105
<210> 95
   <211> 359
   <212> DNA
   <213> Homo sapiens
<400> 95
   caggtgcaat tggtggaaag cggcggcggc ctggtgcaac cgggcggcag cctgcgtctg 60
   agctgcgcgg cctccggatt taccttttct aattattatc tttcttgggt gcgccaagcc 120
   cctgggaagg gtctcgagtg ggtgagcggt atctcttata atggtagctc taccaattat 180
   gcggatagcg tgaaaggccg ttttaccatt tcacgtgata attcgaaaaa caccctgtat 240
   ctgcaaatga acagcctgcg tgcggaagat acggccgtgt attattgcgc gcgtatgtgg 300
   cgttattctc ttggtgctga ttcttggggc caaggcaccc tggtgacggt tagctcagc 359
<210> 96
   <211> 119
   <212> PRT
   <213> Homo sapiens
<400> 96
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Tyr
               20                  25                  30
   Tyr Leu Ser Trp Val Arg Gln Ala Pro Gly Lys Gly Leu Glu Trp Val
           35                  40                  45
   Ser Gly Ile Ser Tyr Asn Gly Ser Ser Thr Asn Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Met Trp Arg Tyr Ser Leu Gly Ala Asp Ser Trp Gly Gln Gly
               100                 105                 110
   Thr Leu Val Thr Val Ser Ser
           115
<210> 97
   <211> 327
   <212> DNA
   <213> Homo sapiens
<400> 97
   gatatcgaac tgacccagcc gccttcagtg agcgttgcac caggtcagac cgcgcgtatc       60
   tcgtgtagcg gcgataatat tggttctaag tatgttcatt ggtaccagca gaaacccggg      120
   caggcgccag ttgttgtgat ttatgaggat tctgatcgtc cctcaggcat cccggaacgc      180
   tttagcggat ccaacagcgg caacaccgcg accctgacca ttagcggcac tcaggcggaa      240
   gacgaagcgg attattattg ccagtcttgg gataagtctg agggttatgt gtttggcggc      300
   ggcacgaagt taaccgttct tggccag                                          327
<210> 98
   <211> 109
   <212> PRT
   <213> Homo sapiens
<400> 98
   Asp Ile Glu Leu Thr Gln Pro Pro Ser Val Ser Val Ala Pro Gly Gln
   1               5                   10                  15
   Thr Ala Arg Ile Ser Cys Ser Gly Asp Asn Ile Gly Ser Lys Tyr Val
               20                  25                  30
   His Trp Tyr Gln Gln Lys Pro Gly Gln Ala Pro Val Val Val Ile Tyr
           35                  40                  45
   Glu Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ser Trp Asp Lys Ser Glu Gly Tyr
                   85                  90                  95
   Val Phe Gly Gly Gly Thr Lys Leu Thr Val Leu Gly Gln
               100                 105
<210> 99
   <211> 371
   <212> DNA
   <213> Homo sapiens
<400> 99
   caggtgcaat tggtggaaag cggcggcggc ctggtgcaac cgggcggcag cctgcgtctg        60
   agctgcgcgg cctccggatt tacctttaat aataatgcta tttcttgggt gcgccaagcc       120
   cctgggaagg gtctcgagtg ggtgagcgct atcaattctt cttctagctc tacctcttat       180
   gcggatagcg tgaaaggccg ttttaccatt tcacgtgata attcgaaaaa caccctgtat       240
   ctgcaaatga acagcctgcg tgcggaagat acggccgtgt attattgcgc gcgtggtcat       300
   catcgtggtc attcttgggc ttcttttatt gattattggg gccaaggcac cctggtgacg       360
   gttagctcag c                                                            371
<210> 100
   <211> 123
   <212> PRT
   <213> Homo sapiens
<400> 100
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asn Asn Asn
               20                  25                  30
   Ala Ile Ser Trp Val Arg Gln Ala Pro Gly Lys Gly Leu Glu Trp Val
           35                  40                  45
   Ser Ala Ile Asn Ser Ser Ser Ser Ser Thr Ser Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly His His Arg Gly His Ser Trp Ala Ser Phe Ile Asp Tyr
               100                 105                 110
   Trp Gly Gln Gly Thr Leu Val Thr Val Ser Ser
           115                 120
<210> 101
   <211> 333
   <212> DNA
   <213> Homo sapiens
<400> 101
   gatatcgaac tgacccagcc gccttcagtg agcgttgcac caggtcagac cgcgcgtatc      60
   tcgtgtagcg gcgataatct tcgtgataag tatgcttctt ggtaccagca gaaacccggg     120
   caggcgccag ttcttgtgat ttattctaag tctgagcgtc cctcaggcat cccggaacgc     180
   tttagcggat ccaacagcgg caacaccgcg accctgacca ttagcggcac tcaggcggaa     240
   gacgaagcgg attattattg ctcttcttat actcttaatc ctaatcttaa ttatgtgttt     300
   ggcggcggca cgaagttaac cgttcttggc cag                                  333
<210> 102
   <211> 111
   <212> PRT
   <213> Homo sapiens
<400> 102
   Asp Ile Glu Leu Thr Gln Pro Pro Ser Val Ser Val Ala Pro Gly Gln
   1               5                   10                  15
   Thr Ala Arg Ile Ser Cys Ser Gly Asp Asn Leu Arg Asp Lys Tyr Ala
               20                  25                  30
   Ser Trp Tyr Gln Gln Lys Pro Gly Gln Ala Pro Val Leu Val Ile Tyr
           35                  40                  45
   Ser Lys Ser Glu Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Ser Ser Tyr Thr Leu Asn Pro Asn Leu
                   85                  90                  95
   Asn Tyr Val Phe Gly Gly Gly Thr Lys Leu Thr Val Leu Gly Gln
               100                 105                 110
<210> 103
   <211> 347
   <212> DNA
   <213> Homo sapiens
<400> 103
   caggtgcaat tggtggaaag cggcggcggc ctggtgcaac cgggcggcag cctgcgtctg      60
   agctgcgcgg cctccggatt taccttttct tcttattgga tgcattgggt gcgccaagcc     120
   cctgggaagg gtctcgagtg ggtgagctct atctcttatg attctagcaa tacctattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgttatggt     300
   ggtatggatt attggggcca aggcaccctg gtgacggtta gctcagc                   347
<210> 104
   <211> 115
   <212> PRT
   <213> Homo sapiens
<400> 104
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Trp Met His Trp Val Arg Gln Ala Pro Gly Lys Gly Leu Glu Trp Val
           35                  40                  45
   Ser Ser Ile Ser Tyr Asp Ser Ser Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Tyr Gly Gly Met Asp Tyr Trp Gly Gln Gly Thr Leu Val Thr
               100                 105                 110
   Val Ser Ser
           115
<210> 105
   <211> 327
   <212> DNA
   <213> Homo sapiens
<400> 105
   gatatcgaac tgacccagcc ggcttcagtg agcgttgcac caggtcagac cgcgcgtatc        60
   tcgtgtagcg gcgataatct tcgttctaag tatgctcatt ggtaccagca gaaacccggg       120
   caggcgccag ttcttgtgat ttatggtgat aataatcgtc cctcaggcat cccggaacgc       180
   tttagcggat ccaacagcgg caacaccgcg accctgacca ttagcggcac tcaggcggaa       240
   gacgaagcgg attattattg ctctgcttat gctatgggtt cttctcctgt gtttggcggc       300
   ggcacgaagt taaccgttct tggccag                                           327
<210> 106
   <211> 109
   <212> PRT
   <213> Homo sapiens
<400> 106
   Asp Ile Glu Leu Thr Gln Pro Ala Ser Val Ser Val Ala Pro Gly Gln
   1               5                   10                  15
   Thr Ala Arg Ile Ser Cys Ser Gly Asp Asn Leu Arg Ser Lys Tyr Ala
               20                  25                  30
   His Trp Tyr Gln Gln Lys Pro Gly Gln Ala Pro Val Leu Val Ile Tyr
           35                  40                  45
   Gly Asp Asn Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Ser Ala Tyr Ala Met Gly Ser Ser Pro
                   85                  90                  95
   Val Phe Gly Gly Gly Thr Lys Leu Thr Val Leu Gly Gln
               100                 105
<210> 107
   <211> 356
   <212> DNA
   <213> Homo sapiens
<400> 107
   caggtgcaat tggtggaaag cggcggcggc ctggtgcaac cgggcggcag cctgcgtctg       60
   agctgcgcgg cctccggatt taccttttct tcttatggta tgcattgggt gcgccaagcc      120
   cctgggaagg gtctcgagtg ggtgagcaat atctcttata tgggtagcaa taccaattat      180
   gcggatagcg tgaaaggccg ttttaccatt tcacgtgata attcgaaaaa caccctgtat      240
   ctgcaaatga acagcctgcg tgcggaagat acggccgtgt attattgcgc gcgtggtctt      300
   tttcctggtt attttgatta ttggggccaa ggcaccctgg tgacggttag ctcagc          356
<210> 108
   <211> 118
   <212> PRT
   <213> Homo sapiens
<400> 108
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ser Asn Ile Ser Tyr Met Gly Ser Asn Thr Asn Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Leu Phe Pro Gly Tyr Phe Asp Tyr Trp Gly Gln Gly Thr
               100                 105                 110
   Leu Val Thr Val Ser Ser
           115
<210> 109
   <211> 327
   <212> DNA
   <213> Homo sapiens
<400> 109
   gatatccaga tgacccagag cccgtctagc ctgagcgcga gcgtgggtga tcgtgtgacc        60
   attacctgca gagcgagcca gaatatttct aattatctga attggtacca gcagaaacca       120
   ggtaaagcac cgaaactatt aatttatggt acttcttctt tgcaaagcgg ggtcccgtcc       180
   cgttttagcg gctctggatc cggcactgat tttaccctga ccattagcag cctgcaacct       240
   gaagactttg cggtttatta ttgccagcag tatggtaata atcctactac ctttggccag       300
   ggtacgaaag ttgaaattaa acgtacg                                           327
<210> 110
   <211> 109
   <212> PRT
   <213> Homo sapiens
<400> 110
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asn Ile Ser Asn Tyr
               20                  25                  30
   Leu Asn Trp Tyr Gln Gln Lys Pro Gly Lys Ala Pro Lys Leu Leu Ile
           35                  40                  45
   Tyr Gly Thr Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Tyr Gly Asn Asn Pro Thr
                   85                  90                  95
   Thr Phe Gly Gln Gly Thr Lys Val Glu Ile Lys Arg Thr
               100                 105
<210> 111
   <211> 377
   <212> DNA
   <213> Homo sapiens
<400> 111
   caggtgcaat tgcaacagtc tggtccgggc ctggtgaaac cgagccaaac cctgagcctg 60
   acctgtgcga tttccggaga tagcgtgagc tctaatggtg ctgcttgggg ttggattcgc 120
   cagtctcctg ggcgtggcct cgagtggctg ggccatatct attatcgtag caagtggtat 180
   aactcttatg cggtgagcgt gaaaagccgg attaccatca acccggatac ttcgaaaaac 240
   cagtttagcc tgcaactgaa cagcgtgacc ccggaagata cggccgtgta ttattgcgcg 300
   cgttggggtg gtattcatga tggtgatatt tattttgatt attggggcca aggcaccctg 360
   gtgacggtta gctcagc 377
<210> 112
   <211> 125
   <212> PRT
   <213> Homo sapiens
<400> 112
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Gly Ala Ala Trp Gly Trp Ile Arg Gln Ser Pro Gly Arg Gly Leu Glu
           35                  40                  45
   Trp Leu Gly His Ile Tyr Tyr Arg Ser Lys Trp Tyr Asn Ser Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Trp Gly Gly Ile His Asp Gly Asp Ile Tyr Phe
               100                 105                 110
   Asp Tyr Trp Gly Gln Gly Thr Leu Val Thr Val Ser Ser
           115                 120                 125
<210> 113
   <211> 327
   <212> DNA
   <213> Homo sapiens
<400> 113
   gatatccaga tgacccagag cccgtctagc ctgagcgcga gcgtgggtga tcgtgtgacc      60
   attacctgca gagcgagcca gtctattact aattatctga attggtacca gcagaaacca     120
   ggtaaagcac cgaaactatt aatttatgat gtttctaatt tgcaaagcgg ggtcccgtcc     180
   cgttttagcg gctctggatc cggcactgat tttaccctga ccattagcag cctgcaacct     240
   gaagactttg cggtttatta ttgccagcag tattctggtt atcctcttac ctttggccag     300
   ggtacgaaag ttgaaattaa acgtacg                                         327
<210> 114
   <211> 109
   <212> PRT
   <213> Homo sapiens
<400> 114
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Thr Asn Tyr
               20                  25                  30
   Leu Asn Trp Tyr Gln Gln Lys Pro Gly Lys Ala Pro Lys Leu Leu Ile
           35                  40                  45
   Tyr Asp Val Ser Asn Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Tyr Ser Gly Tyr Pro Leu
                   85                  90                  95
   Thr Phe Gly Gln Gly Thr Lys Val Glu Ile Lys Arg Thr
               100                 105
<210> 115
   <211> 362
   <212> DNA
   <213> Homo sapiens
<400> 115
   caggtgcaat tgcaacagtc tggtccgggc ctggtgaaac cgagccaaac cctgagcctg      60
   acctgtgcga tttccggaga tagcgtgagc tcttcttctg ctgcttggtc ttggattcgc     120
   cagtctcctg ggcgtggcct cgagtggctg ggcatgatct attatcgtag caagtggtat     180
   aaccattatg cggtgagcgt gaaaagccgg attaccatca acccggatac ttcgaaaaac     240
   cagtttagcc tgcaactgaa cagcgtgacc ccggaagata cggccgtgta ttattgcgcg     300
   cgtggtggtt ctggtgttat ggatgtttgg ggccaaggca ccctggtgac ggttagctca     360
   gc                                                                    362
<210> 116
   <211> 120
   <212> PRT
   <213> Homo sapiens
<400> 116
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Ser
               20                  25                  30
   Ser Ala Ala Trp Ser Trp Ile Arg Gln Ser Pro Gly Arg Gly Leu Glu
           35                  40                  45
   Trp Leu Gly Met Ile Tyr Tyr Arg Ser Lys Trp Tyr Asn His Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Gly Gly Ser Gly Val Met Asp Val Trp Gly Gln
               100                 105                 110
   Gly Thr Leu Val Thr Val Ser Ser
           115                 120
<210> 117
   <211> 327
   <212> DNA
   <213> Homo sapiens
<400> 117
   gatatccaga tgacccagag cccgtctagc ctgagcgcga gcgtgggtga tcgtgtgacc      60
   attacctgca gagcgagcca gtctattaat ccttatctga attggtacca gcagaaacca     120
   ggtaaagcac cgaaactatt aatttatgct gcttctaatt tgcaaagcgg ggtcccgtcc     180
   cgttttagcg gctctggatc cggcactgat tttaccctga ccattagcag cctgcaacct     240
   gaagactttg cggtttatta ttgccagcag cttgataatc gttctattac ctttggccag     300
   ggtacgaaag ttgaaattaa acgtacg                                         327
<210> 118
   <211> 109
   <212> PRT
   <213> Homo sapiens
<400> 118
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Asn Pro Tyr
   20 25 30
   Leu Asn Trp Tyr Gln Gln Lys Pro Gly Lys Ala Pro Lys Leu Leu Ile
   35 40 45
   Tyr Ala Ala Ser Asn Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Val Tyr Tyr Cys Gln Gln Leu Asp Asn Arg Ser Ile
   85 90 95
   Thr Phe Gly Gln Gly Thr Lys Val Glu Ile Lys Arg Thr
   100 105
<210> 119
   <211> 371
   <212> DNA
   <213> Homo sapiens
<400> 119
   caggtgcaat tgcaacagtc tggtccgggc ctggtgaaac cgagccaaac cctgagcctg 60
   acctgtgcga tttccggaga tagcgtgagc tctaattctg ctgcttgggg ttggattcgc 120
   cagtctcctg ggcgtggcct cgagtggctg ggcgttatct attatcgtag caagtggtat 180
   aacgattatg cggtgagcgt gaaaagccgg attaccatca acccggatac ttcgaaaaac 240
   cagtttagcc tgcaactgaa cagcgtgacc ccggaagata cggccgtgta ttattgcgcg 300
   cgtgctcgtg ctaagaagtc tggtggtttt gattattggg gccaaggcac cctggtgacg 360
   gttagctcag c 371
<210> 120
   <211> 123
   <212> PRT
   <213> Homo sapiens
<400> 120
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Gly Trp Ile Arg Gln Ser Pro Gly Arg Gly Leu Glu
           35                  40                  45
   Trp Leu Gly Val Ile Tyr Tyr Arg Ser Lys Trp Tyr Asn Asp Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Ala Arg Ala Lys Lys Ser Gly Gly Phe Asp Tyr
               100                 105                 110
   Trp Gly Gln Gly Thr Leu Val Thr Val Ser Ser
           115                 120
<210> 121
   <211> 330
   <212> DNA
   <213> Homo sapiens
<400> 121
   gatatcgaac tgacccagcc gccttcagtg agcgttgcac caggtcagac cgcgcgtatc      60
   tcgtgtagcg gcgattctct tggttctaag tttgctcatt ggtaccagca gaaacccggg     120
   caggcgccag ttcttgtgat ttatgatgat tctaatcgtc cctcaggcat cccggaacgc     180
   tttagcggat ccaacagcgg caacaccgcg accctgacca ttagcggcac tcaggcggaa     240
   gacgaagcgg attattattg ctctacttat acttctcgtt ctcattctta tgtgtttggc     300
   ggcggcacga agttaaccgt tcttggccag                                      330
<210> 122
   <211> 110
   <212> PRT
   <213> Homo sapiens
<400> 122
   Asp Ile Glu Leu Thr Gln Pro Pro Ser Val Ser Val Ala Pro Gly Gln
   1               5                   10                  15
   Thr Ala Arg Ile Ser Cys Ser Gly Asp Ser Leu Gly Ser Lys Phe Ala
               20                  25                  30
   His Trp Tyr Gln Gln Lys Pro Gly Gln Ala Pro Val Leu Val Ile Tyr
           35                  40                  45
   Asp Asp Ser Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Ser Thr Tyr Thr Ser Arg Ser His Ser
                   85                  90                  95
   Tyr Val Phe Gly Gly Gly Thr Lys Leu Thr Val Leu Gly Gln
               100                 105                 110
<210> 123
   <211> 350
   <212> DNA
   <213> Homo sapiens
<400> 123
   caggtgcaat tggtggaaag cggcggcggc ctggtgcaac cgggcggcag cctgcgtctg      60
   agctgcgcgg cctccggatt taccttttct tcttatgctt cttgggtgcg ccaagcccct     120
   gggaagggtc tcgagtgggt gagcggtatc tctggtgatg gtagcaatac ccattatgcg     180
   gatagcgtga aaggccgttt taccatttca cgtgataatt cgaaaaacac cctgtatctg     240
   caaatgaaca gcctgcgtgc ggaagatacg gccgtgtatt attgcgcgcg ttatgataat     300
   ttttattttg atgtttgggg ccaaggcacc ctggtgacgg ttagctcagc                350
<210> 124
   <211> 116
   <212> PRT
   <213> Homo sapiens
<400> 124
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Ser Trp Val Arg Gln Ala Pro Gly Lys Gly Leu Glu Trp Val Ser
           35                  40                  45
   Gly Ile Ser Gly Asp Gly Ser Asn Thr His Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Tyr Asp Asn Phe Tyr Phe Asp Val Trp Gly Gln Gly Thr Leu Val
               100                 105                 110
   Thr Val Ser Ser
           115
<210> 125
   <211> 327
   <212> DNA
   <213> Homo sapiens
<400> 125
   gatatcgaac tgacccagcc gccttcagtg agcgttgcac caggtcagac cgcgcgtatc 60
   tcgtgtagcg gcgataatat tggttcttat tatgcttatt ggtaccagca gaaacccggg 120
   caggcgccag ttcttgtgat ttatgatgat tctaatcgtc cctcaggcat cccggaacgc 180
   tttagcggat ccaacagcgg caacaccgcg accctgacca ttagcggcac tcaggcggaa 240
   gacgaagcgg attattattg ccagtcttat gattctactg gtcttcttgt gtttggcggc 300
   ggcacgaagt taaccgttct tggccag 327
<210> 126
   <211> 109
   <212> PRT
   <213> Homo sapiens
<400> 126
   Asp Ile Glu Leu Thr Gln Pro Pro Ser Val Ser Val Ala Pro Gly Gln
   1               5                   10                  15
   Thr Ala Arg Ile Ser Cys Ser Gly Asp Asn Ile Gly Ser Tyr Tyr Ala
               20                  25                  30
   Tyr Trp Tyr Gln Gln Lys Pro Gly Gln Ala Pro Val Leu Val Ile Tyr
           35                  40                  45
   Asp Asp Ser Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ser Tyr Asp Ser Thr Gly Leu Leu
                   85                  90                  95
   Val Phe Gly Gly Gly Thr Lys Leu Thr Val Leu Gly Gln
               100                 105
<210> 127
   <211> 377
   <212> DNA
   <213> Homo sapiens
<400> 127
   caggtgcaat tggtggaaag cggcggcggc ctggtgcaac cgggcggcag cctgcgtctg      60
   agctgcgcgg cctccggatt taccttttct aattatgcta tgacttgggt gcgccaagcc     120
   cctgggaagg gtctcgagtg ggtgagcgtt atctcttctg ttggtagcaa tacctattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgtcctact     300
   aaggctggtc gtacttggtg gtggggtcct tatatggatg tttggggcca aggcaccctg     360
   gtgacggtta gctcagc                                                    377
<210> 128
   <211> 125
   <212> PRT
   <213> Homo sapiens
<400> 128
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Tyr
               20                  25                  30
   Ala Met Thr Trp Val Arg Gln Ala Pro Gly Lys Gly Leu Glu Trp Val
           35                  40                  45
   Ser Val Ile Ser Ser Val Gly Ser Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Pro Thr Lys Ala Gly Arg Thr Trp Trp Trp Gly Pro Tyr Met
               100                 105                 110
   Asp Val Trp Gly Gln Gly Thr Leu Val Thr Val Ser Ser
           115                 120                 125
<210> 129
   <211> 312
   <212> DNA
   <213> Homo sapiens
<400> 129
   gatatcgaac tgacccagcc gccttcagtg agcgttgcac caggtcagac cgcgcgtatc        60
   tcgtgtagcg gcgataatat tggttcttat tttgcttctt ggtaccagca gaaacccggg       120
   caggcgccag ttcttgtgat ttatgatgat tctaatcgtc cctcaggcat cccggaacgc       180
   tttagcggat ccaacagcgg caacaccgcg accctgacca ttagcggcac tcaggcggaa       240
   gacgaagcgg attattattg cgagggttct aatgtgtttg gcggcggcac gaagttaacc       300
   gttcttggcc ag                                                           312
<210> 130
   <211> 104
   <212> PRT
   <213> Homo sapiens
<400> 130
   Asp Ile Glu Leu Thr Gln Pro Pro Ser Val Ser Val Ala Pro Gly Gln
   1               5                   10                  15
   Thr Ala Arg Ile Ser Cys Ser Gly Asp Asn Ile Gly Ser Tyr Phe Ala
               20                  25                  30
   Ser Trp Tyr Gln Gln Lys Pro Gly Gln Ala Pro Val Leu Val Ile Tyr
           35                  40                  45
   Asp Asp Ser Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Glu Gly Ser Asn Val Phe Gly Gly Gly
                   85                  90                  95
   Thr Lys Leu Thr Val Leu Gly Gln
               100
<210> 131
   <211> 368
   <212> DNA
   <213> Homo sapiens
<400> 131
   caggtgcaat tggttcagag cggcgcggaa gtgaaaaaac cgggcgaaag cctgaaaatt 60
   agctgcaaag gttccggata ttcctttact gattattgga ttggttgggt gcgccagatg 120
   cctgggaagg gtctcgagtg gatgggcatt atccagccgt ctgatagcga taccaattat 180
   tctccgagct ttcagggcca ggtgaccatt agcgcggata aaagcattag caccgcgtat 240
   cttcaatgga gcagcctgaa agcgagcgat acggccatgt attattgcgc gcgttttatg 300
   tggtggggta agtatgattc tggttttgat gtttggggcc aaggcaccct ggtgacggtt 360
   agctcagc 368
<210> 132
   <211> 122
   <212> PRT
   <213> Homo sapiens
<400> 132
   Gln Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Ser Phe Thr Asp Tyr
               20                  25                  30
   Trp Ile Gly Trp Val Arg Gln Met Pro Gly Lys Gly Leu Glu Trp Met
           35                  40                  45
   Gly Ile Ile Gln Pro Ser Asp Ser Asp Thr Asn Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Phe Met Trp Trp Gly Lys Tyr Asp Ser Gly Phe Asp Val Trp
               100                 105                 110
   Gly Gln Gly Thr Leu Val Thr Val Ser Ser
           115                 120
<210> 133
   <211> 327
   <212> DNA
   <213> Homo sapiens
<400> 133
   gatatcgaac tgacccagcc gccttcagtg agcgttgcac caggtcagac cgcgcgtatc      60
   tcgtgtagcg gcgataatct tccttctaag tctgtttatt ggtaccagca gaaacccggg     120
   caggcgccag ttcttgtgat ttatggtgat aataatcgtc cctcaggcat cccggaacgc     180
   tttagcggat ccaacagcgg caacaccgcg accctgacca ttagcggcac tcaggcggaa     240
   gacgaagcgg attattattg ccagtcttgg acttctcgtc ctatggttgt gtttggcggc     300
   ggcacgaagt taaccgttct tggccag                                         327
<210> 134
   <211> 109
   <212> PRT
   <213> Homo sapiens
<400> 134
   Asp Ile Glu Leu Thr Gln Pro Pro Ser Val Ser Val Ala Pro Gly Gln
   1               5                   10                  15
   Thr Ala Arg Ile Ser Cys Ser Gly Asp Asn Leu Pro Ser Lys Ser Val
               20                  25                  30
   Tyr Trp Tyr Gln Gln Lys Pro Gly Gln Ala Pro Val Leu Val Ile Tyr
           35                  40                  45
   Gly Asp Asn Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ser Trp Thr Ser Arg Pro Met Val
                   85                  90                  95
   Val Phe Gly Gly Gly Thr Lys Leu Thr Val Leu Gly Gln
               100                 105
<210> 135
   <211> 353
   <212> DNA
   <213> Homo sapiens
<220>
   <221> misc_feature
   <222> (156)..(156)
   <223> n is a, c, g, or t
<400> 135
   caggtgcaat tggtggaaag cggcggcggc ctggtgcaac cgggcggcag cctgcgtctg      60
   agctgcgcgg cctccggatt taccttttct tcttattcta tgcattgggt gcgccaagcc     120
   cctgggaagg gtctcgagtg ggtgagcggt atctcntatt cttctagctt tacctattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgtgctctt     300
   ggtggtggtg ttgattattg gggccaaggc accctggtga cggttagctc agc            353
<210> 136
   <211> 117
   <212> PRT
   <213> Homo sapiens
<400> 136
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ser Met His Trp Val Arg Gln Ala Pro Gly Lys Gly Leu Glu Trp Val
           35                  40                  45
   Ser Gly Ile Ser Tyr Ser Ser Ser Phe Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Ala Leu Gly Gly Gly Val Asp Tyr Trp Gly Gln Gly Thr Leu
               100                 105                 110
   Val Thr Val Ser Ser
   115
<210> 137
   <211> 327
   <212> DNA
   <213> Homo sapiens
<400> 137
   gatatccaga tgacccagag cccgtctagc ctgagcgcga gcgtgggtga tcgtgtgacc 60
   attacctgca gagcgagcca gggtatttct tcttatctgc attggtacca gcagaaacca 120
   ggtaaagcac cgaaactatt aatttatggt gcttctactt tgcaaagcgg ggtcccgtcc 180
   cgttttagcg gctctggatc cggcactgat tttaccctga ccattagcag cctgcaacct 240
   gaagactttg cgacttatta ttgccagcag cagaatggtt atccttttac ctttggccag 300
   ggtacgaaag ttgaaattaa acgtacg 327
<210> 138
   <211> 109
   <212> PRT
   <213> Homo sapiens
<400> 138
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Ser Tyr
               20                  25                  30
   Leu His Trp Tyr Gln Gln Lys Pro Gly Lys Ala Pro Lys Leu Leu Ile
           35                  40                  45
   Tyr Gly Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gln Asn Gly Tyr Pro Phe
                   85                  90                  95
   Thr Phe Gly Gln Gly Thr Lys Val Glu Ile Lys Arg Thr
               100                 105
<210> 139
   <211> 374
   <212> DNA
   <213> Homo sapiens
<400> 139
   caggtgcaat tgcaacagtc tggtccgggc ctggtgaaac cgagccaaac cctgagcctg      60
   acctgtgcga tttccggaga tagcgtgagc tctaattctg gtggttgggg ttggattcgc     120
   cagtctcctg ggcgtggcct cgagtggctg ggccttatct attatcgtag caagtggtat     180
   aacgcttatg cggtgagcgt gaaaagccgg attaccatca acccggatac ttcgaaaaac     240
   cagtttagcc tgcaactgaa cagcgtgacc ccggaagata cggccgtgta ttattgcgcg     300
   cgttatcttg gttctaattt ttatgtttat tctgatgttt ggggccaagg caccctggtg     360
   acggttagct cagc                                                        374
<210> 140
   <211> 124
   <212> PRT
   <213> Homo sapiens
<400> 140
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Gly Gly Trp Gly Trp Ile Arg Gln Ser Pro Gly Arg Gly Leu Glu
           35                  40                  45
   Trp Leu Gly Leu Ile Tyr Tyr Arg Ser Lys Trp Tyr Asn Ala Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Tyr Leu Gly Ser Asn Phe Tyr Val Tyr Ser Asp
               100                 105                 110
   Val Trp Gly Gln Gly Thr Leu Val Thr Val Ser Ser
           115                 120
<210> 141
   <211> 327
   <212> DNA
   <213> Homo sapiens
<400> 141
   gatatccaga tgacccagag cccgtctagc ctgagcgcga gcgtgggtga tcgtgtgacc      60
   attacctgca gagcgagcca gaatattcat tctcatctga attggtacca gcagaaacca     120
   ggtaaagcac cgaaactatt aatttatgat gcttcttctt tgcaaagcgg ggtcccgtcc     180
   cgttttagcg gctctggatc cggcactgat tttaccctga ccattagcag cctgcaacct     240
   gaagactttg cggtttatta ttgccagcag tattatgatt atcctcttac ctttggccag     300
   ggtacgaaag ttgaaattaa acgtacg                                         327
<210> 142
   <211> 109
   <212> PRT
   <213> Homo sapiens
<400> 142
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asn Ile His Ser His
               20                  25                  30
   Leu Asn Trp Tyr Gln Gln Lys Pro Gly Lys Ala Pro Lys Leu Leu Ile
           35                  40                  45
   Tyr Asp Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Tyr Tyr Asp Tyr Pro Leu
                   85                  90                  95
   Thr Phe Gly Gln Gly Thr Lys Val Glu Ile Lys Arg Thr
               100                 105
<210> 143
   <211> 353
   <212> DNA
   <213> Homo sapiens
<400> 143
   caggtgcaat tggtggaaag cggcggcggc ctggtgcaac cgggcggcag cctgcgtctg      60
   agctgcgcgg cctccggatt taccttttct tcttattcta tgtcttgggt gcgccaagcc     120
   cctgggaagg gtctcgagtg ggtgagctct atctcttctt cttctagcaa tacctattat     180
   ggggatagcg tgaaaggccg ttttaccatt tcacgtgata attcgaaaaa caccctgtat     240
   ctgcaaatga acagcctgcg tgcggaagat acggccgtgt attattgcgc gcgtatgcat     300
   tataagggta tggatatttg gggccaaggc accctggtga cggttagctc agc           353
<210> 144
   <211> 117
   <212> PRT
   <213> Homo sapiens
<400> 144
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ser Met Ser Trp Val Arg Gln Ala Pro Gly Lys Gly Leu Glu Trp Val
   35 40 45
   Ser Ser Ile Ser Ser Ser Ser Ser Asn Thr Tyr Tyr Gly Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Met His Tyr Lys Gly Met Asp Ile Trp Gly Gln Gly Thr Leu
   100 105 110
   Val Thr Val Ser Ser
   115
<210> 145
   <211> 324
   <212> DNA
   <213> Homo sapiens
<400> 145
   gatatcgaac tgacccagcc gccttcagtg agcgttgcac caggtcagac cgcgcgtatc 60
   tcgtgtagcg gcgataagct tggtaagtat tatgcttatt ggtaccagca gaaacccggg 120
   caggcgccag ttcttgtgat ttatggtgat tctaagcgtc cctcaggcat cccggaacgc 180
   tttagcggat ccaacagcgg caacaccgcg accctgacca ttagcggcac tcaggcggaa 240
   gacgaagcgg attattattg ctcttctgct gcttttggtt ctactgtgtt tggcggcggc 300
   acgaagttaa ccgttcttgg ccag 324
<210> 146
   <211> 108
   <212> PRT
   <213> Homo sapiens
<400> 146
   Asp Ile Glu Leu Thr Gln Pro Pro Ser Val Ser Val Ala Pro Gly Gln
   1               5                   10                  15
   Thr Ala Arg Ile Ser Cys Ser Gly Asp Lys Leu Gly Lys Tyr Tyr Ala
               20                  25                  30
   Tyr Trp Tyr Gln Gln Lys Pro Gly Gln Ala Pro Val Leu Val Ile Tyr
           35                  40                  45
   Gly Asp Ser Lys Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Ser Ser Ala Ala Phe Gly Ser Thr Val
                   85                  90                  95
   Phe Gly Gly Gly Thr Lys Leu Thr Val Leu Gly Gln
               100                 105
<210> 147
   <211> 350
   <212> DNA
   <213> Homo sapiens
<400> 147
   caggtgcaat tggtggaaag cggcggcggc ctggtgcaac cgggcggcag cctgcgtctg      60
   agctgcgcgg cctccggatt tacctttaat tcttattata tgtcttgggt gcgccaagcc     120
   cctgggaagg gtctcgagtg ggtgagcaat atctcttctt ctggtagcaa taccaattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgtgttcat     300
   tatggttttg atttttgggg ccaaggcacc ctggtgacgg ttagctcagc                350
<210> 148
   <211> 116
   <212> PRT
   <213> Homo sapiens
<400> 148
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asn Ser Tyr
               20                  25                  30
   Tyr Met Ser Trp Val Arg Gln Ala Pro Gly Lys Gly Leu Glu Trp Val
           35                  40                  45
   Ser Asn Ile Ser Ser Ser Gly Ser Asn Thr Asn Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Val His Tyr Gly Phe Asp Phe Trp Gly Gln Gly Thr Leu Val
               100                 105                 110
   Thr Val Ser Ser
           115
<210> 149
   <211> 327
   <212> DNA
   <213> Homo sapiens
<400> 149
   gatatcgaac tgacccagcc gccttcagtg agcgttgcac caggtcagac cgcgcgtatc 60
   tcgtgtagcg gcgatgctct tggttctaag tttgctcatt ggtaccagca gaaacccggg 120
   caggcgccag ttcttgtgat ttatgatgat tctgagcgtc cctcaggcat cccggaacgc 180
   tttagcggat ccaacagcgg caacaccgcg accctgacca ttagcggcac tcaggcggaa 240
   gacgaagcgg attattattg ccaggcttat gattctggtc ttctttatgt gtttggcggc 300
   ggcacgaagt taaccgttct tggccag 327
<210> 150
   <211> 109
   <212> PRT
   <213> Homo sapiens
<400> 150
   Asp Ile Glu Leu Thr Gln Pro Pro Ser Val Ser Val Ala Pro Gly Gln
   1               5                   10                  15
   Thr Ala Arg Ile Ser Cys Ser Gly Asp Ala Leu Gly Ser Lys Phe Ala
               20                  25                  30
   His Trp Tyr Gln Gln Lys Pro Gly Gln Ala Pro Val Leu Val Ile Tyr
           35                  40                  45
   Asp Asp Ser Glu Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ala Tyr Asp Ser Gly Leu Leu Tyr
                   85                  90                  95
   Val Phe Gly Gly Gly Thr Lys Leu Thr Val Leu Gly Gln
               100                 105
<210> 151
   <211> 359
   <212> DNA
   <213> Homo sapiens
<400> 151
   caggtgcaat tggtggaaag cggcggcggc ctggtgcaac cgggcggcag cctgcgtctg      60
   agctgcgcgg cctccggatt tacctttcgt aattatgcta tgaattgggt gcgccaagcc     120
   cctgggaagg gtctcgagtg ggtgagcgtt atctctggtt cttctagcta tacctattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgtgctgat     300
   cttccttata tggtttttga ttattggggc caaggcaccc tggtgacggt tagctcagc      359
<210> 152
   <211> 119
   <212> PRT
   <213> Homo sapiens
<400> 152
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Arg Asn Tyr
   20 25 30
   Ala Met Asn Trp Val Arg Gln Ala Pro Gly Lys Gly Leu Glu Trp Val
   35 40 45
   Ser Val Ile Ser Gly Ser Ser Ser Tyr Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Ala Asp Leu Pro Tyr Met Val Phe Asp Tyr Trp Gly Gln Gly
   100 105 110
   Thr Leu Val Thr Val Ser Ser
   115
<210> 153
   <211> 324
   <212> DNA
   <213> Homo sapiens
<400> 153
   gatatcgaac tgacccagcc gccttcagtg agcgttgcac caggtcagac cgcgcgtatc 60
   tcgtgtagcg gcgatgctct tggtaagtat tatgcttctt ggtaccagca gaaacccggg 120
   caggcgccag ttcttgtgat ttatggtgat aataagcgtc cctcaggcat cccggaacgc 180
   tttagcggat ccaacagcgg caacaccgcg accctgacca ttagcggcac tcaggcggaa 240
   gacgaagcgg attattattg ccagtcttat actactcgtt ctcttgtgtt tggcggcggc 300
   acgaagttaa ccgttcttgg ccag 324
<210> 154
   <211> 108
   <212> PRT
   <213> Homo sapiens
<400> 154
   Asp Ile Glu Leu Thr Gln Pro Pro Ser Val Ser Val Ala Pro Gly Gln
   1               5                   10                  15
   Thr Ala Arg Ile Ser Cys Ser Gly Asp Ala Leu Gly Lys Tyr Tyr Ala
               20                  25                  30
   Ser Trp Tyr Gln Gln Lys Pro Gly Gln Ala Pro Val Leu Val Ile Tyr
           35                  40                  45
   Gly Asp Asn Lys Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ser Tyr Thr Thr Arg Ser Leu Val
                   85                  90                  95
   Phe Gly Gly Gly Thr Lys Leu Thr Val Leu Gly Gln
               100                 105
<210> 155
   <211> 353
   <212> DNA
   <213> Homo sapiens
<400> 155
   caggtgcaat tggtggaaag cggcggcggc ctggtgcaac cgggcggcag cctgcgtctg      60
   agctgcgcgg cctccggatt taccttttct tcttatggta tgtcttgggt gcgccaagcc     120
   cctgggaagg gtctcgagtg ggtgagcctt atctctggtg tttctagctc tacctattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgttcttat     300
   cttggttatt ttgatgtttg gggccaaggc accctggtga cggttagctc agc            353
<210> 156
   <211> 117
   <212> PRT
   <213> Homo sapiens
<400> 156
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Gly Met Ser Trp Val Arg Gln Ala Pro Gly Lys Gly Leu Glu Trp Val
           35                  40                  45
   Ser Leu Ile Ser Gly Val Ser Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Ser Tyr Leu Gly Tyr Phe Asp Val Trp Gly Gln Gly Thr Leu
               100                 105                 110
   Val Thr Val Ser Ser
           115
<210> 157
   <211> 342
   <212> DNA
   <213> Homo sapiens
<400> 157
   gatatcgtga tgacccagag cccactgagc ctgccagtga ctccgggcga gcctgcgagc      60
   attagctgca gaagcagcca aagcctggtt ttttctgatg gcaatactta tctgaattgg     120
   taccttcaaa aaccaggtca aagcccgcag ctattaattt ataagggttc taatcgtgcc     180
   agtggggtcc cggatcgttt tagcggctct ggatccggca ccgattttac cctgaaaatt     240
   agccgtgtgg aagctgaaga cgtgggcgtg tattattgcc agcagtatga ttcttatcct     300
   cttacctttg gccagggtac gaaagttgaa attaaacgta cg                        342
<210> 158
   <211> 114
   <212> PRT
   <213> Homo sapiens
<400> 158
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Val Phe Ser
   20 25 30
   Asp Gly Asn Thr Tyr Leu Asn Trp Tyr Leu Gln Lys Pro Gly Gln Ser
   35 40 45
   Pro Gln Leu Leu Ile Tyr Lys Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Gln Gln Tyr
   85 90 95
   Asp Ser Tyr Pro Leu Thr Phe Gly Gln Gly Thr Lys Val Glu Ile Lys
   100 105 110
   Arg Thr
<210> 159
   <211> 371
   <212> DNA
   <213> Homo sapiens
<400> 159
   caggtgcaat tgcaacagtc tggtccgggc ctggtgaaac cgagccaaac cctgagcctg 60
   acctgtgcga tttccggaga tagcgtgagc tctaattctg ctgcttggtc ttggattcgc 120
   cagtctcctg ggcgtggcct cgagtggctg ggcattatct ataagcgtag caagtggtat 180
   aacgattatg cggtgagcgt gaaaagccgg attaccatca acccggatac ttcgaaaaac 240
   cagtttagcc tgcaactgaa cagcgtgacc ccggaagata cggccgtgta ttattgcgcg 300
   cgttggcatt ctgataagca ttggggtttt gattattggg gccaaggcac cctggtgacg 360
   gttagctcag c 371
<210> 160
   <211> 123
   <212> PRT
   <213> Homo sapiens
<400> 160
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Ser Trp Ile Arg Gln Ser Pro Gly Arg Gly Leu Glu
           35                  40                  45
   Trp Leu Gly Ile Ile Tyr Lys Arg Ser Lys Trp Tyr Asn Asp Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Trp His Ser Asp Lys His Trp Gly Phe Asp Tyr
               100                 105                 110
   Trp Gly Gln Gly Thr Leu Val Thr Val Ser Ser
                           115                 120
<210> 161
   <211> 327
   <212> DNA
   <213> Homo sapiens
<400> 161
   gatatcgaac tgacccagcc gccttcagtg agcgttgcac caggtcagac cgcgcgtatc      60
   tcgtgtagcg gcgatgctct tggttctaag tatgtttctt ggtaccagca gaaacccggg     120
   caggcgccag ttcttgtgat ttatggtgat aataagcgtc cctcaggcat cccggaacgc     180
   tttagcggat ccaacagcgg caacaccgcg accctgacca ttagcggcac tcaggcggaa     240
   gacgaagcgg attattattg ccagtcttat acttattctc ttaatcaggt gtttggcggc     300
   ggcacgaagt taaccgttct tggccag                                         327
<210> 162
   <211> 109
   <212> PRT
   <213> Homo sapiens
<400> 162
   Asp Ile Glu Leu Thr Gln Pro Pro Ser Val Ser Val Ala Pro Gly Gln
   1               5                   10                  15
   Thr Ala Arg Ile Ser Cys Ser Gly Asp Ala Leu Gly Ser Lys Tyr Val
               20                  25                  30
   Ser Trp Tyr Gln Gln Lys Pro Gly Gln Ala Pro Val Leu Val Ile Tyr
           35                  40                  45
   Gly Asp Asn Lys Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ser Tyr Thr Tyr Ser Leu Asn Gln
                   85                  90                  95
   Val Phe Gly Gly Gly Thr Lys Leu Thr Val Leu Gly Gln
               100                 105
<210> 163
   <211> 356
   <212> DNA
   <213> Homo sapiens
<400> 163
   caggtgcaat tggtggaaag cggcggcggc ctggtgcaac cgggcggcag cctgcgtctg      60
   agctgcgcgg cctccggatt tacctttaat gattatgcta tgtcttgggt gcgccaagcc     120
   cctgggaagg gtctcgagtg ggtgagcctt atcgagtctg tttctagctc tacctattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgtactatt     300
   ggtgttcttt gggatgatgt ttggggccaa ggcaccctgg tgacggttag ctcagc         356
<210> 164
   <211> 118
   <212> PRT
   <213> Homo sapiens
<400> 164
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asn Asp Tyr
               20                  25                  30
   Ala Met Ser Trp Val Arg Gln Ala Pro Gly Lys Gly Leu Glu Trp Val
           35                  40                  45
   Ser Leu Ile Glu Ser Val Ser Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Thr Ile Gly Val Leu Trp Asp Asp Val Trp Gly Gln Gly Thr
               100                 105                 110
   Leu Val Thr Val Ser Ser
           115
<210> 165
   <211> 324
   <212> DNA
   <213> Homo sapiens
<400> 165
   gatatcgaac tgacccagcc gccttcagtg agcgttgcac caggtcagac cgcgcgtatc 60
   tcgtgtagcg gcgataagct tggttctaag tctgttcatt ggtaccagca gaaacccggg 120
   caggcgccag ttcttgtgat ttatcgtgat actgatcgtc cctcaggcat cccggaacgc 180
   tttagcggat ccaacagcgg caacaccgcg accctgacca ttagcggcac tcaggcggaa 240
   gacgaagcgg attattattg ccagacttat gattatattc ttaatgtgtt tggcggcggc 300
   acgaagttaa ccgttcttgg ccag 324
<210> 166
   <211> 108
   <212> PRT
   <213> Homo sapiens
<400> 166
   Asp Ile Glu Leu Thr Gln Pro Pro Ser Val Ser Val Ala Pro Gly Gln
   1               5                   10                  15
   Thr Ala Arg Ile Ser Cys Ser Gly Asp Lys Leu Gly Ser Lys Ser Val
               20                  25                  30
   His Trp Tyr Gln Gln Lys Pro Gly Gln Ala Pro Val Leu Val Ile Tyr
           35                  40                  45
   Arg Asp Thr Asp Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Thr Tyr Asp Tyr Ile Leu Asn Val
                   85                  90                  95
   Phe Gly Gly Gly Thr Lys Leu Thr Val Leu Gly Gln
               100                 105
<210> 167
   <211> 359
   <212> DNA
   <213> Homo sapiens
<400> 167
   caggtgcaat tggtggaaag cggcggcggc ctggtgcaac cgggcggcag cctgcgtctg       60
   agctgcgcgg cctccggatt taccttttct acttatgcta tgcattgggt gcgccaagcc      120
   cctgggaagg gtctcgagtg ggtgagcact atctctggtt atggtagctt tacctattat      180
   gcggatagcg tgaaaggccg ttttaccatt tcacgtgata attcgaaaaa caccctgtat      240
   ctgcaaatga acagcctgcg tgcggaagat acggccgtgt attattgcgc gcgtaatggt      300
   cgtaagtatg gtcagatgga taattggggc caaggcaccc tggtgacggt tagctcagc       359
<210> 168
   <211> 119
   <212> PRT
   <213> Homo sapiens
<400> 168
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Ala Met His Trp Val Arg Gln Ala Pro Gly Lys Gly Leu Glu Trp Val
   35 40 45
   Ser Thr Ile Ser Gly Tyr Gly Ser Phe Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asn Gly Arg Lys Tyr Gly Gln Met Asp Asn Trp Gly Gln Gly
   100 105 110
   Thr Leu Val Thr Val Ser Ser
   115
<210> 169
   <211> 333
   <212> DNA
   <213> Homo sapiens
<400> 169
   gatatcgaac tgacccagcc gccttcagtg agcgttgcac caggtcagac cgcgcgtatc 60
   tcgtgtagcg gcgattctat tggtaagaag tatgttcatt ggtaccagca gaaacccggg 120
   caggcgccag ttcttgtgat ttatggtgat aataatcgtc cctcaggcat cccggaacgc 180
   tttagcggat ccaacagcgg caacaccgcg accctgacca ttagcggcac tcaggcggaa 240
   gacgaagcgg attattattg ctctactgct gattctgtta ttacttataa gaatgtgttt 300
   ggcggcggca cgaagttaac cgttcttggc cag 333
<210> 170
   <211> 111
   <212> PRT
   <213> Homo sapiens
<400> 170
   Asp Ile Glu Leu Thr Gln Pro Pro Ser Val Ser Val Ala Pro Gly Gln
   1 5 10 15
   Thr Ala Arg Ile Ser Cys Ser Gly Asp Ser Ile Gly Lys Lys Tyr Val
   20 25 30
   His Trp Tyr Gln Gln Lys Pro Gly Gln Ala Pro Val Leu Val Ile Tyr
   35 40 45
   Gly Asp Asn Asn Arg Pro Ser Gly Ile Pro Glu Arg Phe Ser Gly Ser
   50 55 60
   Asn Ser Gly Asn Thr Ala Thr Leu Thr Ile Ser Gly Thr Gln Ala Glu
   65 70 75 80
   Asp Glu Ala Asp Tyr Tyr Cys Ser Thr Ala Asp Ser Val Ile Thr Tyr
   85 90 95
   Lys Asn Val Phe Gly Gly Gly Thr Lys Leu Thr Val Leu Gly Gln
   100 105 110
<210> 171
   <211> 362
   <212> DNA
   <213> Homo sapiens
<400> 171
   caggtgcaat tggtggaaag cggcggcggc ctggtgcaac cgggcggcag cctgcgtctg 60
   agctgcgcgg cctccggatt taccttttct gatcatgcta tgcattgggt gcgccaagcc 120
   cctgggaagg gtctcgagtg ggtgagcgtt atcgagtatt ctggtagcaa gaccaattat 180
   gcggatagcg tgaaaggccg ttttaccatt tcacgtgata attcgaaaaa caccctgtat 240
   ctgcaaatga acagcctgcg tgcggaagat acggccgtgt attattgcgc gcgtggtgat 300
   tattatcctt atcttgtttt tgctatttgg ggccaaggca ccctggtgac ggttagctca 360
   gc 362
<210> 172
   <211> 120
   <212> PRT
   <213> Homo sapiens
<400> 172
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Asp His
   20 25 30
   Ala Met His Trp Val Arg Gln Ala Pro Gly Lys Gly Leu Glu Trp Val
   35 40 45
   Ser Val Ile Glu Tyr Ser Gly Ser Lys Thr Asn Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Asp Tyr Tyr Pro Tyr Leu Val Phe Ala Ile Trp Gly Gln
   100 105 110
   Gly Thr Leu Val Thr Val Ser Ser
   115 120
<210> 173
   <211> 11
   <212> PRT
   <213> Homo sapiens
<400> 173
   Ser Gly Asp Asn Ile Arg Thr Tyr Tyr Val His
   1 5 10
<210> 174
   <211> 11
   <212> PRT
   <213> Homo sapiens
<400> 174
   Ser Gly Asp Asn Ile Pro Glu Lys Tyr Val His
   1 5 10
<210> 175
   <211> 11
   <212> PRT
   <213> Homo sapiens
<400> 175
   Ser Gly Asp Lys Ile Gly Ser Lys Tyr Val Tyr
   1 5 10
<210> 176
   <211> 11
   <212> PRT
   <213> Homo sapiens
<400> 176
   Ser Gly Asp Asn Leu Arg Asn Tyr Tyr Ala His
   1 5 10
<210> 177
   <211> 11
   <212> PRT
   <213> Homo sapiens
<400> 177
   Ser Gly Asp Lys Leu Gly Lys Lys Tyr Val His
   1 5 10
<210> 178
   <211> 11
   <212> PRT
   <213> Homo sapiens
<400> 178
   Ser Gly Asp Asn Leu Gly Asn Lys Tyr Ala His
   1 5 10
<210> 179
   <211> 12
   <212> PRT
   <213> Homo sapiens
<400> 179
   Arg Ala Ser Gln Asn Ile Gly Ser Asn Tyr Leu Ala
   1 5 10
<210> 180
   <211> 11
   <212> PRT
   <213> Homo sapiens
<400> 180
   Ser Gly Asp Ala Leu Gly Thr Tyr Tyr Ala Tyr
   1 5 10
<210> 181
   <211> 11
   <212> PRT
   <213> Homo sapiens
<400> 181
   Ser Gly Asp Asn Leu Arg Gly Tyr Tyr Ala Ser
   1 5 10
<210> 182
   <211> 11
   <212> PRT
   <213> Homo sapiens
<400> 182
   Arg Ala Ser Gln Ser Ile Arg Ser Tyr Leu Ala
   1 5 10
<210> 183
   <211> 11
   <212> PRT
   <213> Homo sapiens
<400> 183
   Ser Gly Asp Ser Ile Gly Ser Tyr Tyr Ala His
   1 5 10
<210> 184
   <211> 11
   <212> PRT
   <213> Homo sapiens
<400> 184
   Arg Ala Ser Gln Asp Ile Arg Asn Asn Leu Ala
   1 5 10
<210> 185
   <211> 16
   <212> PRT
   <213> Homo sapiens
<400> 185
   Arg Ser Ser Gln Ser Leu Leu His Ser Asn Gly Tyr Thr Tyr Leu Ser
   1 5 10 15
<210> 186
   <211> 14
   <212> PRT
   <213> Homo sapiens
<400> 186
   Thr Gly Thr Ser Ser Asp Ile Gly Gly Tyr Asn Tyr Val Ser
   1 5 10
<210> 187
   <211> 11
   <212> PRT
   <213> Homo sapiens
<400> 187
   Arg Ala Ser Gln Pro Ile Tyr Asn Ser Leu Ser
   1               5                   10
<210> 188
   <211> 12
   <212> PRT
   <213> Homo sapiens
<400> 188
   Arg Ala Ser Gln Ser Val Ser Ser Gln Tyr Leu Ala
   1               5                   10
<210> 189
   <211> 11
   <212> PRT
   <213> Homo sapiens
<400> 189
   Ser Gly Asp Asn Ile Arg Lys Phe Tyr Val His
   1               5                   10
<210> 190
   <211> 11
   <212> PRT
   <213> Homo sapiens
<400> 190
   Ser Gly Asp Ala Leu Arg Lys His Tyr Val Tyr
   1               5                   10
<210> 191
   <211> 12
   <212> PRT
   <213> Homo sapiens
<400> 191
   Arg Ala Ser Gln Asn Val Ser Ser Asn Tyr Leu Ala
   1               5                   10
<210> 192
   <211> 12
   <212> PRT
   <213> Homo sapiens
<400> 192
   Arg Ala Ser Gln Tyr Val Thr Ser Ser Tyr Leu Ala
   1 5 10
<210> 193
   <211> 11
   <212> PRT
   <213> Homo sapiens
<400> 193
   Ser Gly Asp Asn Leu Gly Thr Tyr Tyr Val His
   1 5 10
<210> 194
   <211> 14
   <212> PRT
   <213> Homo sapiens
<400> 194
   Thr Gly Thr Ser Ser Asp Leu Gly Gly Phe Asn Thr Val Ser
   1 5 10
<210> 195
   <211> 12
   <212> PRT
   <213> Homo sapiens
<400> 195
   Arg Ala Ser Gln Ser Val Ser Ser Phe Tyr Leu Ala
   1 5 10
<210> 196
   <211> 11
   <212> PRT
   <213> Homo sapiens
<400> 196
   Ser Gly Asp Asn Ile Gly Ser Arg Tyr Ala Tyr
   1 5 10
<210> 197
   <211> 11
   <212> PRT
   <213> Homo sapiens
<400> 197
   Ser Gly Asp Asn Ile Gly Ser Lys Tyr Val His
   1 5 10
<210> 198
   <211> 11
   <212> PRT
   <213> Homo sapiens
<400> 198
   Ser Gly Asp Asn Leu Arg Asp Lys Tyr Ala Ser
   1 5 10
<210> 199
   <211> 11
   <212> PRT
   <213> Homo sapiens
<400> 199
   Ser Gly Asp Asn Leu Arg Ser Lys Tyr Ala His
   1 5 10
<210> 200
   <211> 11
   <212> PRT
   <213> Homo sapiens
<400> 200
   Arg Ala Ser Gln Asn Ile Ser Asn Tyr Leu Asn
   1 5 10
<210> 201
   <211> 11
   <212> PRT
   <213> Homo sapiens
<400> 201
   Arg Ala Ser Gln Ser Ile Thr Asn Tyr Leu Asn
   1 5 10
<210> 202
   <211> 11
   <212> PRT
   <213> Homo sapiens
<400> 202
   Arg Ala Ser Gln Ser Ile Asn Pro Tyr Leu Asn
   1 5 10
<210> 203
   <211> 11
   <212> PRT
   <213> Homo sapiens
<400> 203
   Ser Gly Asp Ser Leu Gly Ser Lys Phe Ala His
   1 5 10
<210> 204
   <211> 11
   <212> PRT
   <213> Homo sapiens
<400> 204
   Ser Gly Asp Asn Ile Gly Ser Tyr Tyr Ala Tyr
   1 5 10
<210> 205
   <211> 11
   <212> PRT
   <213> Homo sapiens
<400> 205
   Ser Gly Asp Asn Ile Gly Ser Tyr Phe Ala Ser
   1 5 10
<210> 206
   <211> 11
   <212> PRT
   <213> Homo sapiens
<400> 206
   Ser Gly Asp Asn Leu Pro Ser Lys Ser Val Tyr
   1 5 10
<210> 207
   <211> 11
   <212> PRT
   <213> Homo sapiens
<400> 207
   Arg Ala Ser Gln Gly Ile Ser Ser Tyr Leu His
   1 5 10
<210> 208
   <211> 11
   <212> PRT
   <213> Homo sapiens
<400> 208
   Arg Ala Ser Gln Asn Ile His Ser His Leu Asn
   1 5 10
<210> 209
   <211> 11
   <212> PRT
   <213> Homo sapiens
<400> 209
   Ser Gly Asp Lys Leu Gly Lys Tyr Tyr Ala Tyr
   1 5 10
<210> 210
   <211> 11
   <212> PRT
   <213> Homo sapiens
<400> 210
   Ser Gly Asp Ala Leu Gly Ser Lys Phe Ala His
   1 5 10
<210> 211
   <211> 11
   <212> PRT
   <213> Homo sapiens
<400> 211
   Ser Gly Asp Ala Leu Gly Lys Tyr Tyr Ala Ser
   1 5 10
<210> 212
   <211> 16
   <212> PRT
   <213> Homo sapiens
<400> 212
   Arg Ser Ser Gln Ser Leu Val Phe Ser Asp Gly Asn Thr Tyr Leu Asn
   1 5 10 15
<210> 213
   <211> 11
   <212> PRT
   <213> Homo sapiens
<400> 213
   Ser Gly Asp Ala Leu Gly Ser Lys Tyr Val Ser
   1 5 10
<210> 214
   <211> 11
   <212> PRT
   <213> Homo sapiens
<400> 214
   Ser Gly Asp Lys Leu Gly Ser Lys Ser Val His
   1 5 10
<210> 215
   <211> 11
   <212> PRT
   <213> Homo sapiens
<400> 215
   Ser Gly Asp Ser Ile Gly Lys Lys Tyr Val His
   1 5 10
<210> 216
   <211> 7
   <212> PRT
   <213> Homo sapiens
<400> 216
   Gly Asp Ser Lys Arg Pro Ser
   1 5
<210> 217
   <211> 7
   <212> PRT
   <213> Homo sapiens
<400> 217
   Gly Asp Asn Asn Arg Pro Ser
   1 5
<210> 218
   <211> 6
   <212> PRT
   <213> Homo sapiens
<400> 218
   Asp Ser Asn Arg Pro Ser
   1 5
<210> 219
   <211> 7
   <212> PRT
   <213> Homo sapiens
<400> 219
   Tyr Asp Asn Asn Arg Pro Ser
   1 5
<210> 220
   <211> 7
   <212> PRT
   <213> Homo sapiens
<400> 220
   Gly Asp Asp Lys Arg Pro Ser
   1 5
<210> 221
   <211> 7
   <212> PRT
   <213> Homo sapiens
<400> 221
   Tyr Asp Asn Lys Arg Pro Ser
   1 5
<210> 222
   <211> 7
   <212> PRT
   <213> Homo sapiens
<400> 222
   Gly Ala Ser Thr Arg Ala Thr
   1 5
<210> 223
   <211> 7
   <212> PRT
   <213> Homo sapiens
<400> 223
   Gly Asp Met Asn Arg Pro Ser
   1 5
<210> 224
   <211> 7
   <212> PRT
   <213> Homo sapiens
<400> 224
   Glu Asp Asn Asn Arg Pro Ser
   1 5
<210> 225
   <211> 7
   <212> PRT
   <213> Homo sapiens
<400> 225
   Lys Ala Ser Asn Leu Gln Ser
   1 5
<210> 226
   <211> 7
   <212> PRT
   <213> Homo sapiens
<400> 226
   Tyr Asp Ser Lys Arg Pro Ser
   1 5
<210> 227
   <211> 7
   <212> PRT
   <213> Homo sapiens
<400> 227
   Ala Ala Ser Ser Leu Gln Ser
   1 5
<210> 228
   <211> 7
   <212> PRT
   <213> Homo sapiens
<400> 228
   Leu Gly Ser Asn Arg Ala Ser
   1 5
<210> 229
   <211> 7
   <212> PRT
   <213> Homo sapiens
<400> 229
   Gly Val Asn Tyr Arg Pro Ser
   1 5
<210> 230
   <211> 7
   <212> PRT
   <213> Homo sapiens
<400> 230
   Gly Val Ser Asn Leu Gln Ser
   1 5
<210> 231
   <211> 7
   <212> PRT
   <213> Homo sapiens
<400> 231
   Ala Ala Ser Ser Arg Ala Thr
   1 5
<210> 232
   <211> 7
   <212> PRT
   <213> Homo sapiens
<400> 232
   Gly Thr Asn Lys Arg Pro Ser
   1 5
<210> 233
   <211> 7
   <212> PRT
   <213> Homo sapiens
<400> 233
   Gly Asp Asn Asn Arg Pro Ser
   1 5
<210> 234
   <211> 7
   <212> PRT
   <213> Homo sapiens
<400> 234
   Asp Ala Ser Asn Arg Ala Thr
   1 5
<210> 235
   <211> 6
   <212> PRT
   <213> Homo sapiens
<400> 235
   Gly Ser Ser Arg Ala Thr
   1 5
<210> 236
   <211> 7
   <212> PRT
   <213> Homo sapiens
<400> 236
   Gly Asp Asn Asn Arg Pro Ser
   1               5
<210> 237
   <211> 7
   <212> PRT
   <213> Homo sapiens
<400> 237
   Ser Val Ser Ser Arg Pro Ser
   1               5
<210> 238
   <211> 7
   <212> PRT
   <213> Homo sapiens
<400> 238
   Gly Ser Ser Ser Arg Ala Thr
   1               5
<210> 239
   <211> 7
   <212> PRT
   <213> Homo sapiens
<400> 239
   Asp Asp Ser Asp Arg Pro Ser
   1               5
<210> 240
   <211> 7
   <212> PRT
   <213> Homo sapiens
<400> 240
   Glu Asp Ser Asp Arg Pro Ser
   1               5
<210> 241
   <211> 7
   <212> PRT
   <213> Homo sapiens
<400> 241
   Ser Lys Ser Glu Arg Pro Ser
   1               5
<210> 242
   <211> 7
   <212> PRT
   <213> Homo sapiens
<400> 242
   Gly Asp Asn Asn Arg Pro Ser
   1               5
<210> 243
   <211> 7
   <212> PRT
   <213> Homo sapiens
<400> 243
   Gly Thr Ser Ser Leu Gln Ser
   1               5
<210> 244
   <211> 7
   <212> PRT
   <213> Homo sapiens
<400> 244
   Asp Val Ser Asn Leu Gln Ser
   1               5
<210> 245
   <211> 7
   <212> PRT
   <213> Homo sapiens
<400> 245
   Ala Ala Ser Asn Leu Gln Ser
   1               5
<210> 246
   <211> 7
   <212> PRT
   <213> Homo sapiens
<400> 246
   Asp Asp Ser Asn Arg Pro Ser
   1               5
<210> 247
   <211> 7
   <212> PRT
   <213> Homo sapiens
<400> 247
   Asp Asp Ser Asn Arg Pro Ser
   1 5
<210> 248
   <211> 7
   <212> PRT
   <213> Homo sapiens
<400> 248
   Asp Asp Ser Asn Arg Pro Ser
   1 5
<210> 249
   <211> 7
   <212> PRT
   <213> Homo sapiens
<400> 249
   Gly Asp Asn Asn Arg Pro Ser
   1 5
<210> 250
   <211> 7
   <212> PRT
   <213> Homo sapiens
<400> 250
   Gly Ala Ser Thr Leu Gln Ser
   1 5
<210> 251
   <211> 7
   <212> PRT
   <213> Homo sapiens
<400> 251
   Asp Ala Ser Ser Leu Gln Ser
   1 5
<210> 252
   <211> 7
   <212> PRT
   <213> Homo sapiens
<400> 252
   Gly Asp Ser Lys Arg Pro Ser
   1 5
<210> 253
   <211> 7
   <212> PRT
   <213> Homo sapiens
<400> 253
   Asp Asp Ser Glu Arg Pro Ser
   1 5
<210> 254
   <211> 7
   <212> PRT
   <213> Homo sapiens
<400> 254
   Gly Asp Asn Lys Arg Pro Ser
   1 5
<210> 255
   <211> 7
   <212> PRT
   <213> Homo sapiens
<400> 255
   Lys Gly Ser Asn Arg Ala Ser
   1 5
<210> 256
   <211> 7
   <212> PRT
   <213> Homo sapiens
<400> 256
   Gly Asp Asn Lys Arg Pro Ser
   1 5
<210> 257
   <211> 7
   <212> PRT
   <213> Homo sapiens
<400> 257
   Arg Asp Thr Asp Arg Pro Ser
   1 5
<210> 258
   <211> 7
   <212> PRT
   <213> Homo sapiens
<400> 258
   Gly Asp Asn Asn Arg Pro Ser
   1 5
<210> 259
   <211> 11
   <212> PRT
   <213> Homo sapiens
<400> 259
   Gln Ser Tyr Asp Ser Glu Ala Asp Ser Glu Val
   1 5 10
<210> 260
   <211> 10
   <212> PRT
   <213> Homo sapiens
<400> 260
   Gln Ser Phe Asp Ala Gly Ser Tyr Phe Val
   1 5 10
<210> 261
   <211> 11
   <212> PRT
   <213> Homo sapiens
<400> 261
   Ala Ser Tyr Asp Ser Ile Tyr Ser Tyr Trp Val
   1 5 10
<210> 262
   <211> 9
   <212> PRT
   <213> Homo sapiens
<400> 262
   Gln Ser Trp Asp Asp Gly Val Pro Val
   1 5
<210> 263
   <211> 10
   <212> PRT
   <213> Homo sapiens
<400> 263
   Gln Ala Trp Gly Ser Ile Ser Arg Phe Val
   1 5 10
<210> 264
   <211> 11
   <212> PRT
   <213> Homo sapiens
<400> 264
   Gln Ser Trp Thr Pro Gly Ser Asn Thr Met Val
   1               5                   10
<210> 265
   <211> 9
   <212> PRT
   <213> Homo sapiens
<400> 265
   Gln Gln Leu Asn Ser Ile Pro Val Thr
   1               5
<210> 266
   <211> 11
   <212> PRT
   <213> Homo sapiens
<400> 266
   Gln Ser Tyr Asp Ala Gly Val Lys Pro Ala Val
   1               5                   10
<210> 267
   <211> 10
   <212> PRT
   <213> Homo sapiens
<400> 267
   Gln Ser Trp Asp Ser Pro Tyr Val His Val
   1               5                   10
<210> 268
   <211> 9
   <212> PRT
   <213> Homo sapiens
<400> 268
   His Gln Tyr Ser Asp Ser Pro Val Thr
   1               5
<210> 269
   <211> 10
   <212> PRT
   <213> Homo sapiens
<400> 269
   Gln Ala Tyr Thr Gly Gln Ser Ile Ser Arg
   1 5 10
<210> 270
   <211> 9
   <212> PRT
   <213> Homo sapiens
<400> 270
   Gln Gln Arg Asn Gly Phe Pro Leu Thr
   1 5
<210> 271
   <211> 9
   <212> PRT
   <213> Homo sapiens
<400> 271
   Gln Gln Tyr Asp Asn Ala Pro Ile Thr
   1 5
<210> 272
   <211> 11
   <212> PRT
   <213> Homo sapiens
<400> 272
   Ser Ser Ala Asp Lys Phe Thr Met Ser Ile Val
   1 5 10
<210> 273
   <211> 9
   <212> PRT
   <213> Homo sapiens
<400> 273
   Leu Gln Val Asp Asn Leu Pro Ile Thr
   1 5
<210> 274
   <211> 9
   <212> PRT
   <213> Homo sapiens
<400> 274
   Gln Gln Asp Ser Asn Leu Pro Ala Thr
   1 5
<210> 275
   <211> 10
   <212> PRT
   <213> Homo sapiens
<400> 275
   Gln Ser Tyr Asp Ser Lys Phe Asn Thr Val
   1 5 10
<210> 276
   <211> 11
   <212> PRT
   <213> Homo sapiens
<400> 276
   Gln Ser Tyr Asp Lys Pro Tyr Pro Ile Leu Val
   1 5 10
<210> 277
   <211> 9
   <212> PRT
   <213> Homo sapiens
<400> 277
   Gln Gln Phe Tyr Asp Ser Pro Gln Thr
   1 5
<210> 278
   <211> 9
   <212> PRT
   <213> Homo sapiens
<400> 278
   Gln Gln Tyr Ser Ser Ser Pro Ile Thr
   1 5
<210> 279
   <211> 11
   <212> PRT
   <213> Homo sapiens
<400> 279
   Gln Thr Tyr Asp Ser Asn Asn Glu Ser Ile Val
   1 5 10
<210> 280
   <211> 9
   <212> PRT
   <213> Homo sapiens
<400> 280
   Gln Ser Tyr Asp Leu Asn Asn Leu Val
   1 5
<210> 281
   <211> 9
   <212> PRT
   <213> Homo sapiens
<400> 281
   Gln Gln Tyr Asp Ser Thr Pro Ser Thr
   1 5
<210> 282
   <211> 10
   <212> PRT
   <213> Homo sapiens
<400> 282
   Ala Ala Tyr Thr Phe Tyr Ala Arg Thr Val
   1 5 10
<210> 283
   <211> 10
   <212> PRT
   <213> Homo sapiens
<400> 283
   Gln Ser Trp Asp Lys Ser Glu Gly Tyr Val
   1 5 10
<210> 284
   <211> 12
   <212> PRT
   <213> Homo sapiens
<400> 284
   Ser Ser Tyr Thr Leu Asn Pro Asn Leu Asn Tyr Val
   1 5 10
<210> 285
   <211> 10
   <212> PRT
   <213> Homo sapiens
<400> 285
   Ser Ala Tyr Ala Met Gly Ser Ser Pro Val
   1 5 10
<210> 286
   <211> 9
   <212> PRT
   <213> Homo sapiens
<400> 286
   Gln Gln Tyr Gly Asn Asn Pro Thr Thr
   1               5
<210> 287
   <211> 9
   <212> PRT
   <213> Homo sapiens
<400> 287
   Gln Gln Tyr Ser Gly Tyr Pro Leu Thr
   1               5
<210> 288
   <211> 9
   <212> PRT
   <213> Homo sapiens
<400> 288
   Gln Gln Leu Asp Asn Arg Ser Ile Thr
   1               5
<210> 289
   <211> 11
   <212> PRT
   <213> Homo sapiens
<400> 289
   Ser Thr Tyr Thr Ser Arg Ser His Ser Tyr Val
   1               5                   10
<210> 290
   <211> 10
   <212> PRT
   <213> Homo sapiens
<400> 290
   Gln Ser Tyr Asp Ser Thr Gly Leu Leu Val
   1               5                   10
<210> 291
   <211> 5
   <212> PRT
   <213> Homo sapiens
<400> 291
   Glu Gly Ser Asn Val
   1 5
<210> 292
   <211> 10
   <212> PRT
   <213> Homo sapiens
<400> 292
   Gln Ser Trp Thr Ser Arg Pro Met Val Val
   1 5 10
<210> 293
   <211> 9
   <212> PRT
   <213> Homo sapiens
<400> 293
   Gln Gln Gln Asn Gly Tyr Pro Phe Thr
   1 5
<210> 294
   <211> 9
   <212> PRT
   <213> Homo sapiens
<400> 294
   Gln Gln Tyr Tyr Asp Tyr Pro Leu Thr
   1 5
<210> 295
   <211> 9
   <212> PRT
   <213> Homo sapiens
<400> 295
   Ser Ser Ala Ala Phe Gly Ser Thr Val
   1 5
<210> 296
   <211> 10
   <212> PRT
   <213> Homo sapiens
<400> 296
   Gln Ala Tyr Asp Ser Gly Leu Leu Tyr Val
   1 5 10
<210> 297
   <211> 9
   <212> PRT
   <213> Homo sapiens
<400> 297
   Gln Ser Tyr Thr Thr Arg Ser Leu Val
   1 5
<210> 298
   <211> 9
   <212> PRT
   <213> Homo sapiens
<400> 298
   Gln Gln Tyr Asp Ser Tyr Pro Leu Thr
   1 5
<210> 299
   <211> 10
   <212> PRT
   <213> Homo sapiens
<400> 299
   Gln Ser Tyr Thr Tyr Ser Leu Asn Gln Val
   1 5 10
<210> 300
   <211> 9
   <212> PRT
   <213> Homo sapiens
<400> 300
   Gln Thr Tyr Asp Tyr Ile Leu Asn Val
   1 5
<210> 301
   <211> 12
   <212> PRT
   <213> Homo sapiens
<400> 301
   Ser Thr Ala Asp Ser Val Ile Thr Tyr Lys Asn Val
   1 5 10
<210> 302
   <211> 5
   <212> PRT
   <213> Homo sapiens
<400> 302
   Asn Asn Ala Met Asn
   1 5
<210> 303
   <211> 4
   <212> PRT
   <213> Homo sapiens
<400> 303
   Ser Tyr Gly Ser
   1
<210> 304
   <211> 5
   <212> PRT
   <213> Homo sapiens
<400> 304
   Arg Tyr Ala Met Ser
   1 5
<210> 305
   <211> 5
   <212> PRT
   <213> Homo sapiens
<400> 305
   Ser Tyr Gly Met Ser
   1 5
<210> 306
   <211> 5
   <212> PRT
   <213> Homo sapiens
<400> 306
   Ser Tyr Ser Met Asn
   1 5
<210> 307
   <211> 5
   <212> PRT
   <213> Homo sapiens
<400> 307
   Ser Tyr Ser Met Ser
   1 5
<210> 308
   <211> 7
   <212> PRT
   <213> Homo sapiens
<400> 308
   Ser Asn Ser Ala Ala Trp Gly
   1               5
<210> 309
   <211> 5
   <212> PRT
   <213> Homo sapiens
<400> 309
   Asn Tyr Ser Met Thr
   1               5
<210> 310
   <211> 5
   <212> PRT
   <213> Homo sapiens
<400> 310
   Gly Asn Ser Met His
   1               5
<210> 311
   <211> 7
   <212> PRT
   <213> Homo sapiens
<400> 311
   Ser Asn Ser Ala Ala Trp Gly
   1               5
<210> 312
   <211> 5
   <212> PRT
   <213> Homo sapiens
<400> 312
   Pro Tyr Val Met Ser
   1               5
<210> 313
   <211> 7
   <212> PRT
   <213> Homo sapiens
<400> 313
   Ser Asn Ser Ala Ala Trp Gly
   1 5
<210> 314
   <211> 7
   <212> PRT
   <213> Homo sapiens
<400> 314
   Ser Asn Ser Ala Ala Trp Gly
   1 5
<210> 315
   <211> 7
   <212> PRT
   <213> Homo sapiens
<400> 315
   Ser Asn Ser Ala Ala Trp Gly
   1 5
<210> 316
   <211> 7
   <212> PRT
   <213> Homo sapiens
<400> 316
   Ser Asn Ser Ala Ala Trp Ser
   1 5
<210> 317
   <211> 5
   <212> PRT
   <213> Homo sapiens
<400> 317
   Lys Tyr Ala Met His
   1 5
<210> 318
   <211> 5
   <212> PRT
   <213> Homo sapiens
<400> 318
   Ser Tyr Ala Met Asn
   1 5
<210> 319
   <211> 5
   <212> PRT
   <213> Homo sapiens
<400> 319
   Ser Tyr Ala Met Thr
   1 5
<210> 320
   <211> 7
   <212> PRT
   <213> Homo sapiens
<400> 320
   Ser Asn Ser Ala Ala Trp Ser
   1 5
<210> 321
   <211> 7
   <212> PRT
   <213> Homo sapiens
<400> 321
   Ser Ser Ser Ala Ala Trp Ser
   1 5
<210> 322
   <211> 5
   <212> PRT
   <213> Homo sapiens
<400> 322
   Ser Tyr Ala Met Ser
   1 5
<210> 323
   <211> 5
   <212> PRT
   <213> Homo sapiens
<400> 323
   Ser Tyr Ala Met Thr
   1 5
<210> 324
   <211> 7
   <212> PRT
   <213> Homo sapiens
<400> 324
   Ser Asn Gly Ala Ala Trp Gly
   1 5
<210> 325
   <211> 5
   <212> PRT
   <213> Homo sapiens
<400> 325
   Asn Tyr Tyr Leu Ser
   1 5
<210> 326
   <211> 5
   <212> PRT
   <213> Homo sapiens
<400> 326
   Asn Asn Ala Ile Ser
   1 5
<210> 327
   <211> 5
   <212> PRT
   <213> Homo sapiens
<400> 327
   Ser Tyr Trp Met His
   1 5
<210> 328
   <211> 5
   <212> PRT
   <213> Homo sapiens
<400> 328
   Ser Tyr Gly Met His
   1 5
<210> 329
   <211> 7
   <212> PRT
   <213> Homo sapiens
<400> 329
   Ser Asn Gly Ala Ala Trp Gly
   1 5
<210> 330
   <211> 7
   <212> PRT
   <213> Homo sapiens
<400> 330
   Ser Ser Ser Ala Ala Trp Ser
   1               5
<210> 331
   <211> 7
   <212> PRT
   <213> Homo sapiens
<400> 331
   Ser Asn Ser Ala Ala Trp Gly
   1               5
<210> 332
   <211> 4
   <212> PRT
   <213> Homo sapiens
<400> 332
   Ser Tyr Ala Ser
   1
<210> 333
   <211> 5
   <212> PRT
   <213> Homo sapiens
<400> 333
   Asn Tyr Ala Met Thr
   1               5
<210> 334
   <211> 5
   <212> PRT
   <213> Homo sapiens
<400> 334
   Asp Tyr Trp Ile Gly
   1               5
<210> 335
   <211> 5
   <212> PRT
   <213> Homo sapiens
<400> 335
   Ser Tyr Ser Met His
   1 5
<210> 336
   <211> 7
   <212> PRT
   <213> Homo sapiens
<400> 336
   Ser Asn Ser Gly Gly Trp Gly
   1 5
<210> 337
   <211> 5
   <212> PRT
   <213> Homo sapiens
<400> 337
   Ser Tyr Ser Met Ser
   1 5
<210> 338
   <211> 5
   <212> PRT
   <213> Homo sapiens
<400> 338
   Ser Tyr Tyr Met Ser
   1 5
<210> 339
   <211> 5
   <212> PRT
   <213> Homo sapiens
<400> 339
   Asn Tyr Ala Met Asn
   1 5
<210> 340
   <211> 5
   <212> PRT
   <213> Homo sapiens
<400> 340
   Ser Tyr Gly Met Ser
   1 5
<210> 341
   <211> 7
   <212> PRT
   <213> Homo sapiens
<400> 341
   Ser Asn Ser Ala Ala Trp Ser
   1 5
<210> 342
   <211> 5
   <212> PRT
   <213> Homo sapiens
<400> 342
   Asp Tyr Ala Met Ser
   1 5
<210> 343
   <211> 5
   <212> PRT
   <213> Homo sapiens
<400> 343
   Thr Tyr Ala Met His
   1 5
<210> 344
   <211> 5
   <212> PRT
   <213> Homo sapiens
<400> 344
   Asp His Ala Met His
   1 5
<210> 345
   <211> 17
   <212> PRT
   <213> Homo sapiens
<400> 345
   Thr Ile Ser Tyr Asp Gly Ser Asn Thr Tyr Tyr Ala Asp Ser Val Lys
   1 5 10 15
   Gly
<210> 346
   <211> 17
   <212> PRT
   <213> Homo sapiens
<400> 346
   Val Ile Ser Gly Ser Gly Ser Ser Thr Tyr Tyr Ala Asp Ser Val Lys
   1               5                   10                  15
   Gly
<210> 347
   <211> 17
   <212> PRT
   <213> Homo sapiens
<400> 347
   Ser Ile Ile Ser Ser Ser Ser Glu Thr Tyr Tyr Ala Asp Ser Val Lys
   1               5                   10                  15
   Gly
<210> 348
   <211> 17
   <212> PRT
   <213> Homo sapiens
<400> 348
   Ser Ile Arg Gly Ser Ser Ser Ser Thr Tyr Tyr Ala Asp Ser Val Lys
   1               5                   10                  15
   Gly
<210> 349
   <211> 17
   <212> PRT
   <213> Homo sapiens
<400> 349
   Ala Ile Ser Tyr Thr Gly Ser Asn Thr His Tyr Ala Asp Ser Val Lys
   1               5                   10                  15
   Gly
<210> 350
   <211> 17
   <212> PRT
   <213> Homo sapiens
<400> 350
   Ser Ile Lys Gly Ser Gly Ser Asn Thr Tyr Tyr Ala Asp Ser Val Lys
   1               5                   10                  15
   Gly
<210> 351
   <211> 18
   <212> PRT
   <213> Homo sapiens
<400> 351
   Met Ile Tyr Tyr Arg Ser Lys Trp Tyr Asn Ser Tyr Ala Val Ser Val
   1               5                   10                  15
   Lys Ser
<210> 352
   <211> 17
   <212> PRT
   <213> Homo sapiens
<400> 352
   Gly Ile Ser Tyr Asn Gly Ser Asn Thr Tyr Tyr Ala Asp Ser Val Lys
   1               5                   10                  15
   Gly
<210> 353
   <211> 17
   <212> PRT
   <213> Homo sapiens
<400> 353
   Thr Ile Phe Pro Tyr Asp Gly Thr Thr Lys Tyr Ala Gln Lys Phe Gln
   1               5                   10                  15
   Gly
<210> 354
   <211> 18
   <212> PRT
   <213> Homo sapiens
<400> 354
   Met Ile Tyr His Arg Ser Lys Trp Tyr Asn Asp Tyr Ala Val Ser Val
   1               5                   10                  15
   Lys Ser
<210> 355
   <211> 17
   <212> PRT
   <213> Homo sapiens
<400> 355
   Ser Ile Ser Ser Ser Ser Ser Asn Thr Tyr Tyr Ala Asp Ser Val Lys
   1 5 10 15
   Gly
<210> 356
   <211> 18
   <212> PRT
   <213> Homo sapiens
<400> 356
   Ile Ile Tyr Tyr Arg Ser Lys Trp Tyr Asn His Tyr Ala Val Ser Val
   1 5 10 15
   Lys Ser
<210> 357
   <211> 18
   <212> PRT
   <213> Homo sapiens
<400> 357
   Leu Ile Tyr Tyr Arg Ser Lys Trp Tyr Asn Asp Tyr Ala Val Ser Val
   1 5 10 15
   Lys Ser
<210> 358
   <211> 18
   <212> PRT
   <213> Homo sapiens
<400> 358
   Met Ile Tyr Tyr Arg Ser Lys Trp Tyr Asn Asp Tyr Ala Val Ser Val
   1 5 10 15
   Lys Ser
<210> 359
   <211> 18
   <212> PRT
   <213> Homo sapiens
<400> 359
   Met Ile Phe Tyr Arg Ser Lys Trp Asn Asn Asp Tyr Ala Val Ser Val
   1               5                   10                  15
   Lys Ser
<210> 360
   <211> 17
   <212> PRT
   <213> Homo sapiens
<400> 360
   Gly Ile Gln Tyr Asp Gly Ser Tyr Thr Tyr Tyr Ala Asp Ser Val Lys
   1               5                   10                  15
   Gly
<210> 361
   <211> 17
   <212> PRT
   <213> Homo sapiens
<400> 361
   Ala Ile Leu Ser Asp Gly Ser Ser Thr Ser Tyr Ala Asp Ser Val Lys
   1               5                   10                  15
   Gly
<210> 362
   <211> 17
   <212> PRT
   <213> Homo sapiens
<400> 362
   Asn Ile Ser Tyr Ser Gly Ser Asn Thr Tyr Tyr Ala Asp Ser Val Lys
   1               5                   10                  15
   Gly
<210> 363
   <211> 18
   <212> PRT
   <213> Homo sapiens
<400> 363
   Phe Ile Tyr Tyr Arg Ser Lys Trp Tyr Asn Asp Tyr Ala Val Ser Val
   1               5                   10                  15
   Lys Ser
<210> 364
   <211> 18
   <212> PRT
   <213> Homo sapiens
<400> 364
   Ile Ile Tyr Tyr Arg Ser Lys Trp Tyr Asn Asp Tyr Ala Val Ser Val
   1               5                   10                  15
   Lys Ser
<210> 365
   <211> 17
   <212> PRT
   <213> Homo sapiens
<400> 365
   Asn Ile Ser Ser Asn Ser Ser Asn Thr Tyr Tyr Ala Asp Ser Val Lys
   1               5                   10                  15
   Gly
<210> 366
   <211> 17
   <212> PRT
   <213> Homo sapiens
<400> 366
   Ala Ile Lys Ser Asp Gly Ser Asn Thr Tyr Tyr Ala Asp Ser Val Lys
   1               5                   10                  15
   Gly
<210> 367
   <211> 18
   <212> PRT
   <213> Homo sapiens
<400> 367
   Phe Ile Tyr Arg Arg Ser Lys Trp Tyr Asn Ser Tyr Ala Val Ser Val
   1               5                   10                  15
   Lys Ser
<210> 368
   <211> 17
   <212> PRT
   <213> Homo sapiens
<400> 368
   Gly Ile Ser Tyr Asn Gly Ser Ser Thr Asn Tyr Ala Asp Ser Val Lys
   1               5                   10                  15
   Gly
<210> 369
   <211> 17
   <212> PRT
   <213> Homo sapiens
<400> 369
   Ala Ile Asn Ser Ser Ser Ser Ser Thr Ser Tyr Ala Asp Ser Val Lys
   1               5                   10                  15
   Gly
<210> 370
   <211> 17
   <212> PRT
   <213> Homo sapiens
<400> 370
   Ser Ile Ser Tyr Asp Ser Ser Asn Thr Tyr Tyr Ala Asp Ser Val Lys
   1               5                   10                  15
   Gly
<210> 371
   <211> 17
   <212> PRT
   <213> Homo sapiens
<400> 371
   Asn Ile Ser Tyr Met Gly Ser Asn Thr Asn Tyr Ala Asp Ser Val Lys
   1               5                   10                  15
   Gly
<210> 372
   <211> 18
   <212> PRT
   <213> Homo sapiens
<400> 372
   His Ile Tyr Tyr Arg Ser Lys Trp Tyr Asn Ser Tyr Ala Val Ser Val
   1               5                   10                  15
   Lys Ser
<210> 373
   <211> 18
   <212> PRT
   <213> Homo sapiens
<400> 373
   Met Ile Tyr Tyr Arg Ser Lys Trp Tyr Asn His Tyr Ala Val Ser Val
   1               5                   10                  15
   Lys Ser
<210> 374
   <211> 18
   <212> PRT
   <213> Homo sapiens
<400> 374
   Val Ile Tyr Tyr Arg Ser Lys Trp Tyr Asn Asp Tyr Ala Val Ser Val
   1               5                   10                  15
   Lys Ser
<210> 375
   <211> 17
   <212> PRT
   <213> Homo sapiens
<400> 375
   Gly Ile Ser Gly Asp Gly Ser Asn Thr His Tyr Ala Asp Ser Val Lys
   1               5                   10                  15
   Gly
<210> 376
   <211> 17
   <212> PRT
   <213> Homo sapiens
<400> 376
   Val Ile Ser Ser Val Gly Ser Asn Thr Tyr Tyr Ala Asp Ser Val Lys
   1               5                   10                  15
   Gly
<210> 377
   <211> 17
   <212> PRT
   <213> Homo sapiens
<400> 377
   Ile Ile Gln Pro Ser Asp Ser Asp Thr Asn Tyr Ser Pro Ser Phe Gln
   1               5                   10                  15
   Gly
<210> 378
   <211> 17
   <212> PRT
   <213> Homo sapiens
<400> 378
   Gly Ile Ser Tyr Ser Ser Ser Phe Thr Tyr Tyr Ala Asp Ser Val Lys
   1               5                   10                  15
   Gly
<210> 379
   <211> 18
   <212> PRT
   <213> Homo sapiens
<400> 379
   Leu Ile Tyr Tyr Arg Ser Lys Trp Tyr Asn Ala Tyr Ala Val Ser Val
   1               5                   10                  15
   Lys Ser
<210> 380
   <211> 17
   <212> PRT
   <213> Homo sapiens
<400> 380
   Ser Ile Ser Ser Ser Ser Ser Asn Thr Tyr Tyr Gly Asp Ser Val Lys
   1               5                   10                  15
   Gly
<210> 381
   <211> 17
   <212> PRT
   <213> Homo sapiens
<400> 381
   Asn Ile Ser Ser Ser Gly Ser Asn Thr Asn Tyr Ala Asp Ser Val Lys
   1               5                   10                  15
   Gly
<210> 382
   <211> 17
   <212> PRT
   <213> Homo sapiens
<400> 382
   Val Ile Ser Gly Ser Ser Ser Tyr Thr Tyr Tyr Ala Asp Ser Val Lys
   1               5                   10                  15
   Gly
<210> 383
   <211> 17
   <212> PRT
   <213> Homo sapiens
<400> 383
   Leu Ile Ser Gly Val Ser Ser Ser Thr Tyr Tyr Ala Asp Ser Val Lys
   1               5                   10                  15
   Gly
<210> 384
   <211> 18
   <212> PRT
   <213> Homo sapiens
<400> 384
   Ile Ile Tyr Lys Arg Ser Lys Trp Tyr Asn Asp Tyr Ala Val Ser Val
   1               5                   10                  15
   Lys Ser
<210> 385
   <211> 17
   <212> PRT
   <213> Homo sapiens
<400> 385
   Leu Ile Glu Ser Val Ser Ser Ser Thr Tyr Tyr Ala Asp Ser Val Lys
   1               5                   10                  15
   Gly
<210> 386
   <211> 17
   <212> PRT
   <213> Homo sapiens
<400> 386
   Thr Ile Ser Gly Tyr Gly Ser Phe Thr Tyr Tyr Ala Asp Ser Val Lys
   1 5 10 15
   Gly
<210> 387
   <211> 17
   <212> PRT
   <213> Homo sapiens
<400> 387
   Val Ile Glu Tyr Ser Gly Ser Lys Thr Asn Tyr Ala Asp Ser Val Lys
   1 5 10 15
   Gly
<210> 388
   <211> 12
   <212> PRT
   <213> Homo sapiens
<400> 388
   Gln Ala Gly Gly Trp Thr Tyr Ser Tyr Thr Asp Val
   1 5 10
<210> 389
   <211> 9
   <212> PRT
   <213> Homo sapiens
<400> 389
   Val Asn Ile Ser Thr His Phe Asp Val
   1 5
<210> 390
   <211> 12
   <212> PRT
   <213> Homo sapiens
<400> 390
   Leu Met Gly Tyr Gly His Tyr Tyr Pro Phe Asp Tyr
   1 5 10
<210> 391
   <211> 8
   <212> PRT
   <213> Homo sapiens
<400> 391
   Lys Tyr Arg Tyr Trp Phe Asp Tyr
   1 5
<210> 392
   <211> 12
   <212> PRT
   <213> Homo sapiens
<400> 392
   Ala Phe Leu Gly Tyr Lys Glu Ser Tyr Phe Asp Ile
   1 5 10
<210> 393
   <211> 7
   <212> PRT
   <213> Homo sapiens
<400> 393
   Asn Gly Gly Leu Ile Asp Val
   1 5
<210> 394
   <211> 12
   <212> PRT
   <213> Homo sapiens
<400> 394
   Thr Met Ser Lys Tyr Gly Gly Pro Gly Met Asp Val
   1 5 10
<210> 395
   <211> 9
   <212> PRT
   <213> Homo sapiens
<400> 395
   Ile Tyr Tyr Met Asn Leu Leu Ala Gly
   1 5
<210> 396
   <211> 8
   <212> PRT
   <213> Homo sapiens
<400> 396
   Gly Val His Ser Tyr Phe Asp Tyr
   1 5
<210> 397
   <211> 9
   <212> PRT
   <213> Homo sapiens
<400> 397
   Tyr Ser Ser Ile Gly His Met Asp Tyr
   1               5
<210> 398
   <211> 8
   <212> PRT
   <213> Homo sapiens
<400> 398
   Gly Asp Ser Tyr Met Tyr Asp Val
   1               5
<210> 399
   <211> 9
   <212> PRT
   <213> Homo sapiens
<400> 399
   Ser Asn Trp Ser Gly Tyr Phe Asp Tyr
   1               5
<210> 400
   <211> 11
   <212> PRT
   <213> Homo sapiens
<400> 400
   Phe Gly Asp Thr Asn Arg Asn Gly Thr Asp Val
   1               5                   10
<210> 401
   <211> 9
   <212> PRT
   <213> Homo sapiens
<400> 401
   Val Asn Gln Tyr Thr Ser Ser Asp Tyr
   1               5
<210> 402
   <211> 12
   <212> PRT
   <213> Homo sapiens
<400> 402
   Val Asn Ala Asn Gly Tyr Tyr Ala Tyr Val Asp Leu
   1 5 10
<210> 403
   <211> 8
   <212> PRT
   <213> Homo sapiens
<400> 403
   Tyr Tyr Cys Lys Cys Val Asp Leu
   1 5
<210> 404
   <211> 10
   <212> PRT
   <213> Homo sapiens
<400> 404
   Tyr Pro Asp Trp Gly Trp Tyr Thr Asp Val
   1 5 10
<210> 405
   <211> 9
   <212> PRT
   <213> Homo sapiens
<400> 405
   Val Gly Tyr Tyr Tyr Gly Phe Asp Tyr
   1 5
<210> 406
   <211> 9
   <212> PRT
   <213> Homo sapiens
<400> 406
   His Asn Pro Asp Leu Gly Phe Asp Tyr
   1 5
<210> 407
   <211> 8
   <212> PRT
   <213> Homo sapiens
<400> 407
   His Ser Met Val Gly Phe Asp Val
   1 5
<210> 408
   <211> 13
   <212> PRT
   <213> Homo sapiens
<400> 408
   Lys Gly Gly Gly Glu His Gly Phe Phe Pro Ser Asp Ile
   1               5                   10
<210> 409
   <211> 8
   <212> PRT
   <213> Homo sapiens
<400> 409
   Asn Asp Ser Gly Trp Phe Asp Val
   1               5
<210> 410
   <211> 9
   <212> PRT
   <213> Homo sapiens
<400> 410
   Gln Asp Gly Met Gly Gly Met Asp Ser
   1               5
<210> 411
   <211> 10
   <212> PRT
   <213> Homo sapiens
<400> 411
   Met Trp Arg Tyr Ser Leu Gly Ala Asp Ser
   1               5                   10
<210> 412
   <211> 14
   <212> PRT
   <213> Homo sapiens
<400> 412
   Gly His His Arg Gly His Ser Trp Ala Ser Phe Ile Asp Tyr
   1               5                   10
<210> 413
   <211> 6
   <212> PRT
   <213> Homo sapiens
<400> 413
   Tyr Gly Gly Met Asp Tyr
   1 5
<210> 414
   <211> 9
   <212> PRT
   <213> Homo sapiens
<400> 414
   Gly Leu Phe Pro Gly Tyr Phe Asp Tyr
   1 5
<210> 415
   <211> 13
   <212> PRT
   <213> Homo sapiens
<400> 415
   Trp Gly Gly Ile His Asp Gly Asp Ile Tyr Phe Asp Tyr
   1 5 10
<210> 416
   <211> 8
   <212> PRT
   <213> Homo sapiens
<400> 416
   Gly Gly Ser Gly Val Met Asp Val
   1 5
<210> 417
   <211> 11
   <212> PRT
   <213> Homo sapiens
<400> 417
   Ala Arg Ala Lys Lys Ser Gly Gly Phe Asp Tyr
   1 5 10
<210> 418
   <211> 8
   <212> PRT
   <213> Homo sapiens
<400> 418
   Tyr Asp Asn Phe Tyr Phe Asp Val
   1 5
<210> 419
   <211> 16
   <212> PRT
   <213> Homo sapiens
<400> 419
   Pro Thr Lys Ala Gly Arg Thr Trp Trp Trp Gly Pro Tyr Met Asp Val
   1 5 10 15
<210> 420
   <211> 13
   <212> PRT
   <213> Homo sapiens
<400> 420
   Phe Met Trp Trp Gly Lys Tyr Asp Ser Gly Phe Asp Val
   1 5 10
<210> 421
   <211> 8
   <212> PRT
   <213> Homo sapiens
<400> 421
   Ala Leu Gly Gly Gly Val Asp Tyr
   1 5
<210> 422
   <211> 12
   <212> PRT
   <213> Homo sapiens
<400> 422
   Tyr Leu Gly Ser Asn Phe Tyr Val Tyr Ser Asp Val
   1 5 10
<210> 423
   <211> 8
   <212> PRT
   <213> Homo sapiens
<400> 423
   Met His Tyr Lys Gly Met Asp Ile
   1 5
<210> 424
   <211> 7
   <212> PRT
   <213> Homo sapiens
<400> 424
   Val His Tyr Gly Phe Asp Phe
   1               5
<210> 425
   <211> 10
   <212> PRT
   <213> Homo sapiens
<400> 425
   Ala Asp Leu Pro Tyr Met Val Phe Asp Tyr
   1               5                   10
<210> 426
   <211> 8
   <212> PRT
   <213> Homo sapiens
<400> 426
   Ser Tyr Leu Gly Tyr Phe Asp Val
   1               5
<210> 427
   <211> 11
   <212> PRT
   <213> Homo sapiens
<400> 427
   Trp His Ser Asp Lys His Trp Gly Phe Asp Tyr
   1               5                   10
<210> 428
   <211> 9
   <212> PRT
   <213> Homo sapiens
<400> 428
   Thr Ile Gly Val Leu Trp Asp Asp Val
   1               5
<210> 429
   <211> 10
   <212> PRT
   <213> Homo sapiens
<400> 429
   Asn Gly Arg Lys Tyr Gly Gln Met Asp Asn
   1               5                   10
<210> 430
   <211> 11
   <212> PRT
   <213> Homo sapiens
<400> 430
   Gly Asp Tyr Tyr Pro Tyr Leu Val Phe Ala Ile
   1               5                   10